# EUROPEAN PATENT APPLICATION

(11) **EP 3 889 174 A1**
(43) Date of publication of application: **06.10.2021**
(21) Application number: 19879228.5
(22) Date of filing: 28.10.2019
(51) Int. Cl.: C07K 16/00, C07K 16/28, A61K 39/395, C12P 21/08

(54) **HOMODIMER-TYPE BISPECIFIC ANTIBODY AGAINST HER2 AND CD3 AND USE THEREOF**

(30) Priority: 01.11.2018 CN 201811294887
(71) Applicant: Ampsource Biopharma Shanghai Inc., Shanghai 201318 (CN)
(72) Inventor: LI, Qiang, Shanghai 201318 (CN); JIA, Shixiang, Shanghai 201318 (CN); MA, Xinlu, Shanghai 201318 (CN); YAN, Yuan, Shanghai 201318 (CN); ZHANG, Yuhua, Shanghai 201318 (CN); LI, Yuanli, Shanghai 201318 (CN)
(74) Representative: Prinz & Partner mbB
(86) International application number: PCT/CN2019/113671
(87) International publication number: WO 2020/088403

(57) **Abstract**

A tetravalent, homodimer-type bispecific antibody molecule that simultaneously targets immune effector cell antigen CD3 and human epidermal growth factor receptor 2 (Her2); the bispecific antibody molecule comprises, from in sequence from N-terminus to C-terminus, a first single-chain Fv capable of specifically binding to Her2, a second single-chain Fv capable of specifically binding to CD3, and an Fc fragment; the first and second single-chain Fv are connected by means of a connection peptide, and the second single-chain Fv is connected to the Fc directly fragment or is connected by means of a connection peptide; the Fc fragment does not have effector functions such as CDC, ADCC and ADCP. The bispecific antibody may significantly inhibit or kill tumor cells, and has controlled toxic side effects that may be caused by excessive activation of effector cells. The maximum safe starting dose in preclinical toxicology evaluation tests is significantly higher than other doses having the same target, and no systemic immunotoxicity occurs, suggesting that the drug administration safety window for the bispecific antibody is wide; in addition, said bispecific antibody is a homodimer that does not experience the problem of heavy chain and light chain mismatching; the steps of purification are simple and efficient, expression is high, and the physicochemical and in vivo stability of the antibody are significantly improved.

SEQUENCE LISTING

<110>  AMPSOURCE BIOPHARMA SHANGHAI INC.

<120>  HOMODIMER-TYPE BISPECIFIC ANTIBODY AGAINST HER2 AND CD3 AND USE
       THEREOF

<130>  A4660EP

<140>  PCT/CN2019/113671
<141>  2019-10-28

<150>  CN201811294887.4
<151>  2018-11-01

<160>  56

<170>  PatentIn version 3.5

<210>  1
<211>  744
<212>  PRT
<213>  Artificial Sequence

<220>
<223>  bispecific antibody AB7K5 amino acid sequence

<400>  1

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Asn Ile Lys Asp Thr
            20                  25                  30

Tyr Ile His Trp Val Arg Gln Ala Pro Gly Lys Gly Leu Glu Trp Val
        35                  40                  45

Ala Arg Ile Tyr Pro Thr Asn Gly Tyr Thr Arg Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ser Arg Trp Gly Gly Asp Gly Phe Tyr Ala Met Asp Tyr Trp Gly Gln
            100                 105                 110

Gly Thr Leu Val Thr Val Ser Ser Gly Gly Gly Gly Ser Gly Gly Gly
        115                 120                 125

Gly Ser Gly Gly Gly Gly Ser Asp Ile Gln Met Thr Gln Ser Pro Ser
    130                 135                 140

Ser Leu Ser Ala Ser Val Gly Asp Arg Val Thr Ile Thr Cys Arg Ala
145                 150                 155                 160

Ser Gln Asp Val Asn Thr Ala Val Ala Trp Tyr Gln Gln Lys Pro Gly
                165                 170                 175

Lys Ala Pro Lys Leu Leu Ile Tyr Ser Ala Ser Phe Leu Tyr Ser Gly
            180                 185                 190

Val Pro Ser Arg Phe Ser Gly Ser Arg Ser Gly Thr Asp Phe Thr Leu
        195                 200                 205

Thr Ile Ser Ser Leu Gln Pro Glu Asp Phe Ala Thr Tyr Tyr Cys Gln
    210                 215                 220

Gln His Tyr Thr Thr Pro Pro Thr Phe Gly Gln Gly Thr Lys Val Glu
225                 230                 235                 240

Ile Lys Asp Lys Thr His Thr Ser Pro Pro Ser Pro Ala Pro Glu Ala
                245                 250                 255

Ala Gly Gly Pro Ser Val Phe Leu Phe Pro Pro Lys Pro Lys Asp Gln
            260                 265                 270

Leu Met Ile Ser Arg Thr Pro Glu Val Thr Cys Val Val Val Asp Val
        275                 280                 285

Ser His Glu Asp Pro Glu Val Lys Phe Asn Trp Tyr Val Asp Gly Val
    290                 295                 300

Glu Val His Asn Ala Lys Thr Lys Pro Arg Glu Glu Gln Tyr Ala Ser
305                 310                 315                 320

Thr Tyr Arg Val Val Ser Val Leu Thr Val Leu His Gln Asp Trp Leu
                325                 330                 335

Asn Gly Lys Glu Tyr Lys Cys Lys Val Ser Asn Lys Ala Leu Pro Ala
            340                 345                 350

Ser Ile Glu Lys Thr Ile Ser Lys Ala Lys Gly Gln Pro Arg Glu Pro
        355                 360                 365

Gln Val Tyr Thr Leu Pro Pro Ser Arg Asp Glu Leu Thr Lys Asn Gln
    370                 375                 380

Val Ser Leu Arg Cys His Val Lys Gly Phe Tyr Pro Ser Asp Ile Ala

385                 390                 395                 400

Val Glu Trp Glu Ser Asn Gly Gln Pro Glu Asn Asn Tyr Lys Thr Thr
                405                 410                 415

Pro Pro Val Leu Asp Ser Asp Gly Ser Phe Phe Leu Tyr Ser Thr Leu
            420                 425                 430

Thr Val Asp Lys Ser Arg Trp Gln Gln Gly Asn Val Phe Ser Cys Ser
        435                 440                 445

Val Leu His Glu Ala Leu His Asn His Tyr Thr Gln Lys Ser Leu Ser
    450                 455                 460

Leu Ser Pro Gly Gly Gly Gly Gly Gly Gly Ser Gly Gly Gly Gly Ser
465                 470                 475                 480

Gly Gly Gly Gly Ser Ser Ser Ser Ser Lys Ala Pro Pro Pro Ser Glu
                485                 490                 495

Val Gln Leu Leu Glu Ser Gly Gly Gly Leu Val Gln Pro Gly Gly Ser
            500                 505                 510

Leu Lys Leu Ser Cys Ala Ala Ser Gly Phe Thr Phe Asn Thr Tyr Ala
        515                 520                 525

Met Asn Trp Val Arg Gln Ala Pro Gly Lys Gly Leu Glu Trp Val Ala
    530                 535                 540

Arg Ile Arg Ser Lys Tyr Asn Asn Tyr Ala Thr Tyr Tyr Ala Asp Ser
545                 550                 555                 560

Val Lys Asp Arg Phe Thr Ile Ser Arg Asp Asp Ser Lys Asn Thr Ala
                565                 570                 575

Tyr Leu Gln Met Asn Asn Leu Lys Thr Glu Asp Thr Ala Val Tyr Tyr
            580                 585                 590

Cys Val Arg His Gly Asn Phe Gly Asn Ser Tyr Val Ser Trp Phe Ala
        595                 600                 605

Tyr Trp Gly Gln Gly Thr Leu Val Thr Val Ser Ser Gly Gly Gly Gly
    610                 615                 620

Ser Gly Gly Gly Gly Ser Gly Gly Gly Gly Ser Glu Leu Val Val Thr
625                 630                 635                 640

Gln Glu Pro Ser Leu Thr Val Ser Pro Gly Gly Thr Val Thr Leu Thr
                645                 650                 655

Cys Arg Ser Ser Thr Gly Ala Val Thr Thr Ser Asn Tyr Ala Asn Trp
            660                 665                 670

Val Gln Gln Lys Pro Gly Gln Ala Pro Arg Gly Leu Ile Gly Gly Thr
        675                 680                 685

Asn Lys Arg Ala Pro Gly Thr Pro Ala Arg Phe Ser Gly Ser Leu Leu
    690                 695                 700

Gly Gly Lys Ala Ala Leu Thr Leu Ser Gly Val Gln Pro Glu Asp Glu
705                 710                 715                 720

Ala Glu Tyr Tyr Cys Ala Leu Trp Tyr Ser Asn Leu Trp Val Phe Gly
                725                 730                 735

Gly Gly Thr Lys Leu Thr Val Leu
            740

<210>  2
<211>  744
<212>  PRT
<213>  Artificial Sequence

<220>
<223>  bispecific antibody AB7K6 amino acid sequence

<400>  2

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Asn Ile Lys Asp Thr
            20                  25                  30

Tyr Ile His Trp Val Arg Gln Ala Pro Gly Lys Gly Leu Glu Trp Val
        35                  40                  45

Ala Arg Ile Tyr Pro Thr Asn Gly Tyr Thr Arg Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ser Arg Trp Gly Gly Asp Gly Phe Tyr Ala Met Asp Tyr Trp Gly Gln

            100                 105                 110

Gly Thr Leu Val Thr Val Ser Ser Gly Gly Gly Gly Ser Gly Gly Gly
        115                 120                 125

Gly Ser Gly Gly Gly Gly Ser Asp Ile Gln Met Thr Gln Ser Pro Ser
    130                 135                 140

Ser Leu Ser Ala Ser Val Gly Asp Arg Val Thr Ile Thr Cys Arg Ala
145                 150                 155                 160

Ser Gln Asp Val Asn Thr Ala Val Ala Trp Tyr Gln Gln Lys Pro Gly
                165                 170                 175

Lys Ala Pro Lys Leu Leu Ile Tyr Ser Ala Ser Phe Leu Tyr Ser Gly
            180                 185                 190

Val Pro Ser Arg Phe Ser Gly Ser Arg Ser Gly Thr Asp Phe Thr Leu
        195                 200                 205

Thr Ile Ser Ser Leu Gln Pro Glu Asp Phe Ala Thr Tyr Tyr Cys Gln
    210                 215                 220

Gln His Tyr Thr Thr Pro Pro Thr Phe Gly Gln Gly Thr Lys Val Glu
225                 230                 235                 240

Ile Lys Gly Gly Gly Gly Gly Gly Ser Gly Gly Gly Gly Ser Gly Gly
                245                 250                 255

Gly Gly Ser Ser Ser Ser Ser Lys Ala Pro Pro Pro Ser Glu Val Gln
            260                 265                 270

Leu Leu Glu Ser Gly Gly Gly Leu Val Gln Pro Gly Gly Ser Leu Lys
        275                 280                 285

Leu Ser Cys Ala Ala Ser Gly Phe Thr Phe Asn Thr Tyr Ala Met Asn
    290                 295                 300

Trp Val Arg Gln Ala Pro Gly Lys Gly Leu Glu Trp Val Ala Arg Ile
305                 310                 315                 320

Arg Ser Lys Tyr Asn Asn Tyr Ala Thr Tyr Tyr Ala Asp Ser Val Lys
                325                 330                 335

Asp Arg Phe Thr Ile Ser Arg Asp Asp Ser Lys Asn Thr Ala Tyr Leu
            340                 345                 350

Gln Met Asn Asn Leu Lys Thr Glu Asp Thr Ala Val Tyr Tyr Cys Val
        355                 360                 365

Arg His Gly Asn Phe Gly Asn Ser Tyr Val Ser Trp Phe Ala Tyr Trp
    370                 375                 380

Gly Gln Gly Thr Leu Val Thr Val Ser Ser Gly Gly Gly Gly Ser Gly
385                 390                 395                 400

Gly Gly Gly Ser Gly Gly Gly Gly Ser Glu Leu Val Val Thr Gln Glu
                405                 410                 415

Pro Ser Leu Thr Val Ser Pro Gly Gly Thr Val Thr Leu Thr Cys Arg
            420                 425                 430

Ser Ser Thr Gly Ala Val Thr Thr Ser Asn Tyr Ala Asn Trp Val Gln
        435                 440                 445

Gln Lys Pro Gly Gln Ala Pro Arg Gly Leu Ile Gly Gly Thr Asn Lys
    450                 455                 460

Arg Ala Pro Gly Thr Pro Ala Arg Phe Ser Gly Ser Leu Leu Gly Gly
465                 470                 475                 480

Lys Ala Ala Leu Thr Leu Ser Gly Val Gln Pro Glu Asp Glu Ala Glu
                485                 490                 495

Tyr Tyr Cys Ala Leu Trp Tyr Ser Asn Leu Trp Val Phe Gly Gly Gly
            500                 505                 510

Thr Lys Leu Thr Val Leu Asp Lys Thr His Thr Ser Pro Pro Ser Pro
        515                 520                 525

Ala Pro Glu Ala Ala Gly Gly Pro Ser Val Phe Leu Phe Pro Pro Lys
    530                 535                 540

Pro Lys Asp Gln Leu Met Ile Ser Arg Thr Pro Glu Val Thr Cys Val
545                 550                 555                 560

Val Val Asp Val Ser His Glu Asp Pro Glu Val Lys Phe Asn Trp Tyr
                565                 570                 575

Val Asp Gly Val Glu Val His Asn Ala Lys Thr Lys Pro Arg Glu Glu
            580                 585                 590

Gln Tyr Ala Ser Thr Tyr Arg Val Val Ser Val Leu Thr Val Leu His
        595                 600                 605

Gln Asp Trp Leu Asn Gly Lys Glu Tyr Lys Cys Lys Val Ser Asn Lys
    610                 615                 620

Ala Leu Pro Ala Ser Ile Glu Lys Thr Ile Ser Lys Ala Lys Gly Gln
625                 630                 635                 640

Pro Arg Glu Pro Gln Val Tyr Thr Leu Pro Pro Ser Arg Asp Glu Leu
                645                 650                 655

Thr Lys Asn Gln Val Ser Leu Arg Cys His Val Lys Gly Phe Tyr Pro
            660                 665                 670

Ser Asp Ile Ala Val Glu Trp Glu Ser Asn Gly Gln Pro Glu Asn Asn
        675                 680                 685

Tyr Lys Thr Thr Pro Pro Val Leu Asp Ser Asp Gly Ser Phe Phe Leu
    690                 695                 700

Tyr Ser Thr Leu Thr Val Asp Lys Ser Arg Trp Gln Gln Gly Asn Val
705                 710                 715                 720

Phe Ser Cys Ser Val Leu His Glu Ala Leu His Asn His Tyr Thr Gln
                725                 730                 735

Lys Ser Leu Ser Leu Ser Pro Gly
            740

<210>  3
<211>  526
<212>  PRT
<213>  Artificial Sequence

<220>
<223>  bispecific antibody AB7K8 amino acid sequence

<400>  3

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Asn Ile Lys Asp Thr
            20                  25                  30

Tyr Ile His Trp Val Arg Gln Ala Pro Gly Lys Gly Leu Glu Trp Val
        35                  40                  45

Ala Arg Ile Tyr Pro Thr Asn Gly Tyr Thr Arg Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ser Arg Trp Gly Gly Asp Gly Phe Tyr Ala Met Asp Tyr Trp Gly Gln
            100                 105                 110

Gly Thr Leu Val Thr Val Ser Ser Gly Gly Gly Gly Ser Gly Gly Gly
        115                 120                 125

Gly Ser Gly Gly Gly Gly Ser Asp Ile Gln Met Thr Gln Ser Pro Ser
    130                 135                 140

Ser Leu Ser Ala Ser Val Gly Asp Arg Val Thr Ile Thr Cys Arg Ala
145                 150                 155                 160

Ser Gln Asp Val Asn Thr Ala Val Ala Trp Tyr Gln Gln Lys Pro Gly
                165                 170                 175

Lys Ala Pro Lys Leu Leu Ile Tyr Ser Ala Ser Phe Leu Tyr Ser Gly
            180                 185                 190

Val Pro Ser Arg Phe Ser Gly Ser Arg Ser Gly Thr Asp Phe Thr Leu
        195                 200                 205

Thr Ile Ser Ser Leu Gln Pro Glu Asp Phe Ala Thr Tyr Tyr Cys Gln
    210                 215                 220

Gln His Tyr Thr Thr Pro Pro Thr Phe Gly Gln Gly Thr Lys Val Glu
225                 230                 235                 240

Ile Lys Gly Gly Gly Gly Gly Gly Ser Gly Gly Gly Gly Ser Gly Gly
                245                 250                 255

Gly Gly Ser Ser Ser Ser Ser Lys Ala Pro Pro Pro Ser Glu Val Gln
            260                 265                 270

Leu Leu Glu Ser Gly Gly Gly Leu Val Gln Pro Gly Gly Ser Leu Lys
        275                 280                 285

Leu Ser Cys Ala Ala Ser Gly Phe Thr Phe Asn Thr Tyr Ala Met Asn
    290                 295                 300

Trp Val Arg Gln Ala Pro Gly Lys Gly Leu Glu Trp Val Ala Arg Ile
305                 310                 315                 320

Arg Ser Lys Tyr Asn Asn Tyr Ala Thr Tyr Tyr Ala Asp Ser Val Lys
                325                 330                 335

Asp Arg Phe Thr Ile Ser Arg Asp Asp Ser Lys Asn Thr Ala Tyr Leu
            340                 345                 350

Gln Met Asn Asn Leu Lys Thr Glu Asp Thr Ala Val Tyr Tyr Cys Val
        355                 360                 365

Arg His Gly Asn Phe Gly Asn Ser Tyr Val Ser Trp Phe Ala Tyr Trp
    370                 375                 380

Gly Gln Gly Thr Leu Val Thr Val Ser Ser Gly Gly Gly Gly Ser Gly
385                 390                 395                 400

Gly Gly Gly Ser Gly Gly Gly Gly Ser Glu Leu Val Val Thr Gln Glu
                405                 410                 415

Pro Ser Leu Thr Val Ser Pro Gly Gly Thr Val Thr Leu Thr Cys Arg
            420                 425                 430

Ser Ser Thr Gly Ala Val Thr Thr Ser Asn Tyr Ala Asn Trp Val Gln
        435                 440                 445

Gln Lys Pro Gly Gln Ala Pro Arg Gly Leu Ile Gly Gly Thr Asn Lys
    450                 455                 460

Arg Ala Pro Gly Thr Pro Ala Arg Phe Ser Gly Ser Leu Leu Gly Gly
465                 470                 475                 480

Lys Ala Ala Leu Thr Leu Ser Gly Val Gln Pro Glu Asp Glu Ala Glu
                485                 490                 495

Tyr Tyr Cys Ala Leu Trp Tyr Ser Asn Leu Trp Val Phe Gly Gly Gly
            500                 505                 510

Thr Lys Leu Thr Val Leu His His His His His His His His
        515                 520                 525

<210>  4
<211>  744
<212>  PRT
<213>  Artificial Sequence

<220>
<223>  bispecific antibody AB7K heavy chain amino acid sequence

<400>  4

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Asn Ile Lys Asp Thr
            20                  25                  30

Tyr Ile His Trp Val Arg Gln Ala Pro Gly Lys Gly Leu Glu Trp Val
        35                  40                  45

Ala Arg Ile Tyr Pro Thr Asn Gly Tyr Thr Arg Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ser Arg Trp Gly Gly Asp Gly Phe Tyr Ala Met Asp Tyr Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Glu Glu Met Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly Lys Gly Ser Gly Gly Gly Gly Ser Gly Gly Gly Gly Ser Gly Gly
    450                 455                 460

Gly Gly Ser Ser Ser Ser Ser Lys Ala Pro Pro Pro Ser Leu Pro Ser
465                 470                 475                 480

Pro Ser Arg Leu Pro Gly Pro Ser Asp Thr Pro Ile Leu Pro Gln Glu
                485                 490                 495

Val Gln Leu Leu Glu Ser Gly Gly Gly Leu Val Gln Pro Gly Gly Ser
            500                 505                 510

Leu Lys Leu Ser Cys Ala Ala Ser Gly Phe Thr Phe Asn Thr Tyr Ala
        515                 520                 525

Met Asn Trp Val Arg Gln Ala Pro Gly Lys Gly Leu Glu Trp Val Ala
    530                 535                 540

Arg Ile Arg Ser Lys Tyr Asn Asn Tyr Ala Thr Tyr Tyr Ala Asp Ser
545                 550                 555                 560

Val Lys Asp Arg Phe Thr Ile Ser Arg Asp Asp Ser Lys Asn Thr Ala
                565                 570                 575

Tyr Leu Gln Met Asn Asn Leu Lys Thr Glu Asp Thr Ala Val Tyr Tyr
            580                 585                 590

Cys Val Arg His Gly Asn Phe Gly Asn Ser Tyr Val Ser Trp Phe Ala
        595                 600                 605

Tyr Trp Gly Gln Gly Thr Leu Val Thr Val Ser Ser Gly Gly Gly Gly
    610                 615                 620

Ser Gly Gly Gly Gly Ser Gly Gly Gly Gly Ser Glu Leu Val Val Thr
625                 630                 635                 640

Gln Glu Pro Ser Leu Thr Val Ser Pro Gly Gly Thr Val Thr Leu Thr
                645                 650                 655

Cys Arg Ser Ser Thr Gly Ala Val Thr Thr Ser Asn Tyr Ala Asn Trp
            660                 665                 670

Val Gln Gln Lys Pro Gly Gln Ala Pro Arg Gly Leu Ile Gly Gly Thr
        675                 680                 685

Asn Lys Arg Ala Pro Gly Thr Pro Ala Arg Phe Ser Gly Ser Leu Leu
    690                 695                 700

Gly Gly Lys Ala Ala Leu Thr Leu Ser Gly Val Gln Pro Glu Asp Glu
705                 710                 715                 720

Ala Glu Tyr Tyr Cys Ala Leu Trp Tyr Ser Asn Leu Trp Val Phe Gly
                725                 730                 735

Gly Gly Thr Lys Leu Thr Val Leu
            740

<210>  5

<211>  214
<212>  PRT
<213>  Artificial Sequence

<220>
<223>  bispecific antibody AB7K light chain amino acid sequence

<400>  5

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Val Asn Thr Ala
            20                  25                  30

Val Ala Trp Tyr Gln Gln Lys Pro Gly Lys Ala Pro Lys Leu Leu Ile
        35                  40                  45

Tyr Ser Ala Ser Phe Leu Tyr Ser Gly Val Pro Ser Arg Phe Ser Gly
    50                  55                  60

Ser Arg Ser Gly Thr Asp Phe Thr Leu Thr Ile Ser Ser Leu Gln Pro
65                  70                  75                  80

Glu Asp Phe Ala Thr Tyr Tyr Cys Gln Gln His Tyr Thr Thr Pro Pro
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  6
<211>  449
<212>  PRT
<213>  Artificial Sequence

<220>
<223>  bispecific antibody AB7K4 heavy chain amino acid sequence

<400>  6

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Asn Ile Lys Asp Thr
            20                  25                  30

Tyr Ile His Trp Val Arg Gln Ala Pro Gly Lys Gly Leu Glu Trp Val
        35                  40                  45

Ala Arg Ile Tyr Pro Thr Asn Gly Tyr Thr Arg Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ser Arg Trp Gly Gly Asp Gly Phe Tyr Ala Met Asp Tyr Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Ala Ala Gly Gly
225                 230                 235                 240

Pro Ser Val Phe Leu Phe Pro Pro Lys Pro Lys Asp Gln Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Ala Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Ser Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Asp Glu Leu Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Leu His
            420                 425                 430

Glu Ala Leu His Asn His Tyr Thr Gln Lys Ser Leu Ser Leu Ser Pro
        435                 440                 445

Gly

<210>  7
<211>  490
<212>  PRT
<213>  Artificial Sequence

<220>
<223>  bispecific antibody AB7K4 light chain amino acid sequence

<400>  7

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Val Asn Thr Ala
            20                  25                  30

Val Ala Trp Tyr Gln Gln Lys Pro Gly Lys Ala Pro Lys Leu Leu Ile
        35                  40                  45

Tyr Ser Ala Ser Phe Leu Tyr Ser Gly Val Pro Ser Arg Phe Ser Gly
    50                  55                  60

Ser Arg Ser Gly Thr Asp Phe Thr Leu Thr Ile Ser Ser Leu Gln Pro
65                  70                  75                  80

Glu Asp Phe Ala Thr Tyr Tyr Cys Gln Gln His Tyr Thr Thr Pro Pro
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys Gly Gly Gly Gly Gly Gly Ser Gly Gly Gly
    210                 215                 220

Gly Ser Gly Gly Gly Gly Ser Ser Ser Ser Ser Lys Ala Pro Pro Pro
225                 230                 235                 240

Ser Glu Val Gln Leu Leu Glu Ser Gly Gly Gly Leu Val Gln Pro Gly
                245                 250                 255

Gly Ser Leu Lys Leu Ser Cys Ala Ala Ser Gly Phe Thr Phe Asn Thr
            260                 265                 270

Tyr Ala Met Asn Trp Val Arg Gln Ala Pro Gly Lys Gly Leu Glu Trp
        275                 280                 285

Val Ala Arg Ile Arg Ser Lys Tyr Asn Asn Tyr Ala Thr Tyr Tyr Ala
    290                 295                 300

Asp Ser Val Lys Asp Arg Phe Thr Ile Ser Arg Asp Asp Ser Lys Asn
305                 310                 315                 320

Thr Ala Tyr Leu Gln Met Asn Asn Leu Lys Thr Glu Asp Thr Ala Val
                325                 330                 335

Tyr Tyr Cys Val Arg His Gly Asn Phe Gly Asn Ser Tyr Val Ser Trp
            340                 345                 350

Phe Ala Tyr Trp Gly Gln Gly Thr Leu Val Thr Val Ser Ser Gly Gly
        355                 360                 365

Gly Gly Ser Gly Gly Gly Gly Ser Gly Gly Gly Gly Ser Glu Leu Val
    370                 375                 380

Val Thr Gln Glu Pro Ser Leu Thr Val Ser Pro Gly Gly Thr Val Thr
385                 390                 395                 400

Leu Thr Cys Arg Ser Ser Thr Gly Ala Val Thr Thr Ser Asn Tyr Ala
                405                 410                 415

Asn Trp Val Gln Gln Lys Pro Gly Gln Ala Pro Arg Gly Leu Ile Gly
            420                 425                 430

Gly Thr Asn Lys Arg Ala Pro Gly Thr Pro Ala Arg Phe Ser Gly Ser
        435                 440                 445

Leu Leu Gly Gly Lys Ala Ala Leu Thr Leu Ser Gly Val Gln Pro Glu
    450                 455                 460

Asp Glu Ala Glu Tyr Tyr Cys Ala Leu Trp Tyr Ser Asn Leu Trp Val
465                 470                 475                 480

Phe Gly Gly Gly Thr Lys Leu Thr Val Leu
                485                 490

<210>  8
<211>  744
<212>  PRT
<213>  Artificial Sequence

<220>
<223>  bispecific antibody AB7K7 amino acid sequence

<400>  8

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Asn Ile Lys Asp Thr
            20                  25                  30

Tyr Ile His Trp Val Arg Gln Ala Pro Gly Lys Gly Leu Glu Trp Val
        35                  40                  45

Ala Arg Ile Tyr Pro Thr Asn Gly Tyr Thr Arg Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ser Arg Trp Gly Gly Asp Gly Phe Tyr Ala Met Asp Tyr Trp Gly Gln
            100                 105                 110

Gly Thr Leu Val Thr Val Ser Ser Gly Gly Gly Gly Ser Gly Gly Gly
        115                 120                 125

Gly Ser Gly Gly Gly Gly Ser Asp Ile Gln Met Thr Gln Ser Pro Ser
    130                 135                 140

Ser Leu Ser Ala Ser Val Gly Asp Arg Val Thr Ile Thr Cys Arg Ala
145                 150                 155                 160

Ser Gln Asp Val Asn Thr Ala Val Ala Trp Tyr Gln Gln Lys Pro Gly
                165                 170                 175

Lys Ala Pro Lys Leu Leu Ile Tyr Ser Ala Ser Phe Leu Tyr Ser Gly
        180                 185                 190

Val Pro Ser Arg Phe Ser Gly Ser Arg Ser Gly Thr Asp Phe Thr Leu
    195                 200                 205

Thr Ile Ser Ser Leu Gln Pro Glu Asp Phe Ala Thr Tyr Tyr Cys Gln
    210                 215                 220

Gln His Tyr Thr Thr Pro Pro Thr Phe Gly Gln Gly Thr Lys Val Glu
225                 230                 235                 240

Ile Lys Gly Gly Gly Gly Gly Gly Ser Gly Gly Gly Gly Ser Gly Gly
                245                 250                 255

Gly Gly Ser Ser Ser Ser Ser Lys Ala Pro Pro Pro Ser Glu Val Gln
            260                 265                 270

Leu Leu Glu Ser Gly Gly Gly Leu Val Gln Pro Gly Gly Ser Leu Lys
        275                 280                 285

Leu Ser Cys Ala Ala Ser Gly Phe Thr Phe Asn Thr Tyr Ala Met Asn
    290                 295                 300

Trp Val Arg Gln Ala Pro Gly Lys Gly Leu Glu Trp Val Ala Arg Ile
305                 310                 315                 320

Arg Ser Lys Tyr Asn Asn Tyr Ala Thr Tyr Tyr Ala Asp Ser Val Lys
                325                 330                 335

Asp Arg Phe Thr Ile Ser Arg Asp Asp Ser Lys Asn Thr Ala Tyr Leu
            340                 345                 350

Gln Met Asn Asn Leu Lys Thr Glu Asp Thr Ala Val Tyr Tyr Cys Val
        355                 360                 365

Arg His Gly Asn Phe Gly Asn Ser Tyr Val Ser Trp Phe Ala Tyr Trp
    370                 375                 380

Gly Gln Gly Thr Leu Val Thr Val Ser Ser Gly Gly Gly Gly Ser Gly
385                 390                 395                 400

Gly Gly Gly Ser Gly Gly Gly Gly Ser Glu Leu Val Val Thr Gln Glu
                405                 410                 415

Pro Ser Leu Thr Val Ser Pro Gly Gly Thr Val Thr Leu Thr Cys Arg
            420                 425                 430

Ser Ser Thr Gly Ala Val Thr Thr Ser Asn Tyr Ala Asn Trp Val Gln
        435                 440                 445

Gln Lys Pro Gly Gln Ala Pro Arg Gly Leu Ile Gly Gly Thr Asn Lys
    450                 455                 460

Arg Ala Pro Gly Thr Pro Ala Arg Phe Ser Gly Ser Leu Leu Gly Gly
465                 470                 475                 480

Lys Ala Ala Leu Thr Leu Ser Gly Val Gln Pro Glu Asp Glu Ala Glu
                485                 490                 495

Tyr Tyr Cys Ala Leu Trp Tyr Ser Asn Leu Trp Val Phe Gly Gly Gly
            500                 505                 510

Thr Lys Leu Thr Val Leu Asp Lys Thr His Thr Cys Pro Pro Cys Pro
        515                 520                 525

Ala Pro Glu Ala Ala Gly Gly Pro Ser Val Phe Leu Phe Pro Pro Lys
    530                 535                 540

Pro Lys Asp Gln Leu Met Ile Ser Arg Thr Pro Glu Val Thr Cys Val
545                 550                 555                 560

Val Val Asp Val Ser His Glu Asp Pro Glu Val Lys Phe Asn Trp Tyr
                565                 570                 575

Val Asp Gly Val Glu Val His Asn Ala Lys Thr Lys Pro Arg Glu Glu
            580                 585                 590

Gln Tyr Ala Ser Thr Tyr Arg Val Val Ser Val Leu Thr Val Leu His
        595                 600                 605

Gln Asp Trp Leu Asn Gly Lys Glu Tyr Lys Cys Lys Val Ser Asn Lys
    610                 615                 620

Ala Leu Pro Ala Ser Ile Glu Lys Thr Ile Ser Lys Ala Lys Gly Gln
625                 630                 635                 640

Pro Arg Glu Pro Gln Val Tyr Thr Leu Pro Pro Ser Arg Asp Glu Leu
                645                 650                 655

Thr Lys Asn Gln Val Ser Leu Thr Cys Leu Val Lys Gly Phe Tyr Pro
            660                 665                 670

Ser Asp Ile Ala Val Glu Trp Glu Ser Asn Gly Gln Pro Glu Asn Asn

675                 680                 685

Tyr Lys Thr Thr Pro Pro Val Leu Asp Ser Asp Gly Ser Phe Phe Leu
    690                 695                 700

Tyr Ser Lys Leu Thr Val Asp Lys Ser Arg Trp Gln Gln Gly Asn Val
705                 710                 715                 720

Phe Ser Cys Ser Val Leu His Glu Ala Leu His Asn His Tyr Thr Gln
                725                 730                 735

Lys Ser Leu Ser Leu Ser Pro Gly
            740

<210>  9
<211>  5
<212>  PRT
<213>  Artificial Sequence

<220>
<223>  anti-Her2 antibody 1 HCDR1 amino acid sequence

<400>  9

Asp Thr Tyr Ile His
1               5

<210>  10
<211>  17
<212>  PRT
<213>  Artificial Sequence

<220>
<223>  anti-Her2 antibody 1 HCDR2 amino acid sequence

<400>  10

Arg Ile Tyr Pro Thr Asn Gly Tyr Thr Arg Tyr Ala Asp Ser Val Lys
1               5                   10                  15

Gly

<210>  11
<211>  11
<212>  PRT
<213>  Artificial Sequence

<220>
<223>  anti-Her2 antibody 1 HCDR3 amino acid sequence

<400>  11

Trp Gly Gly Asp Gly Phe Tyr Ala Met Asp Tyr
1               5                   10

<210>  12
<211>  11
<212>  PRT
<213>  Artificial Sequence

<220>
<223>  anti-Her2 antibody 1 LCDR1 amino acid sequence

<400>  12

Arg Ala Ser Gln Asp Val Asn Thr Ala Val Ala
1               5                   10


<210>  13
<211>  7
<212>  PRT
<213>  Artificial Sequence

<220>
<223>  anti-Her2 antibody 1 LCDR2 amino acid sequence

<400>  13

Ser Ala Ser Phe Leu Tyr Ser
1               5


<210>  14
<211>  9
<212>  PRT
<213>  Artificial Sequence

<220>
<223>  anti-Her2 antibody 1 LCDR3 amino acid sequence

<400>  14

Gln Gln His Tyr Thr Thr Pro Pro Thr
1               5


<210>  15
<211>  120
<212>  PRT
<213>  Artificial Sequence

<220>
<223>  anti-Her2 antibody 1 VH amino acid sequence

<400>  15

<210>  16
<211>  107
<212>  PRT
<213>  Artificial Sequence

<220>
<223>  anti-Her2 antibody 1 VL amino acid sequence

<400>  16

<210>  17
<211>  5

<212>  PRT
<213>  Artificial Sequence

<220>
<223>  anti-Her2 antibody 2 HCDR1 amino acid sequence

<400>  17

Asp Tyr Thr Met Asp
1               5


<210>  18
<211>  17
<212>  PRT
<213>  Artificial Sequence

<220>
<223>  anti-Her2 antibody 2 HCDR2 amino acid sequence

<400>  18

Asp Val Asn Pro Asn Ser Gly Gly Ser Ile Tyr Asn Gln Arg Phe Lys
1               5                   10                  15


Gly




<210>  19
<211>  10
<212>  PRT
<213>  Artificial Sequence

<220>
<223>  anti-Her2 antibody 2 HCDR3 amino acid sequence

<400>  19

Asn Leu Gly Pro Ser Phe Tyr Phe Asp Tyr
1               5                   10


<210>  20
<211>  11
<212>  PRT
<213>  Artificial Sequence

<220>
<223>  anti-Her2 antibody 2 LCDR1 amino acid sequence

<400>  20

Lys Ala Ser Gln Asp Val Ser Ile Gly Val Ala
1               5                   10


<210>  21
<211>  7
<212>  PRT
<213>  Artificial Sequence

<220>
<223>  anti-Her2 antibody 2 LCDR2 amino acid sequence

<400>  21

Ser Ala Ser Tyr Arg Tyr Thr
1               5


<210>  22
<211>  9
<212>  PRT
<213>  Artificial Sequence

<220>
<223>  anti-Her2 antibody 2 LCDR3 amino acid sequence

<400>  22

Gln Gln Tyr Tyr Ile Tyr Pro Tyr Thr
1               5


<210>  23
<211>  119
<212>  PRT
<213>  Artificial Sequence

<220>
<223>  anti-Her2 antibody 2 VH amino acid sequence

<400>  23

<210>  24
<211>  107
<212>  PRT
<213>  Artificial Sequence

<220>
<223>  anti-Her2 antibody 2 VL amino acid sequence

<400>  24


<210>  25
<211>  5
<212>  PRT
<213>  Artificial Sequence

<220>
<223>  anti-Her2 antibody 3 HCDR1 amino acid sequence

<400>  25

Asp Thr Tyr Ile His
1               5


<210>  26
<211>  17
<212>  PRT
<213>  Artificial Sequence

<220>
<223>  anti-Her2 antibody 3 HCDR2 amino acid sequence

<400>  26

Arg Ile Tyr Pro Thr Asn Gly Tyr Thr Arg Tyr Asp Pro Lys Phe Gln
1               5                   10                  15

Asp

<210>  27
<211>  11
<212>  PRT
<213>  Artificial Sequence

<220>
<223>  anti-Her2 antibody 3 HCDR3 amino acid sequence

<400>  27

Trp Gly Gly Asp Gly Phe Tyr Ala Met Asp Tyr
1               5                   10

<210>  28
<211>  11
<212>  PRT
<213>  Artificial Sequence

<220>
<223>  anti-Her2 antibody 3 LCDR1 amino acid sequence

<400>  28

Lys Ala Ser Gln Asp Val Asn Thr Ala Val Ala
1               5                   10

<210>  29
<211>  7
<212>  PRT
<213>  Artificial Sequence

<220>
<223>  anti-Her2 antibody 3 LCDR2 amino acid sequence

<400>  29

Ser Ala Ser Phe Arg Tyr Thr
1               5

<210>  30
<211>  9
<212>  PRT
<213>  Artificial Sequence

<220>
<223>  anti-Her2 antibody 3 LCDR3 amino acid sequence

<400>  30

Gln Gln His Tyr Thr Thr Pro Pro Thr
1               5

<210>  31
<211>  120
<212>  PRT
<213>  Artificial Sequence

<220>
<223>  anti-Her2 antibody 3 VH amino acid sequence

<400>  31

Gln Val Gln Leu Gln Gln Ser Gly Pro Glu Leu Val Lys Pro Gly Ala
1               5                   10                  15

Ser Leu Lys Leu Ser Cys Thr Ala Ser Gly Phe Asn Ile Lys Asp Thr
            20                  25                  30

Tyr Ile His Trp Val Lys Gln Arg Pro Glu Gln Gly Leu Glu Trp Ile
        35                  40                  45

Gly Arg Ile Tyr Pro Thr Asn Gly Tyr Thr Arg Tyr Asp Pro Lys Phe
    50                  55                  60

Gln Asp Lys Ala Thr Ile Thr Ala Asp Thr Ser Ser Asn Thr Ala Tyr
65                  70                  75                  80

Leu Gln Val Ser Arg Leu Thr Ser Glu Asp Thr Ala Val Tyr Tyr Cys
                85                  90                  95

Ser Arg Trp Gly Gly Asp Gly Phe Tyr Ala Met Asp Tyr Trp Gly Gln
            100                 105                 110

Gly Ala Ser Val Thr Val Ser Ser
        115                 120


<210>  32
<211>  107
<212>  PRT
<213>  Artificial Sequence

<220>
<223>  anti-Her2 antibody 3 VL amino acid sequence

<400>  32

Asp Ile Val Met Thr Gln Ser His Lys Phe Met Ser Thr Ser Val Gly
1               5                   10                  15

Asp Arg Val Ser Ile Thr Cys Lys Ala Ser Gln Asp Val Asn Thr Ala
            20                  25                  30

Val Ala Trp Tyr Gln Gln Lys Pro Gly His Ser Pro Lys Leu Leu Ile

35                  40                  45

Tyr Ser Ala Ser Phe Arg Tyr Thr Gly Val Pro Asp Arg Phe Thr Gly
    50                  55                  60

Ser Arg Ser Gly Thr Asp Phe Thr Phe Thr Ile Ser Ser Val Gln Ala
65                  70                  75                  80

Glu Asp Leu Ala Val Tyr Tyr Cys Gln Gln His Tyr Thr Thr Pro Pro
                85                  90                  95

Thr Phe Gly Gly Gly Thr Lys Val Glu Ile Lys
            100                 105

<210>  33
<211>  5
<212>  PRT
<213>  Artificial Sequence

<220>
<223>  anti-CD3 antibody 3 HCDR1 amino acid sequence

<400>  33

Thr Tyr Ala Met Asn
1               5

<210>  34
<211>  19
<212>  PRT
<213>  Artificial Sequence

<220>
<223>  anti-CD3 antibody 3 HCDR2 amino acid sequence

<400>  34

Arg Ile Arg Ser Lys Tyr Asn Asn Tyr Ala Thr Tyr Tyr Ala Asp Ser
1               5                   10                  15

Val Lys Asp

<210>  35
<211>  14
<212>  PRT
<213>  Artificial Sequence

<220>
<223>  anti-CD3 antibody 3 HCDR3 amino acid sequence

<400>  35

His Gly Asn Phe Gly Asn Ser Tyr Val Ser Trp Phe Ala Tyr
1               5                   10

<210>  36
<211>  14
<212>  PRT
<213>  Artificial Sequence

<220>
<223>  anti-CD3 antibody 3 LCDR1 amino acid sequence

<400>  36

Arg Ser Ser Thr Gly Ala Val Thr Thr Ser Asn Tyr Ala Asn
1               5                   10


<210>  37
<211>  7
<212>  PRT
<213>  Artificial Sequence

<220>
<223>  anti-CD3 antibody 3 LCDR2 amino acid sequence

<400>  37

Gly Thr Asn Lys Arg Ala Pro
1               5


<210>  38
<211>  9
<212>  PRT
<213>  Artificial Sequence

<220>
<223>  anti-CD3 antibody 3 LCDR3 amino acid sequence

<400>  38

Ala Leu Trp Tyr Ser Asn Leu Trp Val
1               5


<210>  39
<211>  125
<212>  PRT
<213>  Artificial Sequence

<220>
<223>  anti-CD3 antibody 3 VH amino acid sequence

<400>  39

Glu Val Gln Leu Leu Glu Ser Gly Gly Gly Leu Val Gln Pro Gly Gly
1               5                   10                  15


Ser Leu Lys Leu Ser Cys Ala Ala Ser Gly Phe Thr Phe Asn Thr Tyr
            20                  25                  30


Ala Met Asn Trp Val Arg Gln Ala Pro Gly Lys Gly Leu Glu Trp Val

35                  40                  45

Ala Arg Ile Arg Ser Lys Tyr Asn Asn Tyr Ala Thr Tyr Tyr Ala Asp
    50                  55                  60

Ser Val Lys Asp Arg Phe Thr Ile Ser Arg Asp Asp Ser Lys Asn Thr
65                  70                  75                  80

Ala Tyr Leu Gln Met Asn Asn Leu Lys Thr Glu Asp Thr Ala Val Tyr
                85                  90                  95

Tyr Cys Val Arg His Gly Asn Phe Gly Asn Ser Tyr Val Ser Trp Phe
            100                 105                 110

Ala Tyr Trp Gly Gln Gly Thr Leu Val Thr Val Ser Ser
        115                 120                 125

<210>  40
<211>  109
<212>  PRT
<213>  Artificial Sequence

<220>
<223>  anti-CD3 antibody 3 VL amino acid sequence

<400>  40

Glu Leu Val Val Thr Gln Glu Pro Ser Leu Thr Val Ser Pro Gly Gly
1               5                   10                  15

Thr Val Thr Leu Thr Cys Arg Ser Ser Thr Gly Ala Val Thr Thr Ser
            20                  25                  30

Asn Tyr Ala Asn Trp Val Gln Gln Lys Pro Gly Gln Ala Pro Arg Gly
        35                  40                  45

Leu Ile Gly Gly Thr Asn Lys Arg Ala Pro Gly Thr Pro Ala Arg Phe
    50                  55                  60

Ser Gly Ser Leu Leu Gly Gly Lys Ala Ala Leu Thr Leu Ser Gly Val
65                  70                  75                  80

Gln Pro Glu Asp Glu Ala Glu Tyr Tyr Cys Ala Leu Trp Tyr Ser Asn
                85                  90                  95

Leu Trp Val Phe Gly Gly Gly Thr Lys Leu Thr Val Leu
            100                 105

<210>  41
<211>  5

<212>  PRT
<213>  Artificial Sequence

<220>
<223>  anti-CD3 antibody 4 HCDR1 amino acid sequence

<400>  41

Lys Tyr Ala Met Asn
1               5


<210>  42
<211>  19
<212>  PRT
<213>  Artificial Sequence

<220>
<223>  anti-CD3 antibody 4 HCDR2 amino acid sequence

<400>  42

Arg Ile Arg Ser Lys Tyr Asn Asn Tyr Ala Thr Tyr Tyr Ala Asp Ser
1               5                   10                  15


Val Lys Asp



<210>  43
<211>  14
<212>  PRT
<213>  Artificial Sequence

<220>
<223>  anti-CD3 antibody 4 HCDR3 amino acid sequence

<400>  43

His Gly Asn Phe Gly Asn Ser Tyr Ile Ser Tyr Trp Ala Tyr
1               5                   10


<210>  44
<211>  14
<212>  PRT
<213>  Artificial Sequence

<220>
<223>  anti-CD3 antibody 4 LCDR1 amino acid sequence

<400>  44

Gly Ser Ser Thr Gly Ala Val Thr Ser Gly Tyr Tyr Pro Asn
1               5                   10


<210>  45
<211>  7
<212>  PRT
<213>  Artificial Sequence

<220>
<223>  anti-CD3 antibody 4 LCDR2 amino acid sequence

<400>  45

Gly Thr Lys Phe Leu Ala Pro
1               5


<210>  46
<211>  9
<212>  PRT
<213>  Artificial Sequence

<220>
<223>  anti-CD3 antibody 4 LCDR3 amino acid sequence

<400>  46

Ala Leu Trp Tyr Ser Asn Arg Trp Val
1               5


<210>  47
<211>  125
<212>  PRT
<213>  Artificial Sequence

<220>
<223>  anti-CD3 antibody 4 VH amino acid sequence

<400>  47

Glu Val Gln Leu Leu Glu Ser Gly Gly Gly Leu Val Gln Pro Gly Gly
1               5                   10                  15

Ser Leu Lys Leu Ser Cys Ala Ala Ser Gly Phe Thr Phe Asn Lys Tyr
            20                  25                  30

Ala Met Asn Trp Val Arg Gln Ala Pro Gly Lys Gly Leu Glu Trp Val
        35                  40                  45

Ala Arg Ile Arg Ser Lys Tyr Asn Asn Tyr Ala Thr Tyr Tyr Ala Asp
    50                  55                  60

Ser Val Lys Asp Arg Phe Thr Ile Ser Arg Asp Asp Ser Lys Asn Thr
65                  70                  75                  80

Ala Tyr Leu Gln Met Asn Asn Leu Lys Thr Glu Asp Thr Ala Val Tyr
                85                  90                  95

Tyr Cys Val Arg His Gly Asn Phe Gly Asn Ser Tyr Ile Ser Tyr Trp
            100                 105                 110

Ala Tyr Trp Gly Gln Gly Thr Leu Val Thr Val Ser Ser
        115                 120                 125

<210>  48
<211>  109
<212>  PRT
<213>  Artificial Sequence

<220>
<223>  anti-CD3 antibody 4 VL amino acid sequence

<400>  48

Glu Leu Val Val Thr Gln Glu Pro Ser Leu Thr Val Ser Pro Gly Gly
1               5                   10                  15

Thr Val Thr Leu Thr Cys Gly Ser Ser Thr Gly Ala Val Thr Ser Gly
            20                  25                  30

Tyr Tyr Pro Asn Trp Val Gln Gln Lys Pro Gly Gln Ala Pro Arg Gly
        35                  40                  45

Leu Ile Gly Gly Thr Lys Phe Leu Ala Pro Gly Thr Pro Ala Arg Phe
    50                  55                  60

Ser Gly Ser Leu Leu Gly Gly Lys Ala Ala Leu Thr Leu Ser Gly Val
65                  70                  75                  80

Gln Pro Glu Asp Glu Ala Glu Tyr Tyr Cys Ala Leu Trp Tyr Ser Asn
                85                  90                  95

Arg Trp Val Phe Gly Gly Gly Thr Lys Leu Thr Val Leu
            100                 105


<210>  49
<211>  28
<212>  PRT
<213>  Homo sapiens

<400>  49

Ser Ser Ser Ser Lys Ala Pro Pro Pro Ser Leu Pro Ser Pro Ser Arg
1               5                   10                  15

Leu Pro Gly Pro Ser Asp Thr Pro Ile Leu Pro Gln
            20                  25


<210>  50
<211>  27
<212>  PRT
<213>  Artificial Sequence

<220>
<223>  G2(GGGGS)3CTP1 linker peptide

<400>  50

Gly Gly Gly Gly Gly Gly Ser Gly Gly Gly Gly Ser Gly Gly Gly Gly
1               5                   10                  15

Ser Ser Ser Ser Ser Lys Ala Pro Pro Pro Ser
            20                  25

<210>  51
<211>  25
<212>  PRT
<213>  Artificial Sequence

<220>
<223>  (GGGGS)3CTP1 linker peptide

<400>  51

Gly Gly Gly Gly Ser Gly Gly Gly Gly Ser Gly Gly Gly Gly Ser Ser
1               5                   10                  15

Ser Ser Ser Lys Ala Pro Pro Pro Ser
            20                  25

<210>  52
<211>  22
<212>  PRT
<213>  Artificial Sequence

<220>
<223>  GS(GGGGS)2CTP1 linker peptide

<400>  52

Gly Ser Gly Gly Gly Gly Ser Gly Gly Gly Gly Ser Ser Ser Ser Ser
1               5                   10                  15

Lys Ala Pro Pro Pro Ser
            20

<210>  53
<211>  33
<212>  PRT
<213>  Artificial Sequence

<220>
<223>  (GGGGS)1CTP4 linker peptide

<400>  53

Gly Gly Gly Gly Ser Ser Ser Ser Ser Lys Ala Pro Pro Pro Ser Leu
1               5                   10                  15

Pro Ser Pro Ser Arg Leu Pro Gly Pro Ser Asp Thr Pro Ile Leu Pro
            20                  25                  30

Gln

<210>  54
<211>  227
<212>  PRT
<213>  Artificial Sequence

<220>
<223>  IgG1 Fc(L234A,L235A) mutant constant region amino acid sequence

<400>  54

Asp Lys Thr His Thr Cys Pro Pro Cys Pro Ala Pro Glu Ala Ala Gly
1               5                   10                  15

Gly Pro Ser Val Phe Leu Phe Pro Pro Lys Pro Lys Asp Thr Leu Met
            20                  25                  30

Ile Ser Arg Thr Pro Glu Val Thr Cys Val Val Val Asp Val Ser His
        35                  40                  45

Glu Asp Pro Glu Val Lys Phe Asn Trp Tyr Val Asp Gly Val Glu Val
    50                  55                  60

His Asn Ala Lys Thr Lys Pro Arg Glu Glu Gln Tyr Asn Ser Thr Tyr
65                  70                  75                  80

Arg Val Val Ser Val Leu Thr Val Leu His Gln Asp Trp Leu Asn Gly
                85                  90                  95

Lys Glu Tyr Lys Cys Lys Val Ser Asn Lys Ala Leu Pro Ala Pro Ile
            100                 105                 110

Glu Lys Thr Ile Ser Lys Ala Lys Gly Gln Pro Arg Glu Pro Gln Val
        115                 120                 125

Tyr Thr Leu Pro Pro Ser Arg Glu Glu Met Thr Lys Asn Gln Val Ser
    130                 135                 140

Leu Thr Cys Leu Val Lys Gly Phe Tyr Pro Ser Asp Ile Ala Val Glu
145                 150                 155                 160

Trp Glu Ser Asn Gly Gln Pro Glu Asn Asn Tyr Lys Thr Thr Pro Pro
                165                 170                 175

Val Leu Asp Ser Asp Gly Ser Phe Phe Leu Tyr Ser Lys Leu Thr Val
            180                 185                 190

Asp Lys Ser Arg Trp Gln Gln Gly Asn Val Phe Ser Cys Ser Val Met
        195                 200                 205

His Glu Ala Leu His Asn His Tyr Thr Gln Lys Ser Leu Ser Leu Ser
    210                 215                 220

Pro Gly Lys
225

<210>  55
<211>  226
<212>  PRT
<213>  Artificial Sequence

<220>
<223>  IgG1(L234A,L235A, T250Q,N297A,P331S,M428L,K447-) mutant constant
       region amino acid sequence

<400>  55

Asp Lys Thr His Thr Cys Pro Pro Cys Pro Ala Pro Glu Ala Ala Gly
1               5                   10                  15

Gly Pro Ser Val Phe Leu Phe Pro Pro Lys Pro Lys Asp Gln Leu Met
            20                  25                  30

Ile Ser Arg Thr Pro Glu Val Thr Cys Val Val Val Asp Val Ser His
        35                  40                  45

Glu Asp Pro Glu Val Lys Phe Asn Trp Tyr Val Asp Gly Val Glu Val
    50                  55                  60

His Asn Ala Lys Thr Lys Pro Arg Glu Glu Gln Tyr Ala Ser Thr Tyr
65                  70                  75                  80

Arg Val Val Ser Val Leu Thr Val Leu His Gln Asp Trp Leu Asn Gly
                85                  90                  95

Lys Glu Tyr Lys Cys Lys Val Ser Asn Lys Ala Leu Pro Ala Ser Ile
            100                 105                 110

Glu Lys Thr Ile Ser Lys Ala Lys Gly Gln Pro Arg Glu Pro Gln Val
        115                 120                 125

Tyr Thr Leu Pro Pro Ser Arg Asp Glu Leu Thr Lys Asn Gln Val Ser
    130                 135                 140

Leu Thr Cys Leu Val Lys Gly Phe Tyr Pro Ser Asp Ile Ala Val Glu
145                 150                 155                 160

Trp Glu Ser Asn Gly Gln Pro Glu Asn Asn Tyr Lys Thr Thr Pro Pro
                165                 170                 175

Val Leu Asp Ser Asp Gly Ser Phe Phe Leu Tyr Ser Lys Leu Thr Val
            180                 185                 190

Asp Lys Ser Arg Trp Gln Gln Gly Asn Val Phe Ser Cys Ser Val Leu
        195                 200                 205

His Glu Ala Leu His Asn His Tyr Thr Gln Lys Ser Leu Ser Leu Ser
    210                 215                 220

Pro Gly
225

<210> 56
<211> 2232
<212> DNA
<213> Artificial Sequence

<220>
<223> bispecific antibody AB7K7 nucleotide sequence

<400> 56
gaggtgcagc tcgtagaatc aggaggtggc ctggtccagc ccgggggatc actgaggctg 60

tcctgtgctg cctcaggctt caacattaag gatacctaca tccattgggt ccgccaggct 120

ccaggaaaag ggcttgagtg ggtggctagg atctatccaa caaatggata tacaaggtat 180

gccgactctg tcaaaggccg ctttactatt tcagccgata caagcaagaa cactgcttac 240

ttgcagatga actctctgcg tgctgaagac acagcagtat actattgctc aaggtggggt 300

ggtgacgggt tttacgcaat ggattactgg ggtcagggca ccttggtgac cgtatcttct 360

ggaggaggag gatctggagg aggaggctcc ggaggaggag gaagcgacat tcaaatgaca 420

caatccccct catccctcag cgcctccgtg ggggataggg tcacaataac ctgccgtgcc 480

tcacaggacg tgaacaccgc agtggcatgg tatcagcaaa agccagggaa ggctccaaag 540

ctcctcatct acagcgccag cttcctctat agtggagtcc cttcacgatt ctctggtagc 600

cgctctggga cagactttac cctgactatt agcagtctgc agcctgagga ttttgcaact 660

tattactgtc agcagcacta caccacacca ccaacctttg gacaaggcac caaagtggaa 720

atcaagggtg gcggcggtgg aggatccggc ggtggaggta gcggcggagg cggtagctcc 780

agctctagta aagctccccc tccttccgag gtgcagctgc tggagtccgg aggaggactg 840

gtgcagccag gaggctccct gaagctgagc tgtgctgcct ctggctttac cttcaacaca 900

tatgccatga attgggtgcg gcaggctcca ggcaagggac tggagtgggt ggctaggatc 960

aggtctaagt acaacaatta tgccacctac tatgctgatt ccgtgaagga caggttcacc 1020

atctcccgcg acgatagcaa gaacacagcc tacctgcaga tgaacaatct gaagaccgag 1080

gataccgccg tgtactactg cgtgagacat ggcaactttg gcaatagcta cgtgtcctgg 1140

ttcgcttact ggggacaggg caccctggtc acagtgagct ctggaggagg aggatctgga 1200

ggaggaggct ccggaggagg aggaagcgag ctggtggtga cccaggagcc atctctgaca 1260

gtgtcccccg gcggcacagt gaccctgaca tgtagatcca gcaccggcgc cgtgaccaca 1320

tccaactacg ctaattgggt gcagcagaag ccaggacagg ctccaagggg actgatcgga 1380

ggaaccaaca agagggctcc tggaacacca gctcggttta gcggatctct gctgggaggc 1440

aaggctgccc tgaccctgtc cggagtgcag ccagaggatg aggccgagta ttattgcgct 1500

ctgtggtata gcaatctgtg ggtgttcgga ggaggaacca agctgacagt gctggacaag 1560

acccatacat gcccaccatg ccctgcccct gaagccgccg gaggaccttc cgtgttcctg 1620

ttccctccca agccaaaaga tcagctgatg atctctagaa cccccgaagt cacctgcgtg 1680

gtcgtcgacg tgtcccatga ggaccctgaa gtcaagttca actggtacgt ggacggtgtc 1740

gaagtccaca acgccaagac caagcctagg gaggagcagt atgccagcac ataccgggtg 1800

gtgtctgtgc tgaccgtgct gcatcaggat tggctgaatg gcaaggaata taaatgtaag 1860

gtgagcaata aggctctgcc ggctagcatt gaaaaaacca tttccaaggc taagggccag 1920

cccagggagc ctcaggtcta caccctgcct ccatctagag atgaactgac caaaaaccag 1980

gtgagcctga cttgcctggt caaaggcttc taccccagcg acattgccgt ggagtgggag 2040

tctaatggcc agcctgaaaa taactacaaa actacccctc ctgtgctgga ctctgatggc 2100

tccttctttc tgtactctaa actgaccgtg gacaagtctc gctggcagca gggtaacgtg 2160

ttttcttgct ccgtgctgca cgaggctctg cataaccatt acacccagaa gagcctgtct 2220

ctgtccccag ga 2232

## Description

### CROSS-REFERENCE TO RELATED APPLICATION(S)

This application claims priority to Chinese Patent Application No. 201811294887.4 filed Nov. 1, 2018, the disclosure of which is incorporated herein by reference in its entirety.

### TECHNICAL FIELD

The present disclosure relates to the field of immunology and, in particular, to a bispecific antibody against human epidermal growth factor receptor 2 (Her2) and cluster of differentiation 3 (CD3) and a use thereof, especially a use thereof for treating tumors.

### BACKGROUND

### I. Bispecific antibody

In 1985, the concept of killing tumor cells using T cells was proposed (Stearz UD et al., Nature, 314: 628-631, 1985). It is generally believed that the effective activation of T cells requires a dual signal, in which the first signal comes from the binding of the MHC-antigen complex to the T-cell receptor TCR-CD3 on the antigen-presenting cell and the second signal is a non-antigen-specific costimulatory signal resulting from the interaction of the T cell with a costimulatory molecule expressed by the antigen-presenting cell. Due to the down-regulated expression or even the deletion of MHC on the surface of most tumor cells, the tumor cells can escape the immune killing.

The bispecific antibodies can be classified according to the action mechanism into dual signal blocking type and cell-mediated functional type. Generally, the cell-mediated functional bispecific antibody refers to the anti-CD3 bispecific antibody that mediates the T-cell killing. The CD3 molecule is expressed on the surface of all mature T cells, and non-covalently binds to the TCR to form an intact TCR-CD3 complex, which jointly participates in the immune response to antigen stimulation and is the most used and the most successful trigger molecule on the surface of immune effector cells among bispecific antibodies. The bispecific antibody targeting CD3 can bind to the T cell surface CD3 and the tumor cell surface antigen, respectively, thus shortening the distance between cytotoxic T cells (Tc or CTL) and tumor cells and directly activating T cells to induce T cells to directly kill cancer cells instead of relying on the conventional dual activation signal of T cells. However, the agonistic antibody targeting the T cell antigen CD3, for example, the first generation of mouse monoclonal antibody OKT3 targeting human CD3 applied to the clinical practice (Kung P et al., Science, 206: 347-349, 1979), releases a large number of inflammatory factors such as interleukin-2 (IL-2), TNF-α, IFN-γ, and interleukin-6 (IL-6) due to the hyperactivation of T cells, which clinically causes a severe "cytokine storm syndrome" (Hirsch R et al., J. Immunol., 142: 737-743, 1989) and thus resulting in "influenza-like" symptoms characterized by fever, chills, headache, nausea, vomiting, diarrhea, respiratory distress, aseptic meningitis, and hypotension. Thus, how to attenuate or avoid the excessive cytokine storm is a priority for the development of bifunctional antibodies targeting CD3.

In recent years, to solve the problem of correctly assembling two different half-antibodies, scientists have designed and developed bispecific antibodies of various structures. Overall, there are two categories. The one is that the bispecific antibody does not include an Fc region. The advantages of bispecific antibodies in such a structure are that they have a small molecular weight, can be expressed in prokaryotic cells, and do not need to be correctly assembled, while their disadvantages are that due to the lack of the antibody Fc fragment and the relatively low molecular weight, they have a short half-life period and that such bispecific antibodies are highly susceptible to polymerization, and thus have poor stability and low expression, and thus are limited in the clinical application. Such bispecific antibodies that have been reported so far include BiTE, DART, TrandAbs, bi-Nanobody, etc.

The other is that the bispecific antibody retains an Fc domain. Such bispecific antibodies form an IgG-like structure with a larger molecular structure and have a longer half-life period due to the FcRn-mediated endocytosis and recycling process; meanwhile, they also retain some or all of the Fc-mediated effector functions, such as antibody-dependent cell-mediated cytotoxicity (ADCC), complement-dependent cytotoxicity (CDC), and antibody-dependent cellular phagocytosis (ADCP). Such bispecific antibodies that have been reported so far include Triomabs, kih IgG, Crossmab, orthoFab IgG, DVD IgG, IgG scFv, scFv₂-Fc, etc. As for anti-CD3 bispecific antibodies, currently, except for configurations TandAb and scFv-Fv-scFv, other anti-CD3 bispecific antibodies are widely designed in the form of univalent anti-CD3, mainly because bivalent anti-CD3 bispecific antibodies can easily lead to hyperactivation and thus induce T cell apoptosis and massive transient release of cytokines (Kuhn C et al, Immunotherapy, 8: 889-906, 2016) and, more seriously, they may also trigger non-antigen-dependent activation of T cells and thus disrupt immune homeostasis. Therefore, most of the anti-CD3 bispecific antibodies in the existing art avoid the introduction of bivalent anti-CD3 antibodies. For example, bispecific antibodies in configurations of triFab-Fc, DART-Fc, and BiTE-Fc are designed asymmetrically (that is, heterodimer-type bispecific antibodies) (Z Wu et al, Pharmacology and Therapeutics, 182: 161-175, 2018), but such a design poses many challenges for downstream production of such heterodimeric bispecific antibodies, such as the generation of undesired homodimers or mismatched impurity molecules, which increase the difficulty of expression and purification of bispecific antibodies. Although the use of the "knobs-into-holes" technique to some extent solves the problem of inter-heavy chain mismatching of heterodimeric bispecific antibody molecules, "light chain/heavy chain mismatching" brings about another challenge. One strategy for preventing heavy chain-light chain mismatching is to interchange the partial domains of the light chain and heavy chain of one of Fabs of a bispecific antibody to form a Crossmab (a hybrid antibody), which can allow selective pairing between the light chains/heavy chains. However, the disadvantages of this method are that the generation of mismatching products cannot be completely prevented, and residual fractions of any mismatching molecule are difficult to separate from the products, and in addition, this method requires a large number of genetic engineering modifications such as mutations for two antibody sequences. Thus, this method cannot become simple and universal.

Furthermore, for bispecific antibodies including CD3-specific IgG-like structures, such bispecific antibodies may cause unrestricted long-lasting T-cell activation due to their ability to bind to FcyR, and such activation is non-target cell-restricted, that is, activated T-cells can be found in tissues expressing FcyR (e.g., in hematopoietic, lymphoid, and reticuloendothelial systems), whether or not such bispecific antibodies bind to the target antigen. The systemic activation of such T cells will be accompanied by a substantial release of cytokines, which is a serious adverse effect during the therapeutic application of T cells-activated cytokines or antibodies. Therefore, such anti-CD3 bispecific antibodies that mediate the T cell killing need to avoid Fc-mediated systemic activation of T cells, thus allowing immune effector cells to be restrictedly activated within target cell tissues, that is, relying exclusively on the binding of the bispecific antibodies to the corresponding target antigens.

### II. Human epidermal growth factor receptor 2 (Her2/ErbB2)

The epidermal growth factor receptor (ErbB) family plays an important role in cell growth, development and differentiation processes. The overexpression of the ErbB family leads to the disorder of normal cell functions and is closely related to the occurrence and development of tumors. The overexpression of human epidermal growth factor receptor 2 (Her2/ErbB2) is closely related to the degree of malignancy of many epithelial cell carcinomas. Tumors expressing high level of Her2 exhibit the characteristics of strong migration and infiltration, poor sensitivity to chemotherapeutic drugs, poor prognosis and easy recurrence. Her2 is expressed at an extremely low level in normal cells and at a high level during embryonic development. Her2 can spontaneously form homodimeric receptors or be induced by ligands to form heterodimeric receptors and trigger signal transduction networks (Arteaga CL et al., Cancer Cell, 2014, 25: 282-303; Roskoski R Jr, Pharmacol Res, 2014, 79: 34-74) and plays an important regulatory role in cell proliferation, differentiation, development, adhesion, and migration. Her2 is overexpressed in nearly 20% to 25% of patients with primary breast cancer. In addition to breast cancer, Her2 is overexpressed in ovarian, gastric, colorectal, prostate and lung cancer and the like.

In 1998, the U.S. FDA approved the first monoclonal antibody drug, trastuzumab (Herceptin®), for the clinical treatment of Her2 positive metastatic breast cancer (MBC). The antibody recognizes a membrane-proximal epitope in the extracellular region IV of Her2 and inhibits the Her-2-mediated signal transduction system by blocking Her-2 receptor dimerization on one hand and activates an ADCC effect to kill tumor cells on the other hand. At present, trastuzumab has become the standard treatment for Her-2 positive operable breast cancer (Romond EH et al., N Engl J Med, 2005, 353(16): 1673-1684; Perez EA et al., J Clin Oncol, 2011, 29(34): 4491-4497). However, it has low efficacy in treating breast cancer patients with the low expression of Her2, and a considerable number of patients who are treated effectively with the antibody initially will develop resistance within one year (Thery JC et al., Eur J Cancer, 2014, 50: 892-901). Thus, to improve efficacy, an anti-Her2 therapy is generally used in combination with chemotherapeutic drugs in clinics. Clinical data has demonstrated that trastuzumab has synergistic effects with platinum, docetaxel, and vinorelbine, and additive effects with doxorubicin, paclitaxel, and cyclophosphamide. The side effects of trastuzumab are of great concern because of the expression of Her2 in various tissues and organs. Common side effects include fever, nausea, diarrhea, rash, headache and the like. The most serious side effect is cardiac toxicity of trastuzumab. The incidence of cardiac functional and pathological abnormalities is about 3% when trastuzumab is used alone and 26 % to 28% when trastuzumab is used in combination with anthracycline (which is significantly higher than the incidence of 6.0 % to 9.6% when anthracycline is used alone). Therefore, a novel anti-Her2 drug with better efficacy and fewer adverse reactions needs to be developed in clinics.

In view of the problems of a low response rate, a high dosage in clinical administration and relatively large side effects such as cardiac toxicity of Herceptin®, several novel Her2×CD3 bispecific antibodies have been developed by using variable region sequences of Her2, so as to improve therapeutic efficiency, reduce dosage in administration and reduce clinical side effects. For example, CN104558192A has disclosed an anti-Her2×CD3 bispecific antibody with a configuration of a monomer and ScFv-Fc bispecific antibody (MSBODY), wherein the monovalent unit has a variable region sequence from Herceptin® and contains Fab and Fc domains, and the single-chain unit is in the form of anti-CD3 ScFv-Fc. Both the heavy chain Fc of the monovalent unit and the Fc region of the single-chain unit are modified to make it difficult for the monovalent unit and the single-chain unit to form homodimers and easy to form heterodimers. However, inevitably, a small number of homodimer antibody by-products formed due to the mismatches between heavy chains are still produced, which increases the difficulty in purification and separation. In addition, the anti-CD3 single-chain antibody of the anti-Her2×CD3 bispecific antibody fuses the heavy chain Fc of human IgG1 and retains an FcR binding capacity, which certainly increases the risk of cytokine storms induced thereby.

Although a bispecific antibody targeting Her2×CD3 has been disclosed, its safety and efficacy still need to be improved. For example, a humanized antibody is used to avoid HAMA reactions, and CD3 antibody molecules are modified to reduce the risk of cytokine storms induced thereby. However, it is unpredictable whether a dual-function antibody formed by combining an anti-Her2 antibody and an anti-CD3 antibody has expected biological functions and can effectively kill tumor cells related to breast cancer and the like. In the bispecific antibody, two functional domains are required to correctly bind to their binding sites. Even if every antibody can bind to its antigen correctly, it cannot be guaranteed that a bispecific antibody formed by two antibodies can retain original binding abilities, and the synergistic effect of the bispecific antibody cannot be guaranteed due to steric hindrance.

To conclude, there is still a need in the art to develop a multi-specific molecule against Her2xCD3 that is more suitable for clinical application and capable of targeting and killing Her2-expressing cancer cells with high specificity, especially tumor cells with the low expression of Her2, and a novel bispecific molecule with improved performance in terms of half-life, stability, safety and manufacturability.

### SUMMARY

An object of the present disclosure is to provide a tetravalent, homodimer-type bispecific antibody molecule targeting immune effector cell antigen CD3 and Her2. The bispecific antibody can significantly inhibit or kill tumor cells *in vivo*, and has significantly reduced non-specific killing effect for normal cells with the low expression of Her2, controlled toxic side effects due to the over-activation of effector cells, and significantly improved physicochemical and *in vivo* stability.

In a first aspect of the present disclosure, there is provided a bispecific antibody, which is a tetravalent homodimer formed by two identical polypeptide chains that bind to each other by a covalent bond, wherein each of the polypeptide chains includes a first single-chain Fv that specifically binds to Her2 (anti-Her2 scFv), a second single-chain Fv that specifically binds to effector cell antigen CD3 (anti-CD3 scFv), and an Fc fragment in sequence from N-terminus to C-terminus; wherein the first single-chain Fv is linked to the second single-chain Fv by a linker peptide, the second single-chain Fv is linked to the Fc fragment directly or by a linker peptide, and the Fc fragment has no effector functions.

The first single-chain Fv has specificity to Her2 and includes a VH domain and a VL domain linked by a linker peptide (L1), wherein VH, L1 and VL are arranged in order of VH-L1-VL or VL-L1-VH and the linker peptide L1 has an amino acid sequence of (GGGGX)ₙ, wherein X includes Ser or Ala, preferably Ser, and n is a natural number from 1 to 5, preferably 3.

For example, some preferred amino acid sequences of the VH domain and its complementarity determining regions (HCDR1, HCDR2 and HCDR3) and amino acid sequences of the VL domain and its complementarity determining regions (LCDR1, LCDR2 and LCDR3) of the anti-Her2 scFv are exemplarily listed in Table 5-9 of the present disclosure.

Preferably, the complementarity determining regions (HCDR1, HCDR2 and HCDR3) of the VH domain and the complementarity determining regions (LCDR1, LCDR2 and LCDR3) of the VL domain of the first single-chain Fv against Her2 are selected from the group consisting of:
(i) a VH domain that contains HCDR1, HCDR2 and HCDR3 as shown in SEQ ID NOs: 9, 10 and 11, respectively or having sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 9, 10 and 11; and a VL domain that contains LCDR1, LCDR2 and LCDR3 as shown in SEQ ID NOs: 12, 13 and 14, respectively or having sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 12, 13 and 14;
(ii) a VH domain that contains HCDR1, HCDR2 and HCDR3 as shown in SEQ ID NOs: 17, 18 and 19, respectively or having sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 17, 18 and 19; and a VL domain that contains LCDR1, LCDR2 and LCDR3 as shown in SEQ ID NOs: 20, 21 and 22, respectively or having sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 20, 21 and 22; and
(iii) a VH domain that contains HCDR1, HCDR2 and HCDR3 as shown in SEQ ID NOs: 25, 26 and 27, respectively or having sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 25, 26 and 27; and a VL domain that contains LCDR1, LCDR2 and LCDR3 as shown in SEQ ID NOs: 28, 29 and 30, respectively or having sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 28, 29 and 30.

More preferably, the first single-chain Fv against Her2 comprises a VH domain and a VL domain selected from the group consisting of:
(i) a VH domain comprising an amino acid sequence as shown in SEQ ID NO: 15 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 15; and a VL domain comprising an amino acid sequence as shown in SEQ ID NO: 16 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 16;
(ii) a VH domain comprising an amino acid sequence as shown in SEQ ID NO: 23 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 23; and a VL domain comprising an amino acid sequence as shown in SEQ ID NO: 24 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 24; and
(iii) a VH domain comprising an amino acid sequence as shown in SEQ ID NO: 31 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 31; and a VL domain comprising an amino acid sequence as shown in SEQ ID NO: 32 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 32.

The linker peptide (L2) that links the first single-chain Fv and the second single-chain Fv of the present disclosure consists of a flexible peptide and a rigid peptide.

Further, the flexible peptide includes two or more amino acids, and preferably selected from the following amino acids: Gly(G), Ser(S), Ala(A) and Thr(T). More preferably, the flexible peptide includes G and S residues. Most preferably, an amino acid composition structure of the flexible peptide has a general formula of GₓS_{y}(GGGGS)_{z}, wherein x, y and z are integers greater than or equal to 0 and x + y + z ≥ 1. For example, in a preferred embodiment, the amino acid sequence of the flexible peptide is G₂(GGGGS)₃.

Further, the rigid peptide is derived from a full-length sequence (as shown in SEQ ID NO: 49) consisting of amino acids 118 to 145 at carboxyl-terminus of natural human chorionic gonadotropin β-subunit or a truncated fragment thereof (hereinafter collectively referred to as CTP). Preferably, the CTP rigid peptide includes 10 amino acids at N-terminus of SEQ ID NO: 49, that is, SSSSKAPPPS (CTP¹); or the CTP rigid peptide includes 14 amino acids at C-terminus of SEQ ID NO: 49, that is, SRLPGPSDTPILPQ (CTP²); as another example, in another embodiment, the CTP rigid peptide includes 16 amino acids at the N-terminus of SEQ ID NO: 49, that is, SSSSKAPPPSLPSPSR (CTP³); as another example, in some other embodiments, the CTP rigid peptide includes 28 amino acids which begin at position 118 and end at position 145 of the human chorionic gonadotropin β-subunit, that is, SSSSKAPPPSLPSPSRLPGPSDTPILPQ (CTP⁴).

For example, some preferred amino acid sequences of the linker peptide L2 that links the first single-chain Fv and the second single-chain Fv are exemplarily listed in Table 5-11 of the present disclosure.

In a preferred embodiment of the present disclosure, the linker peptide has an amino acid sequence as shown in SEQ ID NO: 50, wherein the amino acid composition of the flexible peptide is G₂(GGGGS)₃, and the amino acid composition of the rigid peptide is SSSSKAPPPS (i.e. CTP¹).

The second single-chain Fv has specificity to immune effector cell antigen CD3 and includes a VH domain and a VL domain linked by a linker peptide (L3), wherein VH, L3 and VL are arranged in order of VH-L3-VL or VL-L3-VH, and the linker peptide L3 has an amino acid sequence of (GGGGX)ₙ, wherein X includes Ser or Ala, preferably Ser; and n is a natural number from 1 to 5, preferably 3;

Preferably, the second single-chain Fv of the bispecific antibody binds to effector cells at an EC₅₀ value greater than about 50 nM, or greater than 100 nM, or greater than 300 nM, or greater than 500 nM in an *in vitro* FACS binding assay; more preferably, the second single-chain Fv of the bispecific antibody is capable of binding to human CD3 and specifically binding to CD3 of a cynomolgus monkey or a rhesus monkey. In a preferred embodiment of the present disclosure, the bispecific antibody specifically binds to effector cells at an EC₅₀ value of 132.3 nM.

For example, some preferred amino acid sequences of the VH domain and its complementarity determining regions (HCDR1, HCDR2 and HCDR3) and amino acid sequences of the VL domain and its complementarity determining regions (LCDR1, LCDR2 and LCDR3) of the anti-CD3 scFv are exemplarily listed in Table 5-10 of the present disclosure.

Preferably, the second single-chain Fv specifically binds to CD3; the VH domain of the second single-chain Fv contains HCDR1, HCDR2 and HCDR3 as shown in SEQ ID NOs: 33, 34 and 35, respectively or has sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 33, 34 and 35; and the VL domain of the second single-chain Fv contains LCDR1, LCDR2 and LCDR3 as shown in SEQ ID NOs: 36, 37 and 38, respectively or has sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 36, 37 and 38.

Preferably, the second single-chain Fv specifically binds to CD3; the VH domain of the second single-chain Fv contains HCDR1, HCDR2 and HCDR3 as shown in SEQ ID NOs: 41, 42 and 43, respectively or has sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 41, 42 and 43; and the VL domain of the second single-chain Fv contains LCDR1, LCDR2 and LCDR3 as shown in SEQ ID NOs: 44, 45 and 46, respectively or has sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 44, 45 and 46.

More preferably, the second single-chain Fv specifically binds to CD3; the VH domain of the second single-chain Fv contains an amino acid sequence as shown in SEQ ID NO: 39 or has a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 39; and the VL domain of the second single-chain Fv contains an amino acid sequence as shown in SEQ ID NO: 40 or has a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 40.

More preferably, the second single-chain Fv specifically binds to CD3; the VH domain of the second single-chain Fv contains an amino acid sequence as shown in SEQ ID NO: 47 or has a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 47; and the VL domain of the second single-chain Fv contains an amino acid sequence as shown in SEQ ID NO: 48 or has a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 48.

The Fc fragment of the present disclosure is linked to the second single-chain Fv directly or by a linker peptide L4, and the linker peptide L4 includes 1-20 amino acids, and preferably selected from the following amino acids: Gly(G), Ser(S), Ala(A) and Thr(T), preferably the linker peptide L4 selected from Gly (G) and Ser (S); more preferably, the linker peptide L4 consists of (GGGGS)n, wherein n = 1, 2, 3 or 4. In a preferred embodiment of the disclosure, the Fc fragment is directly linked to the second single-chain Fv.

In another aspect, the Fc fragment of the present disclosure includes a hinge region, a CH2 domain and a CH3 domain from a human immunoglobulin heavy chain constant region. For example, in some embodiments, the Fc fragment of the present disclosure is selected from, for example, heavy chain constant regions of human IgG1, IgG2, IgG3, IgG4, IgM, IgA1, IgA2, IgD and IgE; particularly selected from heavy chain constant regions of human IgG1, IgG2, IgG3 and IgG4, and more particularly selected from a heavy chain constant region of human IgG1 or IgG4; in addition, the Fc fragment has one or more amino acid substitutions, deletions or additions (for example, at most 20, at most 15, at most 10, or at most 5 substitutions, deletions, or additions) than the natural sequence from which the Fc fragment is derived.

In some preferred embodiments, the Fc fragment is changed, for example, mutated to modify the properties of the bispecific antibody molecule of the present disclosure (for example, to change one or more of the following properties: Fc receptor binding, antibody glycosylation, an effector cell function or a complement function).

For example, the bispecific antibody provided by the present disclosure includes an Fc variant containing amino acid substitutions, deletions or additions that change (for example, reduce or eliminate) effector functions. Fc region of the antibody mediates several important effector functions such as ADCC, ADCP and CDC. Methods for changing the affinity of the antibody to an effector ligand (such as FcyR or a complement C1q) by substituting amino acid residues in the Fc region of the antibody to change the effector functions are known in the art (see, for example, EP388151A1; US5648260; US5624821; Natsume A et al., Cancer Res., 68: 3863-3872, 2008; Idusogie EE et al., J. Immunol., 166: 2571-2575, 2001; Lazar GA et al., PNAS, 103: 4005-4010, 2006; Shields RL et al., JBC, 276: 6591-6604, 2001; Stavenhagen JB et al., Cancer Res., 67: 8882-8890, 2007; Stavenhagen JB et al., Advan. Enzyme. Regul., 48: 152-164, 2008; Alegre ML et al., J. Immunol., 148: 3461-3468, 1992; Kaneko E et al., Biodrugs, 25: 1-11, 2011). In some preferred embodiments of the present disclosure, amino acid L235 (EU numbering) in the constant region of the antibody is modified to change an interaction with an Fc receptor, such as L235E or L235A. In some other preferred embodiments, amino acids 234 and 235 in the constant region of the antibody are modified simultaneously, such as L234A and L235A (L234A/L235A) (according to EU numbering, with an amino acid sequence as shown in SEQ ID NO: 54).

For example, the bispecific antibody provided by the present disclosure may include an Fc variant containing amino acid substitutions, deletions or additions that extend a circulating half-life. Studies show that M252Y/S254T/T256E, M428L/N434S or T250Q/M428L can extend the half-life of the antibody in primates. For more mutation sites included in the Fc variant with enhanced binding affinity to neonatal receptor (FcRn), see Chinese invention patent CN 201280066663.2, US 2005/0014934A1, WO 97/43316, US 5,869,046, US 5,747,03 and WO 96/32478. In some preferred embodiments of the present disclosure, amino acid M428 (EU numbering) in the constant region of the antibody is modified to enhance the binding affinity to the FcRn receptor, such as M428L. In some other preferred embodiments, amino acids 250 and 428 (EU numbering) in the constant region of the antibody are modified simultaneously, such as T250Q and M428L (T250Q/M428L).

For example, the bispecific antibody provided by the present disclosure may also include an Fc variant containing amino acid substitutions, deletions or additions that may reduce or eliminate Fc glycosylation. For example, the Fc variant contains reduced glycosylation of the N-linked glycan normally present at amino acid site 297 (EU numbering). The glycosylation at position N297 has a great effect on the activity of IgG. If the glycosylation at this position is eliminated, the conformation of the upper half of CH2 of an IgG molecule is affected, thus losing the ability of binding to FcyRs and affecting the biological activity related to the antibody. In some preferred embodiments of the present disclosure, amino acid N297 (EU numbering) in the constant region of human IgG is modified to avoid the glycosylation of the antibody, such as N297A.

For example, the bispecific antibody provided by the present disclosure may also include an Fc variant containing amino acid substitutions, deletions or additions that eliminate charge heterogeneity. Various post-translational modifications during expression in engineered cells will cause the charge heterogeneity of monoclonal antibodies. The heterogeneity of lysine at C-terminus of an IgG antibody is one of the main reasons for charge heterogeneity. Lysine at C-terminus of a heavy chain may be deleted at a certain proportion during the production of the antibody, resulting in charge heterogeneity and affecting the stability, effectiveness, immunogenicity or pharmacokinetic of the antibody. In some preferred embodiments of the present disclosure, K447 (EU numbering) at the C-terminus of the IgG antibody is removed or deleted to eliminate the charge heterogeneity of the antibody and improve the homogeneity of the expressed product.

The amino acid sequences of some preferred Fc fragments are exemplarily listed in Table 5-12 of the present disclosure. Compared with a bispecific antibody containing the Fc region of wild-type human IgG, the bispecific antibody provided by the present disclosure contains an Fc fragment that exhibits reduced affinity to at least one of human FcγRs (FcγRI, FcγRIIa, or FcγRIIIa) and C1q, and has reduced effector cell functions or complement functions. For example, in a preferred embodiment of the present disclosure, the bispecific antibody includes an Fc fragment that is from human IgG1, has L234A and L235A substitutions (L234A/L235A), and exhibits reduced binding ability to FcγRI. In addition, the Fc fragment included in the bispecific antibody provided by the present disclosure may also contain amino acid substitutions that change one or more other characteristics (such as an ability of binding to the FcRn receptor, the glycosylation of the antibody or the charge heterogeneity of the antibody). For example, in a preferred embodiment of the present disclosure, the Fc fragment has an amino acid sequence as shown in SEQ ID NO: 55, which has amino acid substitutions L234A/L235A/T250Q/N297A/P331 S/M428L and a deleted or removed K447 compared with the natural sequence from which it is derived.

In a preferred embodiment of the present disclosure, the bispecific antibody binds to human Her2 and CD3 and has an amino acid sequence as follows:
(i) a sequence as shown in SEQ ID NO: 8;
(ii) a sequence having one or more substitutions, deletions or additions (such as 1, 2, 3, 4 or 5 substitutions, deletions or additions) relative to the sequence as shown in SEQ ID NO: 8; or
(iii) a sequence having at least 80%, at least 85%, at least 90%, at least 91%, at least 92%, at least 93%, at least 94%, at least 95%, at least 96%, at least 97%, at least 98%, at least 99% or 100% sequence identity relative to the sequence as shown in SEQ ID NO: 8;

In some preferred embodiments, the substitutions in (ii) are conservative substitutions.

In a second aspect of the present disclosure, there is provided a DNA molecule encoding the bispecific antibody as described above.

In a preferred embodiment of the present disclosure, the DNA molecule encoding the bispecific antibody as described above has a nucleotide sequence as shown in SEQ ID NO: 56.

In a third aspect of the present disclosure, there is provided a vector including the DNA molecule as described above.

In a fourth aspect of the present disclosure, there is provided a host cell including the vector as described above; the host cell includes a prokaryotic cell, a yeast or a mammalian cell, such as a CHO cell, an NS0 cell or another mammalian cell, preferably a CHO cell.

In a fifth aspect of the present disclosure, there is provided a pharmaceutical composition including the bispecific antibody as described above and a pharmaceutically acceptable excipient, carrier or diluent.

Exemplarily, in a preferred embodiment of the present disclosure, there is provided a stable solution formulation including the bispecific antibody as described above. The solution formulation further includes a pH regulator, a stabilizer, and a surfactant; preferably, the pH regulator is a citrate buffer or a histidine buffer, the stabilizer is sucrose, and the surfactant is tween-80; more preferably, the formulation includes 0.5 mg/mL of the bispecific antibody, 20 mM of citrate or histidine, 8% (w/v) of sucrose and 0.02% (w/v) of PS80; and the formulation has a pH of 5.5.

In a sixth aspect of the present disclosure, there is further provided a method for preparing the bispecific antibody of the present disclosure, comprising: (a) obtaining a fusion gene of the bispecific antibody, and constructing an expression vector of the bispecific antibody; (b) transfecting the expression vector into a host cell by a genetic engineering method; (c) culturing the host cell under conditions that allow the bispecific antibody to be generated; (d) separating and purifying the generated antibody.

The expression vector in step (a) is one or more selected from a plasmid, a bacterium and a virus; preferably, the expression vector is PCDNA3.1;
The host cell into which the constructed vector is transfected by the genetic engineering method in step (b) includes a prokaryotic cell, a yeast or a mammalian cell, such as a CHO cell, an NS0 cell or another mammalian cell, preferably a CHO cell.

The bispecific antibody is separated and purified in step (d) by a conventional immunoglobulin purification method including protein A affinity chromatography and ion exchange, hydrophobic chromatography or molecular sieve.

In a seventh aspect of the present disclosure, there is provided use of the bispecific antibody for preparing a drug for treating, preventing or alleviating a tumor; examples of the tumor include, but are not limited to, breast cancer, prostate cancer, non-small cell lung cancer, bladder cancer, ovarian cancer, gastric cancer, colorectal cancer, esophageal cancer, head and neck squamous cell carcinoma, cervical cancer, pancreatic cancer, testicular cancer, malignant melanoma and soft tissue cancer; preferably, the tumor is selected from breast cancer, gastric cancer, or rectal cancer; more preferably, the tumor is selected from breast cancer.

In an eighth aspect of the present disclosure, there is provided a method for treating, delaying development of, or reducing/inhibiting recurrence of a tumor, comprising administering an effective amount of the bispecific antibody or the pharmaceutical composition to an individual suffering from the above disease or disorder; examples of the tumor include, but are not limited to, breast cancer, prostate cancer, non-small cell lung cancer, bladder cancer, ovarian cancer, gastric cancer, colorectal cancer, esophageal cancer, head and neck squamous cell carcinoma, cervical cancer, pancreatic cancer, testicular cancer, malignant melanoma, and soft tissue cancer; preferably, the tumor is selected from breast cancer, gastric cancer, or rectal cancer; more preferably, the tumor is selected from breast cancer.

In a ninth aspect of the present disclosure, there is provided a method for enhancing or stimulating an immune response or function, comprising administering a therapeutically effective amount of the bispecific antibody or the pharmaceutical composition to a patient/subject.

In a tenth aspect of the present disclosure, there is provided a method for treating, preventing or ameliorating an immune disorder or disease in a patient/subject, comprising administering a therapeutically effective amount of the bispecific antibody or the pharmaceutical composition to the patient/subject.

The technical solutions provided by the present disclosure have beneficial effects summarized as follows:
1. The bispecific antibody provided by the present disclosure includes anti-Her2 scFv that is located at the N terminus of the bispecific antibody and has changed spatial conformation, so that the bispecific antibody has reduced binding ability to Her2 under some conditions, especially that the bispecific antibody is difficult to bind to normal cells with weak expression or low expression of Her2, thereby exhibiting reduced non-specific killing effect. However, the binding specificity to cells with over-expression or high expression of Her2 is not significantly reduced, and the bispecific antibody exhibits a good killing effect *in vivo.* It can be known that when a target antigen is merely expressed on tumor cells or the bispecific antibody of the present disclosure specifically binds to only tumor cells over-expressing the target antigen, immune effector cells are activated restrictively and merely in target cell tissues, which can minimize the non-specific killing of the bispecific antibody on normal cells and the accompanying release of cytokines and reduce the toxic side effects of the bispecific antibody in clinical treatment. The maximum safe starting dose in preclinical toxicology evaluation tests is much higher than other doses for the same target and there is no systemic immunotoxicity, which indicates that the bispecific antibody provided by the present disclosure has a relatively wide administration safety window.
2. The anti-CD3 scFv selected by the bispecific antibody provided by the present disclosure specifically binds to effector cells with weak binding affinity (EC₅₀ value greater than about 50 nM, or greater than 100 nM, or greater than 300 nM, or greater than 500 nM). In addition, the anti-CD3 scFv is embedded between the anti-TAA scFv and Fc, and the CTP rigid peptide contained in the linker peptide L3 at its N terminus and the Fc fragment located at its C terminus partially "cover" or "shield" the antigen binding domain of the anti-CD3 scFv. Such steric hindrance effect makes the anti-CD3 scFv bind to CD3 with weaker binding affinity (for example, greater than 1 µM), which reduces its ability to activate and stimulate T cells, limits the excessive release of cytokines, and provides higher safety. In addition, the anti-CD3 scFv used in the present disclosure can bind to CD3 natural antigens of a human and a cynomolgus monkey and/or a rhesus monkey at the same time, so that no alternative molecule needs to be constructed for preclinical toxicology evaluation, and the effective dose, toxic dose and toxic side effects obtained are more objective and accurate and can be directly converted into a clinical dose to reduce the risk of clinical studies. Further, the bispecific antibody provided by the present disclosure creatively adopts a divalent anti-CD3 scFv, which avoids the asymmetric structure of a heterodimer (including a monovalent anti-CD3 scFv) commonly used in the existing art in terms of the configuration design of the bispecific antibody, and solves the problem of heavy chain mismatches, thereby simplifying downstream purification steps. Moreover, unexpectedly, non-specific binding of the anti-CD3 scFv to T cells is not observed in an *in vitro* cell binding assay, and the degree of cell activation (the release of cytokines such as IL-2) is controlled within a safe and effective range. That is, the bivalent anti-CD3 scFv structure used in the present disclosure has not induced over-activation of T cells in a non-antigen-dependent manner, whereas for other bispecific antibodies including bivalent anti-CD3 domains, the uncontrollable over-activation of T cells is common and thus anti-CD3 bispecific antibodies are generally designed to avoid the introduction of a bivalent anti-CD3 structure.
3. The modified Fc fragment included in the bispecific antibody provided by the present disclosure has no ability of binding to FcyR, avoiding the systemic activation of T cells mediated by FcyR and allowing immune effector cells to be activated restrictively and merely in target cell tissues.
4. The bispecific antibody provided by the present disclosure is homodimeric without mismatches of heavy chains and light chains. The bispecific antibody is produced by a stable downstream process and purified by simple and efficient steps, with a homogeneous expression product and significantly improved physicochemical and *in vivo* stability.
5. The bispecific antibody provided by the present disclosure has a relatively long *in vivo* circulating half-life due to the inclusion of the Fc fragment. Pharmacokinetic tests show that the *in vivo* circulating half-lives in mice and cynomolgus monkeys are about 40 h and 8h, respectively, which will greatly reduce a clinical administration frequency.

### Detailed description

### Abbreviations and definitions

- Her2: Human epidermal growth factor receptor 2
- BiAb: Bispecific antibody
- CDR: Complementarity determining region in a variable region of an immunoglobulin, defined by a Kabat numbering system
- EC₅₀: A concentration at which 50% efficacy or binding is generated
- ELISA: Enzyme-linked immunosorbent assay
- FR: Framework region of an antibody: a variable region of an immunoglobulin excluding the CDRs
- HRP: Horseradish peroxidase
- IL-2: Interleukin 2
- IFN: Interferon
- IC₅₀: A concentration at which 50% inhibition is generated
- IgG: Immunoglobulin G
- Kabat: Immunoglobulin comparison and numbering system advocated by Elvin A Kabat
- mAb: Monoclonal antibody
- PCR: Polymerase chain reaction
- V region: IgG chain segment whose sequence is variable among different antibodies. It extends to Kabat residue 109 of the light chain and residue 113 of the heavy chain.
- VH: Heavy chain variable region of an immunoglobulin
- VK: κ light chain variable region of an immunoglobulin
- K_{D}: Equilibrium dissociation constant
- kₐ: Association rate constant
- k_{d}: Dissociation rate constant

In the present disclosure, unless otherwise specified, the scientific and technical terms used herein have meanings generally understood by those skilled in the art. The antibody or fragments thereof used in the present disclosure may be further modified using conventional techniques known in the art alone or in combination, such as amino acid deletion, insertion, substitution, addition, and/or recombination and/or other modification methods. A method for introducing such modifications into a DNA sequence of an antibody according to the amino acid sequence of the antibody is well known to those skilled in the art. See, for example, Sambrook, Molecular cloning: a laboratory manual, Cold Spring Harbor Laboratory (1989) N.Y.. Such modifications are preferably performed at a nucleic acid level. Meanwhile, for a better understanding of the present disclosure, the definitions and explanations of related terms are provided below.

"Her2 (human epidermal growth factor receptor 2)" is a member of the human epidermal growth factor receptor family. The occurrence, development and severity of many tumors are closely related to the activity of Her2. In addition to gene mutations or amplification, the up-regulation of Her2 may also activate two downstream signaling pathways, trigger a series of cascade reactions, promote unlimited cell proliferation, and ultimately lead to cancer. In addition, Her2 may initiate multiple metastasis-related mechanisms to increase tumor cells metastasis ability. The amplification or overexpression of Her2 genes occurs in various tumors such as breast cancer, ovarian cancer, gastric cancer, lung adenocarcinoma, prostate cancer, and invasive uterine cancer. Indications targeting Her2 include other related diseases or conditions that are found in the existing art and will be found in the future. The term also includes any variants, isotypes, derivatives, and species homologues of Her2 that are naturally expressed by cells including tumor cells or expressed by cells transfected with Her2 genes or cDNA. A species homologue includes Her2 of the rhesus monkey.

"CD3" refers to a part of a T-cell receptor complex and consists of three different chains CD3ε, CD3δ and CD3γ. CD3 is clustered on T cells by, for example, being immobilized by an anti-CD3 antibody, leading to the activation of T cells, which is similar to T cell receptor-mediated activation but independent of the specificity of TCR clones. Most anti-CD3 antibodies recognize the chain CD3ε. The second functional domain that specifically recognizes the T cell surface receptor CD3 in the present disclosure is not specifically limited as long as it can specifically recognize CD3. Preferably, the antibody against human CD3 used in the present disclosure is cross-reactive with cynomolgus monkeys and/or rhesus monkeys. The term also includes any variants, isotypes, derivatives, and species homologues of CD3 that are naturally expressed by cells or expressed by cells transfected with a gene or cDNA encoding the preceding chains.

The term "antibody" generally refers to proteinaceous binding molecules with immunoglobulin-like functions. Typical examples of the antibody are immunoglobulins and its derivatives or functional fragments as long as they exhibit the desired binding specificity. Techniques for preparing the antibody are well known in the art. The "antibody" includes different classes of natural immunoglobulins (such as IgA, IgG, IgM, IgD and IgE) and subclasses (such as IgG1, IgG2, IgA1 and IgA2). The "antibody" further includes non-natural immunoglobulins which include, for example, a single-chain antibody, a chimeric antibody (e.g., humanized murine antibody), and a heterologous conjugated antibody (e.g., bispecific antibody) as well as antigen-binding fragments thereof (e.g., Fab', F(ab'), Fab, Fv and rlgG). See, for example, Pierce Catalog and Handbook, 1994-1995, Pierce Chemical Co., Rockford, IL; Kuby, J., Immunology, 3rd Ed., 1998, NY; W.H. Freeman&Co..

The antibody can bind to one antigen to be "monospecific"; or bind to two different antigens to be "bispecific"; or bind to more than one different antigens to be "multi-specific." The antibody may be monovalent, bivalent, or multivalent, i.e. the antibody may bind to one, two or multiple antigen molecules at one time.

The antibody "monovalently" binds to a particular protein, that is, one molecular of an antibody binds to merely one molecular of a protein, but the antibody may also bind to different proteins. When the antibody binds to merely one molecular of two different proteins, the antibody binds to each protein "monovalently" and the antibody is "bispecific" and "monovalently" binds to each of the two different proteins. The antibody may be "monomeric," that is, the antibody contains a single polypeptide chain. The antibody may contain multiple polypeptide chains ("multimeric") or may contain two ("dimeric"), three ("trimeric") or four ("tetrameric") polypeptide chains. If the antibody is multimeric, the antibody may be a homomultimer, that is, the antibody contains more than one molecular of only one type of polypeptide chain, including a homodimer, a homotrimer or a homotetramer. Alternatively, the multimeric antibody may be a heteromultimer, that is, the antibody contains more than one type of polypeptide chains that are different, including a heterodimer, a heterotrimer or a heterotetramer.

The term "hypervariable region", "CDR" or "complementarity determining region" refers to amino acid residues of an antibody, which are responsible for antigen binding, and is a discontinuous amino acid sequence. CDR sequences are amino acid residues in the variable region that may be defined by the IMGT, Kabat, Chothia or AbM method or identified by any CDR sequence determination method well known in the art. For example, the hypervariable region includes the following amino acid residues: amino acid residues from a "complementarity determining region" or "CDR" defined by sequence comparison, for example, residues at positions 24-34 (L1), 50-56 (L2) and 89-97 (L3) in a light chain variable domain and residues at positions 31-35 (H1), 50-65 (H2) and 95-102 (H3) in a heavy chain variable domain (see Kabat et al., 1991, Sequences of Proteins of Immunological Interest (5th edition), Public Health Service, National Institutes of Health, Bethesda, Md.), and/or amino acid residues from a "hypervariable loop" (HVL) defined according to the structure, for example, residues at positions 26-32 (L1), 50-52 (L2) and 91-96 (L3) in the light chain variable domain and residues at positions 26-32 (H1), 53-55 (H2) and 96-101 (H3) in the heavy chain variable domain (see Chothia and Leskl, J. Mol Biol, 196: 901-917, 1987). "Framework" residues or "FR" residues refer to variable domain residues other than the hypervariable region residues as defined in the present disclosure. In some embodiments, the antibody or the antigen-binding fragment thereof in the present disclosure is preferably determined through the Kabat, Chothia or IMGT numbering system. Those skilled in the art may explicitly assign each system to any variable domain sequence without relying on any experimental data beyond the sequence itself. For example, the Kabat residue numbering method of a given antibody may be determined by comparing the sequence of the given antibody to each "standard" numbered sequence. Based on the numbers of the sequences provided herein, the numbering scheme of determining any variable region sequence in the sequence table is entirely within the conventional technical scope of those skilled in the art.

The term "single-chain Fv antibody" (or "scFv antibody") refers to an antibody fragment comprising VH and VL domains of an antibody. It is a fusion protein of the variable regions of the heavy (VH) and light chains (VL) connected with a linker. The linker enables these two domains to be cross-linked to form an antigen-binding site, and the sequence of the linker generally consists of a flexible peptide, for example, but not limited to, G₂(GGGGS)₃. The size of scFv is generally 1/6 of an intact antibody. The single-chain antibody is preferably an amino acid chain sequence encoded by a nucleotide chain. For the review of scFv, reference may be made to Pluckthun (1994), The Pharmacology of Monoclonal Antibodies, Vol. 113, edited by Rosenburg and Moore, Springer-Verlag, New York, pages 269-315. Reference may also be made to International Patent Application Publication No. WO 88/01649 and U.S. Patent Nos. 4,946,778 and 5,260,203.

The term "Fab fragment" consists of a light chain and a CH1 and a variable domain of each of a heavy chain. The heavy chain of the Fab molecule cannot form a disulfide bond with another heavy chain molecule. The size of "Fab antibody" is 1/3 of an intact antibody, and "Fab antibody" includes only one antigen-binding site.

The term "Fab' fragment" contains a light chain, and a VH domain and a CH1 domain of a heavy chain, and a constant region between CH1 and CH2 domains.

The term "F(ab')₂ fragment" contains two light chains, VH domains and CH1 domains of two heavy chains, and constant regions between CH1 and CH2 domains, so that an inter-chain disulfide bond is formed between the two heavy chains. Therefore, the F(ab')₂ fragment is composed of two Fab' fragments held together by the disulfide bond between the two heavy chains.

The term "Fc" region refers to the antibody heavy chain constant region fragment, including at least a hinge region and CH2 and CH3 domains.

The term "Fv region" includes variable regions from the heavy chain and the light chain but lacks the constant regions, and is the minimum fragment containing a complete antigen recognition and binding site.

The term "Fd fragment" consists of CH1 and variable regions of a heavy chain and is the heavy chain part of a Fab fragment excluding the light chain.

The term "disulfide-stabilized variable fragment (dsFv)" introduces one cysteine mutation site into the VH region and the VL region, respectively, so as to form a disulfide bond between the VH and the VL to achieve structural stability.

The term "antibody fragment" and "antigen-binding fragment" refers to an antigen-binding fragment of the antibody that retains a specific binding ability to an antigen (e.g., Her2), as well as antibody analogs. It generally includes at least part of an antigen-binding region or a variable region of a parental antibody. The antibody fragment retains at least part of the binding specificity of the parental antibody. Generally, when the activity is represented in moles, the antibody fragment retains at least 10% of parental binding activity. Preferably, the antibody fragment retains at least 20%, 50%, 70%, 80%, 90%, 95% or 100% of the binding affinity of the parental antibody to a target. The antibody fragments include, but are not limited to, Fab fragments, Fab' fragments, F(ab')2 fragments, Fv fragments, Fd fragments, complementarity determining region (CDR) fragments, disulfide-stabilized variable fragments (dsFv); linear antibodies, single-chain antibodies (e.g., scFv monoclonal antibodies) (technology from Genmab), bivalent single-chain antibodies, single-chain phage antibodies, single domain antibodies (e.g., VH domain antibodies), domain antibodies (technology from Ablynx); multispecific antibodies formed from antibody fragments (e.g., triabodies and tetrabodies); and engineered antibodies such as chimeric antibodies (e.g., humanized mouse antibodies) and heteroconjugate antibodies. These antibody fragments can be obtained using any conventional technologies known to those skilled in the art, and the utility of these fragments can be screened in the same way as the intact antibody.

The term "linker peptide" refers to a peptide linking two polypeptides, wherein the linker peptide may be two immunoglobulin variable regions or one variable region. The length of the linker peptide may be 0 to 30 amino acids or 0 to 40 amino acids. In some embodiments, the linker peptide may be in the length of 0 to 25, 0 to 20, or 0 to 18 amino acids. In some embodiments, the linker peptide may be a peptide having no more than 14, 13, 12, 11, 10, 9, 8, 7, 6, or 5 amino acids. In other embodiments, the linker peptide may include 0 to 25, 5 to 15, 10 to 20, 15 to 20, 20 to 30, or 30 to 40 amino acids. In other embodiments, the linker peptide may have about 0, 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30 amino acids. The linker peptide is known to those skilled in the art. The linker peptide may be prepared by any method in the art. For example, the linker peptide may be originated from synthesis.

The term "heavy chain constant region" includes an amino acid sequence from the immunoglobulin heavy chain. The polypeptide comprising the heavy chain constant region includes at least one of: a CH1 domain, a hinge domain (e.g., an upper hinge region, an intermediate hinge region, and/or a lower hinge region), a CH2 domain, a CH3 domain, or a variant or fragment thereof. For example, the antigen-binding polypeptide used herein may include a polypeptide chain having a CH1 domain; a polypeptide having a CH1 domain, at least part of a hinge domain, and a CH2 domain; a polypeptide chain having a CH1 domain and a CH3 domain; a polypeptide chain having a CH1 domain, at least part of a hinge domain, and a CH3 domain; or a polypeptide chain having a CH1 domain, at least part of a hinge domain, a CH2 domain, and a CH3 domain. In another embodiment, the polypeptide of the present application includes a polypeptide chain having a CH3 domain. In addition, the antibody used in the present application may lack at least part of a CH2 domain (e.g., all or part of a CH2 domain). As described above, it is appreciated by those of ordinary skill in the art that heavy chain constant regions may be modified such that they differ in amino acid sequence from naturally immunoglobulin molecules.

The term "light chain constant region" includes an amino acid sequence from the antibody light chain. Preferably, the light chain constant region includes at least one of a constant kappa domain and a constant lambda domain.

The term "VH domain" includes an amino-terminal variable domain of the immunoglobulin heavy chain, while the term "CH1 domain" includes a first (mostly amino-terminal) constant region of the immunoglobulin heavy chain. The CH1 domain is adjacent to the VH domain and is the amino-terminal of the hinge region of the immunoglobulin heavy chain molecule.

The term "hinge region" includes the portion of a heavy chain molecule that links the CH1 domain to the CH2 domain. The hinge region contains about 25 residues and is flexible so that two N-terminal antigen-binding regions move independently. The hinge region may be divided into three different domains: upper, middle and lower hinge domains (Roux KH et al., J. Immunol., 161: 4083, 1998).

The term "disulfide bond" includes a covalent bond formed between two sulfur atoms. The amino acid cysteine contains a sulfhydryl group which can form a disulfide bond or a bridge with a second sulfhydryl group. In most naturally occurring IgG molecules, CH1 and CK regions are linked by a disulfide bond and two heavy chains are linked by two disulfide bonds at positions 239 and 242 according to the Kabat numbering system (position 226 or 229, EU numbering system).

"Binding" defines the affinity interaction between a specific epitope on an antigen and its corresponding antibody and is generally understood as "specific recognition." "Specific recognition" means that the bispecific antibody of the present disclosure does not or substantially does not have cross-reaction with any polypeptide other than the target antigen. The degree of specificity may be determined by immunological techniques, including, but not limited to, immunoblotting, immunoaffinity chromatography, and flow cytometry. In the present disclosure, the specific recognition is preferably determined by flow cytometry, and the criteria for the specific recognition in particular cases can be judged by those of ordinary skill in the art according to the general knowledge of the art which he/she knows.

The term "*in vivo* half-life" refers to the biological half-life of the polypeptide of interest in the circulation of a given animal and is expressed as the time it takes to clear half of the amount present in the circulation of the animal from the circulation and/or other tissues in the animal.

The term "identity" refers to the matching of sequences between two polypeptides or between two nucleic acids. When a certain position in each of two sequences for comparison is occupied by the same base group or amino acid monomer subunit (e.g., a certain position in each of the two DNA molecules is occupied by adenine, or a certain position in each of the two polypeptides is occupied by lysine), then the molecules are identical at that position. The "percentage identity" between two sequences is a function of the number of matching positions of the two sequences divided by the number of positions to be compared and then multiplied by 100. For example, if 6 of the 10 positions in two sequences are matched, the identity between the two sequences is 60%. For example, DNA sequences CTGACT and CAGGTT have a total identity of 50% (three of a total of six positions are matched). Generally, the comparison is made when two sequences are aligned to produce maximum identity. Such alignment may be implemented, for example, through a computer program, such as an Align program (DNAstar, Inc.) to conveniently perform the method described by Needleman et al. Mol. Biol., 48: 443-453. The percentage identity between the two amino acid sequences may also be determined by using the algorithm proposed by E. Meyers and W. Miller (Comput. Appl Biosci., 4: 11-17) which has been incorporated into ALIGN program (version 2), using the PAM 120 weight residue table with a gap length penalty score of 12 and a gap penalty score of 4. In addition, the percentage identity between the two amino acid sequences may also be determined by using the algorithm proposed by Needleman and Wunsch (J. Mol. Biol., 48: 444-453) which has been incorporated into the GAP program in the GCG software package (available on www.gcg.com), using Blossum 62 matrix or PAM 250 matrix with a gap weight of 16, 14, 12, 10, 8, 6 or 4 and a length weight of 1, 2, 3, 4, 5 or 6.

The term "isolated antibody molecules" refers to antibody molecules that have been identified and isolated and/or recovered from components in their natural environment. The contaminant components in the natural environment of the antibody molecules are substances that interfere with the diagnostic or therapeutic use of the antibody and may include enzymes, hormones, and other proteinaceous or non-proteinaceous solutes.

The term "isolated" in reference to cells and nucleic acids (such as DNA or RNA), as used herein, refers to molecules isolated from other DNA or RNA present as macromolecules of natural origin. The term "isolated" used herein also refers to nucleic acids or peptides that, when manufactured by a recombinant DNA technology, are substantially free of cellular materials, viral materials or culture media or those that are substantially free of chemical precursors or other chemicals when prepared by a chemical synthesis. In addition, "isolated nucleic acids" refer to nucleic acid fragments that are not naturally generated as fragments and are not present in the natural state. The term "isolated" is also used herein to refer to cells or polypeptides isolated from other cellular proteins or tissues. Isolated polypeptides include purified and recombinant polypeptides.

The term "Fc region" or "Fc fragment" refers to the C-terminal regions of the immunoglobulin heavy chain, which includes at least part of a hinge region, a CH2 domain and a CH3 domain. It mediates the binding of immunoglobulins to host tissues or factors, including the binding of immunoglobulins to Fc receptors located on various cells (e.g., effector cells) of the immune system or the binding of immunoglobulins to the first component (C1q) of the classical complement system. It includes the native sequence Fc region and the variant Fc region.

Generally, the human IgG heavy chain Fc region is a segment from the amino acid residue at the position Cys226 or Pro230 of the human IgG heavy chain Fc region to the carboxy terminus, but its boundaries may vary. The C-terminal lysine of the Fc region (residue 447, according to the EU numbering system) may or may not be present. Fc may also refer to this region in isolation, or in the case of a protein polypeptide comprising Fc, for example, "binding protein comprising an Fc region", and is also referred to as "Fc fusion protein" (e.g., antibody or immunoadhesin). The native Fc region of the antibody of the present disclosure includes mammalian (e.g., human) IgG1, IgG2 (IgG2A, IgG2B), IgG3, and IgG4. Among human IgG1 Fc regions, at least two allotypes are known. In some embodiments, there is a single amino acid substitution, insertion, and/or deletion of about 10 amino acids per 100 amino acids in the amino acid sequences of two Fc polypeptide chains relative to the sequence of the amino acid sequence of the mammalian Fc polypeptide. In some embodiments, the difference may be changes in Fc that extend the half-life, changes that increase FcRn binding, changes that inhibit Fcγ receptor (FcγR) binding, and/or changes that decrease or remove ADCC and CDC.

In antibody isotypes IgG, IgA and IgD, the Fc region contains CH2 and CH3 constant domains of each of two heavy chains of the antibody; The Fc region of IgM and IgE contains three heavy chain constant domains (CH domains 2 to 4) in each polypeptide chain.

The term "Fc receptor" or "FcR" refers to a receptor that binds to the Fc region of an immunoglobulin. FcR may be a native sequence human FcR, or may be an FcR that binds to the IgG antibody (a γ receptor), as well as allelic variants and alternatively spliced forms of these receptors. The FcyR family is composed of three activating receptors (FcγRI, FcγRIII and FcγRIV in mice; FcγRIA, FcyRIIA and FcγRIIIA in humans) and one inhibitory receptor (FcyRllb or equivalent FcyRIIB). The FcγRII receptor includes FcγRIIA ("activating receptor") and FcγRIIB ("inhibitory receptor") which have similar amino acid sequences. The cytoplasmic domain of FcγRIIA includes an immunoreceptor tyrosine-based activation motif (ITAM). The cytoplasmic domain of FcγRIIB contains an immunoreceptor tyrosine-based inhibitory motif (ITIM) (see M. Annu. Rev. Immunol., 15: 203-234 (1997)). Most native effector cell types co-express one or more activating FcyRs and inhibitory FcγRIIbs, while NK cells selectively express one activating Fc receptor (FcγRIII in mice and FcγRIIIA in humans) but do not express inhibitory FcγRIIb in mice and humans. Human IgG1 binds to most human Fc receptors and is considered equivalent to murine IgG2a in terms of the type of activating Fc receptor to which Human IgG1 binds. The term "FcR" herein covers other FcRs, including those which will be identified in the future. Methods of measuring the binding to FcRn are known (see, e.g., Ghetie V et al., Immunol Today, 18: 592-8, 1997); Ghetie V et al., Nature Biotechnology, 15: 637-40, 1997)). The *in vivo* binding and serum half-life of the human FcRn high-affinity binding polypeptide to FcRn can be determined, for example, in transgenic mice expressing human FcRn or transfected human cell lines. The term "Fc receptor" or "FcR" also includes the neonatal receptor FcRn which is responsible for transferring maternal IgG to the fetus (Guyer RL et al., J. Immunol., 117: 587, 1976) and Kim YJ et al., J. Immunol., 24: 249, 1994)).

The term "functional domain" refers to a three-dimensional structure capable of specifically recognizing and/or binding to an epitope, such as an antibody or an antibody fragment which includes a natural intact antibody, a single-chain antibody (scFv), a Fd fragment, a Fab fragment, a F(ab')₂ fragment, a single-domain antibody fragment, an isolated CDR fragment, and derivatives thereof. The "single-chain" here means that the first and second functional domains are covalently linked, preferably formed in a sequence of collinear amino acids that can be encoded by one nucleic acid molecule.

The term "domain antibody" is an immunologically functional immunoglobulin fragment that contains merely a heavy chain variable region or a light chain variable region. In some cases, two or more VH regions are covalently joined with a peptide linker to produce a bivalent domain antibody. Two VH regions of the bivalent domain antibody may target the same antigen or different antigens.

The term "monoclonal antibody (mAb)" refers to an antibody obtained from a population of substantially homogeneous antibodies, that is, individual antibodies comprised in the population are identical except for possible naturally occurring mutations that present in minor amounts and show single binding specificity for and affinity to a particular epitope. The modifier "monoclonal" denotes the property of the antibody obtained from a substantially homogeneous population of antibodies, where the antibody does not need to be produced by a particular method. The monoclonal antibody is produced by methods known to those skilled in the art, such as the preparation of heterozygous antibody-producing cells through the fusion of myeloma cells and immune spleen cells. The monoclonal antibody can be synthesized by culturing hybridomas without being contaminated by any other antibodies. The monoclonal antibody may be obtained through recombination technology, phage display technology, synthesis technology, or other existing technologies.

The term "chimeric antibody" refers to an antibody in which a portion of a heavy chain and/or a light chain is identical or homologous to a corresponding sequence in an antibody derived from a particular species or belonging to a particular antibody class or subclass, while the remainder of the chain is identical or homologous to a corresponding sequence in an antibody derived from another species or belonging to another antibody class or subclass, and a fragment of such antibody, as long as they exhibit desired biological activity (U.S. patent No. No 4,816,567; Morrison SL et al., Proc. Natl. Acad. Sci. USA, 81: 6851-6855, 1984). For example, the term "chimeric antibody" may include such an antibody (such as a human-murine chimeric antibody) in which the heavy and light chain variable regions of the antibody are from a first antibody (such as a murine antibody) and the heavy and light chain constant regions of the antibody are from a second antibody (such as a human antibody).

The term "intact antibody" refers to an antibody consisting of two antibody heavy chains and two antibody light chains. An "intact antibody heavy chain" is composed of an antibody heavy chain variable domain (VH), antibody heavy chain constant domain 1 (CH1), an antibody hinge region (HR), antibody heavy chain constant domain 2 (CH2) and antibody heavy chain constant domain 3 (CH3) from N-terminus to C-terminus, abbreviated as VH-CH1-HR-CH2-CH3; and optionally includes antibody heavy chain constant domain 4 (CH4) in the case of an antibody of the IgE subclass. Preferably, the "intact antibody heavy chain" is a polypeptide consisting of VH, CH1, HR, CH2 and CH3 from the N-terminus to the C-terminus. An "intact antibody heavy chain" is a polypeptide consisting of an antibody light chain variable domain (VL) and an antibody light chain constant domain (CL) from N-terminus to C-terminus, abbreviated as VL-CL. The antibody light chain constant domain (CL) may be κ (kappa) or λ (lambda). Intact antibody chains are linked together by an inter-peptide disulfide bond between the CL domain and the CH1 domain (i.e., between the light chain and the heavy chain) and an inter-peptide disulfide bond between hinge regions of intact antibody heavy chains. Typical examples of the intact antibody are natural antibodies such as IgG (e.g., IgG1 and IgG2), IgM, IgA, IgD and IgE.

The term "humanized antibody" refers to a genetically engineered non-human antibody whose amino acid sequence has been modified to increase homology to the sequence of the human antibody. Most or all of the amino acids outside the CDR domain of a non-human antibody, for example, a mouse antibody, are substituted by corresponding amino acids from human immunoglobulins, while most or all of the amino acids within one or more CDR regions are not altered. The addition, deletion, insertion, substitution or modification of small molecule amino acids is permissible as long as they do not eliminate the ability of the antibody to bind a particular antigen. The "humanized" antibody retains antigen specificity similar to that of the original antibody. The origin of the CDRs is not particularly limited and may be derived from any animal. For example, antibodies derived from mouse antibodies, rat antibodies, rabbit antibodies, or non-human primate (e.g., cynomolgus monkeys) antibodies may be used. Examples of human frameworks useful in the present disclosure are KOL, NEWM, REI, EU, TUR, TEI, LAY, and POM (Kabat et al., ibid). For example, KOL and NEWM may be used for heavy chains, REI may be used for light chains, and EU, LAY, and POM may be used for both heavy and light chains. Alternatively, human germline sequences may be used, and these sequences are available at http://www2.mrc-lmb.cam.ac.uk/vbase/list2.php. In the humanized antibody molecules of the present disclosure, the receptor heavy and light chains do not need to be derived from the same antibody, and may, if needed, include complex chains having framework regions derived from different chains.

The term "human" antibody (HuMab) refers to an antibody with variable regions in which the framework regions and CDR regions are derived from human germline immunoglobulin sequences. In addition, if the antibody contains a constant region, the constant region is also derived from human germline immunoglobulin sequences. The human antibody of the present disclosure may include amino acid residues that are not encoded by human germline immunoglobulin sequences (such as mutations introduced by random or site-specific mutagenesis *in vitro* or by somatic mutation *in vivo*). However, as used herein, the term "human antibody" is not intended to include an antibody in which a CDR sequence derived from the germline of another mammal species, such as a mouse, has been grafted onto a human frame sequence.

The term "epitope" or "antigenic determinant" refers to a particular chemical group or peptide sequence on a molecule that is antigenic (i.e., can induce a specific immune response) and is a site on an antigen (such as Her2) at which an immunoglobulin or an antibody specifically binds to. The immunoglobulin or the antibody specifically binds to a particular antigenic epitope on a polypeptide (such as Her2). An OK epitope determining region is generally composed of chemically active surface groups (such as amino acids or glycosyl side chains) of a molecule and generally has particular three-dimensional structural properties and particular charge properties.

The term "recombinant" in reference to polypeptides or polynucleotides refers to a form of polypeptides or polynucleotides that is not present at the natural state. As one non-limiting example, it may be achieved through a combination of polynucleotides or polypeptides that are generally not present together.

The terms "conjugation" and "linking" refer to the association of two or more molecules. The linking may be genetic (i.e., fused through recombination). In specific context, the terms include reference to linking a ligand such as an antibody moiety with an effector molecule. Such linking may be achieved using a variety of well-known techniques in the art such as a chemical or recombinant manner. The "chemical manner" refers to a reaction between the antibody moiety and the effector molecule that allows a covalent bond to be formed between two molecules to form a molecule.

The term "target cell" is a cell that binds to an antibody and participates in mediating disease. In some cases, the target cell may be a cell that generally mediates an immune response and also mediates a disease. For example, in B-cell lymphoma, B cells that generally mediate immune responses may be target cells. In some embodiments, the target cell is a cancer cell, a pathogen-infected cell, or a cell that mediates an auto-immunological disease or inflammatory disease (such as fibrosis). The antibody may bind to the target cell by binding to an antigen on a "target cell protein" which is a protein displayed on the surface of the target cell and may be a highly expressed protein.

The term "cytotoxic agent" used herein refers to a substance that inhibits or prevents the function of a cell and/or causes damages to the cell. The term is intended to include radioisotopes (such as I¹³¹, I¹²⁵, Y⁹⁰ and Re¹⁸⁶), chemotherapeutic agents, and toxins (such as enzyme-active toxins derived from bacteria, fungi, plants or animals), or fragments thereof.

The term "cytokine" generally refers to a protein released by a population of cells, and acting as an intercellular mediator on another cell or having an autocrine effect on the cell that produce the protein. Examples of such cytokines include lymphokines, mononuclear factors; interleukin ("IL") such as IL-2, IL-6 and IL-17A-F; tumor necrosis factors such as TNF-α or TNF-β; and other polypeptide factors such as leukemia inhibitory factor (LIF).

The term "chemotherapy" refers to the administration of any chemical agent for therapeutic purposes in the treatment of diseases characterized by the growth of abnormal cells. Such diseases include, for example, tumors, neoplasia, cancer and diseases characterized by proliferative growth. A "chemotherapeutic agent" specifically targets cells involved in cell division rather than cells not involved in cell division. The chemotherapeutic agent directly interferes with processes closely related to cell division, such as DNA replication, RNA synthesis, protein synthesis, or the assembly, decomposition, or function of mitotic spindles, and/or the synthesis or stability of molecules (such as nucleotides or amino acids) that function in these processes. Therefore, the chemotherapeutic agent has cytotoxicity or cytostatic effects on cancer cells and other cells involved in cell division. Combined chemotherapy refers to the administration of more than one agent for the treatment of cancer.

The term "immunobinding" and "immunobinding property" refers to a non-covalent interaction between an immunoglobulin molecule and an antigen (to the antigen, the immunoglobulin is specific). The strength or affinity of the immunobinding interaction may be represented by the equilibrium dissociation constant (K_{D}) of the interaction, where the smaller the K_{D} value, the higher the affinity. The immunobinding property of the selected polypeptide may be quantified using a method known in the art. One method relates to the measurement of rates at which an antigen-binding site/antigen complex is formed and dissociated. Both the "binding rate constant" (Kₐ or Kₒₙ) and the "dissociation rate constant" (K_{d} or K_{off}) may be calculated according to the concentration and actual rates of association and dissociation (see Malmqvist M et al., Nature, 361: 186-187, 1993). The ratio of k_{d}/kₐ is the dissociation constant K_{D} (generally see Davies et al., Annual Rev Biochem., 1990, 59: 439-473). Any effective method may be used for measuring values of K_{D}, kₐ and k_{d}.

The term "cross-reaction" refers to the ability of the antibody described herein to bind to tumor-associated antigens from different species. For example, the antibody described herein that binds to human TAA may also bind to TAAs from other species (e.g., cynomolgus monkey TAA). Cross-reactivity may be measured by detecting specific reactivity with purified antigens in binding assays (e.g., SPR, ELISA), or detecting the binding to cells physiologically expressing TAA or the interaction with the function of cells physiologically expressing TAA. Examples of assays known in the art for determining the binding affinity include surface plasmon resonance (e.g., Biacore) or similar techniques (e.g., Kinexa or Octet).

The term "EC₅₀" refers to the maximum response of the concentration of the antibody or antigen-binding fragment thereof that induces a 50% response in an *in vitro* or *in vivo* assay using the antibody or antigen-binding fragment thereof, that is, half between the maximum response and the baseline.

The term "immune response" refers to the action of immune system cells (such as T lymphocytes, B lymphocytes, NK cells, antigen-presenting cells, macrophages, eosinophils, mast cells, DCs, or neutrophils) and soluble macromolecules produced by any one of the immune cells or the liver (including antibodies, cytokines and complements) that results in selective injuries to, damages to, or elimination from the human body of invading pathogens, cells or tissues infected with pathogens, cancer cells, or normal human cells or tissues in the context of pathological inflammation. The immune response includes, for example, the activation or inhibition of T cells (e.g., effector T cells or Th cells such as CD⁸⁺ or CD⁴⁺ T cells) or the inhibition or depletion of NK cells or Treg cells.

The term "immune cells" includes cells of hematopoietic origin that function in immune responses, for example, lymphocytes such as B cells and T cells; natural killer cells; and myeloid cells such as monocytes, macrophages, eosinophils, mast cells, basophils and granulocytes.

An "effector cell" refers to a cell of the immune system that expresses one or more FcRs and mediates one or more effector functions. Preferably, the cell expresses at least one type of activating Fc receptor such as human FcγRIII and implements an ADCC effector function. Examples of human leukocytes that mediate ADCC include peripheral blood mononuclear cells (PBMC), NK cells, monocytes, macrophages, neutrophils and eosinophils. The effector cell also includes, for example, T cells. The effector cell may be derived from any organism including, but not limited to, humans, mice, rats, rabbits and monkeys.

The term "immunogenicity" refers to the ability of a particular substance to elicit an immune response. Tumors are immunogenic, and enhancing immunogenicity of tumors may help clear tumor cells by the immune response. Examples of enhancing immunogenicity of tumors include, but are not limited to, the treatment with certain inhibitors (e.g., bispecific antibodies).

The term "immunotherapy" refers to the treatment of subjects suffering from or at risk of diseases or disease recurrence by modifying immune responses through induction, enhancement, inhibition, or other manners.

The term "effector function" refers to biological activities that can be attributed to the biological activities of the antibody Fc region (a native sequence Fc region or amino acid sequence variant Fc region) and that vary with antibody isotypes. Examples of antibody effector functions include, but are not limited to, Fc receptor binding affinity, ADCC, ADCP, CDC, downregulation of cell surface receptors (e.g., B cell receptors), B cell activation, cytokine secretion, and half-life/clearance rate of antibodies and antigen-antibody complexes. Methods of altering the effector function of antibodies are known in the art, for example, the effector function of antibodies may be altered by introducing mutations in the Fc region.

The term "antibody-dependent cell-mediated cytotoxicity (ADCC)" refers to a cytotoxic form in which Ig binds to FcRs on cytotoxic cells (e.g., NK cells, neutrophils or macrophages) to enable these cytotoxic effector cells to specifically bind to antigen-attached target cells and then secret cytotoxins to kill the target cells. Methods for detecting the ADCC activity of an antibody are known in the art, for example, the ADCC activity may be detected by measuring the binding activity between a to-be-tested antibody and FcR (e.g., CD16a).

The term "antibody-dependent cell-mediated phagocytosis (ADCP)" refers to a cell-mediated reaction in which a non-specific cytotoxic active cell expressing FcyR recognizes a bound antibody on a target cell and subsequently causes phagocytosis of the target cell.

The term "complement-dependent cytotoxicity (CDC)" refers to a cytotoxic form that activates the complement cascade by binding the complement component C1q to the antibody Fc. Methods for detecting the CDC activity of an antibody are known in the art, for example, the CDC activity may be detected by measuring the binding activity between a to-be-tested antibody and an Fc receptor (e.g., C1q).

The term "tumor immunity" refers to the process in which tumors evade immune recognition and clearance. Thus, as a therapeutic concept, the tumor immunity receives "treatment" when such evasion is attenuated, and tumors are recognized and attacked by the immune system. Examples of tumor recognition include tumor binding, tumor shrinkage and tumor clearance.

The term "host cell" refers to a cell in which a vector can proliferate and its DNA can be expressed. The cell may be a prokaryotic or eukaryotic cell. The term also includes any offspring of the test host cell. It should be understood that not all offspring are the same as parent cells due to mutations during replication and such offspring are included. The host cell includes a prokaryotic cell, a yeast or a mammalian cell such as a CHO cell, a NS0 cell or another mammalian cell, preferably a CHO cell.

The term "vector" refers to a nucleic acid molecule capable of transporting another nucleic acid to which it is linked. One type of vector is a "plasmid", which refers to a circular double-stranded DNA loop into which an additional DNA segment can be linked. Another type of vector is a viral vector in which an additional DNA segment may be linked into the viral genome. Some vectors can be self-replicated in host cells into which these vectors are introduced (e.g., a bacterial vector with a bacterial replication origin and an episomal mammalian vector). Other vectors (such as non-additional mammalian vectors) may be integrated into the genome of the host cell after being introduced into the host cell and thus replicated with the genome of the host cell. In addition, some vectors can direct the expression of genes to which they are effectively linked. Such vectors are referred to as "recombinant expression vectors" (or simply referred to as "expression vectors") herein. In general, expression vectors useful in recombinant DNA techniques are generally present in the form of plasmids. However, other forms of expression vectors are also included, such as viral vectors (e.g., replication-defective retroviruses, adenoviruses, and adeno concomitant viruses), which perform equivalent functions.

The term "pharmaceutically acceptable carrier and/or excipient and/or stabilizer" refers to a carrier and/or excipient and/or stabilizer that are pharmacologically and/or physiologically compatible with a subject and an active ingredient, and are non-toxic to cells or mammals exposed to the carrier and/or excipient and/or stabilizer at the dose and concentration used. The pharmaceutically acceptable carrier and/or excipient and/or stabilizer include, but are not limited to, a PH regulator, a surfactant, an adjuvant, an ionic strength enhancer, a diluent, a reagent for maintaining osmotic pressure, a reagent for delaying absorption and a preservative. For example, the pH regulator includes, but is not limited to, a phosphate buffer, a citrate buffer and a histidine buffer. The surfactant includes, but is not limited to, cationic, anionic or nonionic surfactants, such as Tween-80. The ionic strength enhancer includes, but is not limited to, sodium chloride. The reagent for maintaining osmotic pressure includes, but is not limited to, sugar, NaCl and analogues thereof. The reagent for delaying absorption includes, but is not limited to, monostearate and gelatin. The diluent includes, but is not limited to, water, an aqueous buffer (such as buffered saline), an alcohol and a polyol (such as glycerol), etc. The preservative includes, but is not limited to, various antibacterial reagents and antifungal reagents such as thimerosal, 2-phenoxyethanol, p-hydroxybenzoate, chloretone, phenol and sorbic acid. The stabilizer has the meaning generally understood by those skilled in the art, can stabilize the desired activity of active ingredients in a drug, and includes, but is not limited to, sodium glutamate, gelatin, SPGA, sugars (e.g., sorbitol, mannitol, starch, sucrose, lactose, dextran or glucose), amino acids (e.g., glutamate, glycine), proteins (e.g., dried whey, albumin or casein), or degradation products thereof (e.g., lactalbumin hydrolysate).

The term "prevention" refers to a method implemented to prevent or delay the occurrence of or, in case of occurrence, minimize the action of a disease or disorder or condition (e.g., a tumor or an infection) in a subject. The term "treatment" refers to a method implemented to obtain beneficial or desired clinical results. The beneficial or desired clinical results include, but are not limited to, reducing the rate of disease progression, ameliorating or alleviating a disease status, and remission or improved prognosis no matter whether they are detectable or undetectable. The amount of a therapeutic agent for effectively alleviating the symptoms of any particular disease may be varied depending on factors such as a disease status, age and weight of a patient and an ability of a drug to cause a desired response in a subject. Whether the symptoms of the disease are alleviated may be assessed through any clinical measurement that is generally used by a physician or other skilled healthcare provider to assess the severity or progression status of the symptoms.

The term "cytotoxicity" molecule refers to such as the toxicity of an immunotoxin to cells intended to be targeted rather than other cells of an organism.

The terms "patient" and "subject", "individual" or "object" refer to any human or non-human animal, in particular human, that receives prophylactic or therapeutic treatment. For example, the method and composition described herein may be used to treat a subject with cancer. The term "non-human animal" includes all vertebrates, for example, mammals and non-mammals such as non-human primates, sheep, dogs, cattle, chickens, amphibians, reptiles and the like.

The term "effective amount" refers to an amount sufficient to obtain or at least partially obtain the desired effect. For example, a prophylactically (e.g., tumor or infection) effective amount refers to an amount sufficient to prevent, arrest, or delay the onset of a disease (e.g., tumor or infection) when used alone or used together with one or more therapeutic agents; a therapeutically effective amount refers to an amount sufficient to cure or at least partially arrest the disease and complications thereof in a patient already suffering from the disease when used alone or used together with one or more therapeutic agents. It is well within the ability of those skilled in the art to determine such effective amounts. For example, the amount effective for therapeutic use depends on the severity of the to-be-treated disease, the overall state of the patient's own immune system, the general condition of the patient such as age, weight and sex, the mode of administration of the drug, and other treatments administered concurrently. The terms "efficacy" and "effectiveness" with respect to treatment include both pharmacological effectiveness and physiological safety. The pharmacological effectiveness refers to the ability of a drug to promote regression of a condition or symptom in a patient. The physiological safety refers to the level of toxicity or other adverse physiological effects (adverse effects) at the cellular, organ and/or organism level due to drug administration.

"Treatment" or "therapy" on a subject refers to any type of intervention or treatment of, or administration of an active agent to, a subject for the purpose of reversing, alleviating, ameliorating, inhibiting, slowing or preventing the occurrence, progression, progression, severity, or recurrence of symptoms, complications, disorders or biochemical indicators associated with a disease.

The term "autoimmunization" refers to that in an immune-tolerant state, a certain amount of autoreactive T cells and autoantibodies are ubiquitous in the whole peripheral immune system of an individual, which helps to eliminate aging and degenerated auto components and has important physiological significance for maintaining the autoimmune stability of the immune system.

The terms "mutated", "mutant" and "mutation" in reference to nucleic acids and polypeptides refer to the replacement, deletion or insertion of one or more nucleotides or amino acids, respectively, compared with natural nucleic acids or polypeptides (i.e., reference sequences that may be used for defining wild types).

The term "site" is an amino acid site within an amino acid sequence described herein. This site may be indicated relative to a similar natural sequence such as a sequence of a naturally occurring IgG domain or chain. The term "corresponding" used herein also includes sites that do not necessarily or not solely depend on the preceding number of nucleotides or amino acids. Therefore, possible substitutions at given amino acid sites of the present disclosure may change due to amino acid deletions or additions at other positions of an antibody chain.

The term "binding protein" refers to a protein that specifically binds to a particular part or target with high affinity. Examples of binding proteins include, but are not limited to, antibodies, antigen-binding fragments of antibodies, adnectin, a micro-antibody, affibodies, affilin, target binding regions of receptors, cell adhesion molecules, ligands, enzymes, cytokines and chemokines. In a preferred embodiment of the present disclosure, the binding protein includes the Fc region of an antibody.

The term "complement system" refers to a large number of small proteins found in blood, referred to as complement factors, which are generally circulated as inactive precursors (pro-proteins). This term refers to the following ability of the constant and inadaptive system: an ability of "supplemental" antibodies and phagocytes to remove pathogens such as bacteria and antigen-antibody complexes from organisms. An example of the complement factor is complex C1 which includes C1q and two serine proteases C1r and C1s. Complex C1 is a component of CDC pathway. C1q is a hexavalent molecule with a molecular weight of about 460,000 and has a structure similar to a bouquet of a tulip, where six collagen "stems" are linked to six spherical head regions. To activate the complement cascade, C1q has to bind to at least two molecules of IgG1, IgG2 or IgG3.

The term "T-cell receptor (TCR)" is a specific receptor present on the surface of T cells, i.e., T lymphocytes. *In vivo*, the T-cell receptor is present as a complex of several proteins. The T-cell receptor generally has two separate peptide chains, typically T-cell receptors α and β (TCRα and TCRβ) chains, and in some T cells, they are T-cell receptor γ and δ (TCRγ and TCRδ). The other proteins in the complex are CD3 proteins: CD3εγ and CD3εδ heterodimers, most importantly, CD3ζ homodimers having six ITAMs. The ITAMs on CD3ζ can be phosphorylated by Lck, which in turn recruit ZAP-70. Lck and/or ZAP-70 may also phosphorylate tyrosine on many other molecules, particularly CD28, LAT, and SLP-76, which allows aggregation of signal transduction complexes around these proteins.

The term "bispecific antibody" refers to the bispecific antibody of the present disclosure, for example, an anti-Her2 antibody or an antigen-binding fragment thereof, which may be derivatized or linked to another functional molecule, for example, another peptide or protein (e.g., TAA, a cytokine and a cell surface receptor), to generate a bispecific antibody that binds to at least two different binding sites or target molecules. To produce the bispecific molecule of the present disclosure, the antibody of the present disclosure may be functionally linked (e.g., by chemical coupling, gene fusion, non-covalent binding or other means) to one or more other binding molecules, for example, another antibody, antibody fragment, peptide or binding mimetic, to produce the bispecific molecule. For example, the "bispecific antibody" refers to one including two variable domains or ScFv units such that the antibody obtained recognizes two different antigens. Various different forms and uses of the bispecific antibody are known in the art (Chames P et al., Curr. Opin. Drug Disc. Dev., 12:.276, 2009; Spiess C et al., Mol. Immunol., 67: 95-106, 2015).

The term "hCG-β carboxy terminal peptide (CTP)" is a short peptide from the carboxy terminus of a human chorionic gonadotropin (hCG) β-subunit. Four reproduction-related polypeptide hormones, follicle-stimulating hormone (FSH), luteinizing hormone (LH), thyroid-stimulating hormone (TSH), and human chorionic gonadotropin (hCG), each contain the same α-subunit and their respective specific β-subunits. The *in vivo* half-life of hCG is significantly longer than those of the other three hormones, mainly due to the specific carboxy terminal peptide (CTP) on the β-subunit of hCG. The CTP includes 37 amino acid residues and four O-glycosylation sites, in which sugar side chain terminals are sialic acid residues. The electronegative highly-sialyl CTP can resist renal clearance and thus extend the half-life *in vivo* (Fares F A et al., Proc Natl Acad. Sci. USA, 1992, 89: 4304-4308, 1992).

The term "glycosylation" means that an oligosaccharide (a carbohydrate containing two or more monosaccharides that are linked together, e.g., a carbohydrate containing 2 to about 12 monosaccharides that are linked together) is attached to form a glycoprotein. The oligosaccharide side chains are generally linked to the backbone of the glycoprotein via N- or O-linkages. The oligosaccharides of the antibodies disclosed herein are generally CH2 domains linked to the Fc region as *N*-linked oligosaccharides. "*N*-linked glycosylation" refers to carbohydrate moiety attachment to an asparagine residue of a glycoprotein chain. For example, the skilled artisan can recognize a single site useful for *N*-linked glycosylation at residue 297 of each of CH2 domains of murine IgG1, IgG2a, IgG2b and IgG3 and human IgG1, IgG2, IgG3, IgG4, IgA and IgD.

### Homologous antibody

In another aspect, amino acid sequences included in the heavy and light chain variable regions of the antibody of the present disclosure are homologous with amino acid sequences of the preferred antibody described herein, and the antibody retains desired functional properties of the bispecific antibody of the present disclosure, for example, Her2×CD3 bispecific antibody.

### Antibody with conservative modifications

The term "conservative modification" is intended to mean that an amino acid modification does not significantly affect or change the binding characteristics of the antibody containing an amino acid sequence. Such conservative modifications include amino acid substitutions, additions and deletions. A modification may be introduced into the antibody of the present disclosure by using a standard technology known in the art, such as a site-directed mutagenesis and a PCR-mediated mutagenesis. A conservative amino acid substitution refers to the substitution of an amino acid residue with an amino acid residue with a similar side chain. Families of amino acid residues with similar side chains have been described in detail in the art. These families include amino acids with basic side chains (such as lysine, arginine and histidine), amino acids with acidic side chains (such as aspartic acid and glutamic acid), amino acids with uncharged polar side chains (such as glycine, asparagine, glutamine, serine, threonine, tyrosine, cysteine and tryptophan), amino acids with non-polar side chains (such as alanine, valine, leucine, isoleucine, proline, phenylalanine and methionine), amino acids with β-branched side chains (such as threonine, valine and isoleucine) and amino acids with aromatic side chains (such as tyrosine, phenylalanine, tryptophan and histidine). Therefore, one or more amino acid residues in the CDR of the antibody of the present disclosure may be substituted with other amino acid residues from the same side chain family.

### Fc variant with enhanced binding affinity to a neonatal receptor (FcRn)

"FcRn" used herein refers to a protein that binds to the Fc region of an IgG antibody and is encoded at least in part by an FcRn gene. FcRn may be derived from any organism including, but not limited to, humans, mice, rats, rabbits and monkeys. The functional FcRn protein includes two polypeptides which are generally referred to as a heavy chain and a light chain wherein the light chain is β-2-microgiobuiin and the heavy chain is encoded by an FcRn gene.

The plasma half-life of IgG depends on its binding to FcRn, where IgG generally binds to FcRn at a pH of 6.0 and dissociates from FcRn at a pH of 7.4 (the pH of plasma). Through studies on the binding site, a binding site of IgG to FcRn is modified to increase the binding capacity at the pH of 6.0. It has been proved that mutations of some residues of a human Fcγ domain, which are essential to the binding to FcRn, can increase the serum half-life. It has been reported that mutations at T250, M252, S254, T256, V308, E380, M428, and N434 (EU numbering) can increase or decrease the binding affinity for FcRn (Roopenian et al., Nat. Rev. Immunol., 7: 715-725, 2007). Korean Patent No. KR 10-1027427 discloses trastuzumab (Herceptin, Genentech) variants with enhanced binding affinity for FcRn, where these variants include one or more amino acid modifications selected from 257C, 257M, 257L, 257N, 257Y, 279Q, 279Y, 308F, and 308Y. Korean Patent Publication No. KR 2010-0099179 provides bevacizumab (Avastin, Genentech) variants, where these variants exhibit an increased *in vivo* half-life through amino acid modifications included in N434S, M252Y/M428L, M252Y/N434S, and M428L/N434S. In addition, Hinton et al. have found that T250Q and M428L mutants increase the binding to FcRn threefold and sevenfold, respectively. At the same time, the mutation of two sites increases the binding 28-fold. In rhesus monkeys, the M428L or T250QM/428 L mutant exhibits the plasma half-life increased twofold (Hinton P.R. et al., J. Immunol., 176: 346-356, 2006). For more mutational sites included in the Fc variant with the enhanced binding affinity for the neonatal receptor (FcRn), see Chinese invention patent CN 201280066663.2. In addition, studies have shown that through the T250Q/M428L mutation on Fc fragments of five humanized antibodies, the interaction between Fc and FcRn is improved, and in subsequent *in vivo* pharmacokinetic assays, the pharmacokinetic parameters of the Fc-mutation antibody are improved compared with the wild-type antibody through subcutaneous administration, for example, the *in vivo* exposure is increased, the clearance rate is reduced, and the subcutaneous bioavailability is increased (Datta-Mannan A et al., MAbs. Taylor&Francis, 4: 267-273, 2012).

Other mutational sites capable of enhancing the affinity of the antibody of the present disclosure for FcRn include, but are not limited to, the following amino acid modifications: 226, 227, 230, 233, 239, 241, 243, 246, 259, 264, 265, 267, 269, 270, 276, 284, 285, 288, 289, 290, 291, 292, 294, 298, 299, 301, 302, 303, 305, 307, 309, 311, 315, 317, 320, 322, 325, 327, 330, 332, 334, 335, 338, 340, 342, 343, 345, 347, 350, 352, 354, 355, 356, 359, 360, 361, 362, 369, 370, 371, 375, 378, 382, 383, 384, 385, 386, 387, 389, 390, 392, 393, 394, 395, 396, 397, 398, 399, 400, 401, 403, 404, 408, 411, 412, 414, 415, 416, 418, 419, 420, 421, 422, 424, 426, 433, 438, 439, 440, 443, 444, 445, and 446, where the numbers of the amino acids in the Fc region is numbers of the EU indexes in Kabat.

Fc variants with enhanced binding affinity for FcRn also include all other known amino acid modification sites as well as amino acid modification sites that have not yet been found.

In an optional embodiment, the IgG variant can be optimized to gain increased or decreased affinity for FcRn and increased or decreased affinity for human FcγR including, but not limited to, FcγRI, FcγRIIa, FcγRIIb, FcγRIIc, FcγRIIIa and FcγRIIIb, including allelic variants thereof.

Preferentially, the Fc ligand specificity of an IgG variant determines its therapeutic application. The given IgG variant for therapeutic purposes depends on the epitope or form of the target antigen as well as the to-be-treated disease or indication. Enhanced FcRn binding may be more preferred for most targets and indications because enhanced FcRn binding may result in extended serum half-life. A relatively long serum half-life allows administration at relatively low frequencies and doses during treatment. This property may be particularly preferred when the therapeutic agent is administered in order to respond to indications requiring repeated administration. For some targets and indications, the reduced affinity for FcRn may be particularly preferred when the variant Fc is required to have an increased clearance or reduced serum half-life, for example, when the Fc polypeptide is used as an imaging agent or a radiotherapy agent.

The affinity of the polypeptide for FcRn can be evaluated by methods well known in the art. For example, those skilled in the art can perform appropriate ELISA assays. As illustrated in Example 5.6, appropriate ELISA assays enabled the comparison of the binding strengths of the variants and parents to FcRn. At the pH of 7.0, the specific signals detected for the variant and the parent polypeptide are compared, if the specific signal of the variant is at least 1.9 times weaker than the specific signal of the parent polypeptide, this variant is a preferred variant of the present disclosure and is more suitable for clinical application.

FcRn may be derived from any organism including, but not limited to, humans, mice, rats, rabbits or monkeys.

### Alterations to inhibit FcγR binding

As used herein, "alterations to inhibit FcγR binding" refers to one or more insertions, deletions or substitutions in the Fc polypeptide chain that inhibit binding of FcγRIIA, FcγRIIB and/or FcγRIIIA, in which the binding is determined, for example, by a competitive binding assay (PerkinElmer, Waltham, MA). These alterations may be included in the Fc polypeptide chain as part of the bispecific antibody. More specifically, alterations that inhibit binding of the Fcγ receptor (FcγR) include L234A, L235A, or any alteration that inhibits glycosylation at the position N297, including any substitution at N297. In addition, along with the alterations that inhibit glycosylation of the position N297, additional alterations to stabilize the dimer Fc region by establishing additional disulfide bridges are also expected. Further examples of alterations that inhibit FcyR binding include D265A alterations in one Fc polypeptide chain and A327Q alterations in another Fc polypeptide chain. Some of the above mutations are described, for example, in Xu D et al., Cellular Immunol., 200: 16-26, 2000, of which the section about the above mutations and the activity evaluation thereof is incorporated herein by reference. The above numbers are based on EU numbering.

For example, the Fc fragment included by the bispecific antibody provided by the present disclosure in the alterations that inhibit FcγR binding exhibits reduced affinity for at least one of human FcγR (FcγRI, FcγRIIa or FcγRIIIa) or C1q, and has reduced effector cell functions or complement functions.

Other alterations that inhibit FcγR binding include sites and modifications thereof that are well known in the art or may be discovered in the future.

FcyR may be derived from any organism including, but not limited to, humans, mice, rats, rabbits or monkeys.

### Fc alterations to extend the half-life

As used herein, "Fc alterations to extend the half-life" refers to an alteration to extend the *in vivo* half-life of a protein that includes an altered Fc polypeptide in the Fc polypeptide chain as compared with the half-life of a protein that includes the same Fc polypeptide but does not include any altered but similar Fc. These alterations may be included in the Fc polypeptide chain as part of the bispecific antibody. Alterations T250Q, M252Y, S254T and T256E (alteration of threonine at position 250 to glutamine; alteration of methionine at position 252 to tyrosine; alteration of serine at position 254 to threonine; and alteration of threonine at position 256 to glutamic acid; where numbers are based on EU numbering) is an Fc alteration to extend half-life and may be used jointly, alone or in any combination. These and other alterations are described in detail in U.S. Pat. No. 7,083,784. The section about this alteration described in U.S. Pat. No. 7,083,784 is incorporated herein by reference.

Similarly, M428L and N434S are Fc alterations that extend the half-life and can be used jointly, alone or in any combination. These alterations and other alterations are described in detail in U.S. Patent Application Publication No. 2010/0234575 and U.S. Pat. No. 7,670,600. Sections of such alterations described in U.S. Patent Application Publication No. 2010/0234575 and U.S. Pat. No. 7,670,600 are incorporated herein by reference.

In addition, according to the meaning herein, any substitution at one of the following positions can be considered to be an Fc alternation that extends the half-life: 250, 251, 252, 259, 307, 308, 332, 378, 380, 428, 430, 434, and 436. Each of these alterations or a combination of these alterations may be used to extend the half-life of the bispecific antibody described herein. Other alternations that can be used to extend the half-life are described in detail in International Application No. PCT/US2012/070146 (Publication No. WO 2013/096221) filed December 17, 2012. The section about the above alterations of this application is incorporated herein by reference.

Fc alternations that extend the half-life also include sites and modifications thereof that are well known in the art or may be discovered in the future.

Fc may be derived from any organism including, but not limited to, humans, mice, rats, rabbits or monkeys.

### Nucleic acid encoding bispecific antibody

Using the therapeutic agent, the antibody or the antibody fragment described herein, those skilled in the art may easily construct multiple clones containing functionally equivalent nucleic acids (e.g., nucleic acids with different sequences but encoding the same effector moiety or antibody sequence). Therefore, the present disclosure provides bispecific antibodies, nucleic acids encoding the antibodies, antibody fragments and conjugates and fusion protein thereof, and variants, derivatives and species homologues of the nucleic acids.

Many nucleic acid sequences encoding immunoglobulin regions including VH, VL, hinge, CH1, CH2, CH3 and CH4 regions are known in the art. See, for example, Kabat et al.., Sequences of Proteins of Immunological Interest, Public Health Service N.I.H., Bethesda, MD, 1991. According to the teachings provided herein, those skilled in the art may combine the nucleic acid sequences and/or other nucleic acid sequences known in the art to construct a nucleic acid sequence encoding the bispecific antibody of the present disclosure. An exemplary nucleotide encoding the bispecific antibody of the present disclosure includes (1) SEQ ID NO: 56.

In addition, based on the amino acid sequences provided herein and elsewhere and the common knowledge in the art, those skilled in the art may determine the nucleic acid sequence encoding the bispecific antibody of the present disclosure. In addition to more conventional methods for producing cloned DNA fragments encoding particular amino acid sequences, companies such as DNA 2.0 (Menlo Park, CA, USA) and Blue Heron (Bothell, WA, USA) generally employ chemical synthesis to produce DNA that has any size of genes arranged in a desired order, thus simplifying the process of producing the DNA.

### Method for preparing a bispecific antibody

The bispecific antibody of the present disclosure may be prepared by any method known in the art. Early methods for constructing the bispecific antibody include chemical cross-linking or hybridoma heterozygosis or quadroma method (e.g., Staerz UD et al., Nature, 314: 628-31, 1985; Milstein C et al., Nature, 305: 537-540, 1983; Karpovsky B et al., J. Exp. Med., 160: 1686-1701, 1984). The chemical coupling method is to connect two different monoclonal antibodies by chemical coupling to prepare a bispecific monoclonal antibody. For example, two different monoclonal antibodies chemically bind to each other, or two antibody fragments, for example, two Fab fragments chemically bind to each other. The heterozygosis-hybridoma method is to prepare a bispecific monoclonal antibody by a cell hybridization method or a ternary hybridoma method, where the cell hybridoma or the ternary hybridoma is obtained by the fusion of constructed hybridomas or the fusion of a constructed hybridoma and lymphocytes derived from mice. Although these techniques are used to manufacture BiAb, various generation problems make such complexes difficult to use, such as the generation of mixed populations containing different combinations of antigen-binding sites, difficulties in protein expression, the need for purifying the target BiAb, low yields, and high production costs.

Recent methods utilize genetically engineered constructs that can produce a single homogeneous product of BiAb so that there is no need for thorough purification to remove unwanted by-products. Such constructs include tandem scFv, diabodies, tandem diabodies, double variable domain antibodies, and heterdimeric antibodies using the Ch1/Ck domain or DNLTM motifs (Chames & Baty, Curr. Opin. Drug. Discov. Devel., 12: 276-83, 2009; Chames & Baty, mAbs, 1: 539-47). Related purification techniques are well known.

The antibody may also be produced by cloning and expressing immunoglobulin variable region cDNAs produced from a single lymphocyte selected to produce the specific antibody using a single lymphocyte antibody method, for example, by a method described in Babcook J et al., Proc. Natl. Acad. Sci. USA. 93: 7843-7848, 1996; WO92/02551; WO2004/051268 and WO2004/106377.

Antigen polypeptides for producing, for example, antibodies for immunizing hosts or for screening, for example, antibodies for phage display (or the expression on the surface of yeast cells or bacterial cells) may be prepared from genetically engineered host cells including expression systems by methods well known in the art, or they may be recycled from natural biological sources. For example, nucleic acids encoding one or two polypeptide chains of the bispecific antibody may be introduced into cultured host cells by a variety of known methods (such as transformation, transfection, electroporation and bombardment with nucleic acid-coated microparticles, etc.). In some embodiments, the nucleic acids encoding the bispecific antibody may be inserted into a vector suitable to be expressed in the host cell before introduced into the host cell. A typical vector may include sequence elements that enable the inserted nucleic acids to be expressed at RNA and protein levels.

The vector is well known in the art and many vectors are commercially available. The host cell containing the nucleic acids may be cultured under conditions that enable the cell to express the nucleic acids, and the resulting BiAb may be collected from a cell population or a culture medium. Optionally, the BiAb may be produced *in vivo,* for example, in a plant leaf (see, for example, Scheller J et al., Nature Biotechnol., 19: 573-577, 2001 and references cited therein), in a bird egg (see, for example, Zhu L et al., Nature Biotechnol., 23: 1159-1169, 2005 and references cited therein) or in milk of a mammal (see, for example, Laible G et al., Reprod. Fertil. Dev., 25: 315, 2012).

Various cultured host cells that may be used include, for example, prokaryotic cells, eukaryotic cells, bacterial cells (such as *Escherichia coli* or *Bacilisstearothermophilus*), fungal cells (such as Saccharomyces cerevisiae or Pichia pastoris), insect cells (such as lepidopteran insect cells including Spodoptera frugiperda cells), or mammalian cells (such as Chinese hamster ovary (CHO) cells, NS0 cells, baby hamster kidney (BHK) cells, monkey kidney cells, Hela cells, human hepatocellular carcinoma cells or 293 cells).

The bispecific antibody may be prepared by immunizing an appropriate subject (such as a rabbit, a goat, a mouse, or other mammals, including transgenic and knocked-out mammals) with an immunogenic preparation of the bispecific antigen. An appropriate immunogenic preparation may be, for example, a chemically synthesized or recombinantly expressed bispecific antigen. The preparation may further include adjuvants such as Freund's complete or incomplete adjuvants or similar immunostimulatory compounds. Furthermore, the bispecific antigen of the present disclosure may be used alone or preferably used as a conjugate with a vector protein when used for preparing antibodies, in particular by *in vivo* immunization. Such methods of enhancing an antibody response are well known in the art. Depending on different antibodies required, different animal hosts may be used for in vivo immunization. A host that expresses an endogenous antigen of interest itself may be used, or a host that has been caused deficient in an endogenous antigen of interest may be used.

The bispecific antibody may be prepared by a combination of the methods described above.

The bispecific antibody molecule of the present disclosure may act as a monoclonal antibody (MAb) for each target. In some embodiments, the antibody is chimeric, humanized or fully human.

The monoclonal antibody may be prepared by any method known in the art, such as a hybridoma technique (Kohler&Milstein, Nature, 256: 495-497, 1975), a trioma technique, a human B-cell hybridoma technique (Kozbor D et al., Immunology Today, 4: 72, 1983), and an EBV-hybridoma technique (Cole SPC et al., Monoclonal Antibodies and Cancer Therapy, pp 77-96, Alan R Liss, Inc., 1985).

The bispecific antibody or a portion thereof in the present disclosure may be used for detecting any or all of these antigens (for example, in biological samples such as serum or plasma) by conventional immunological analysis methods such as an enzyme-linked immunosorbent assay (ELISA), a radioimmunoassay (RIA) or tissue immunohistochemistry. The present disclosure provides a method for detecting an antigen in a biological sample, comprising: contacting a biological sample with the bispecific antibody or the antibody portion of the present disclosure that specifically recognizes the antigen, and detecting an antigen-bound antibody (or antibody portion) or a non-bound antibody (or antibody portion), so as to detect the antigen in the biological sample. The antibody is directly or indirectly labeled with a detectable substance to facilitate the detection of the bound or non-bound antibody. Suitable detectable substances include various enzymes, repair groups, fluorescent substances, luminescent substances and radioactive substances. Examples of suitable enzymes include horseradish peroxidase, alkaline phosphatase, β-galactosidase and acetylcholinesterase; examples of suitable repair group complexes include streptavidin/biotin and avidin/biotin; examples of suitable fluorescent substances include 7-hydroxycoumarin, fluorescein, fluorescein isothiocyanate, rhodamine, dichlorotriazinylamine fluorescein, dansyl chloride or phycoerythrin; an example of the luminescent substance includes 3-aminophthalhydrazide; examples of suitable radioactive substances include I¹²⁵, I¹³¹, ³⁵S or ³H.

### Cancer

The term "cancer" refers to a broad class of diseases characterized by uncontrolled growth of abnormal cells *in vivo.* "Cancer" includes benign and malignant cancers as well as dormant tumors or micrometastases. Cancer includes primary malignant cells or tumors (e.g., tumors in which cells have not migrated to a site other than the site of the original malignant disease or tumor in the subject) and secondary malignant cells or tumors (e.g., tumors resulting from metastasis in which cells are metastasized to malignant cells or tumor cells and then migrate to a secondary site different from the original tumor site). Cancers also include hematologic malignancies. "Hematologic malignancies" include lymphomas, leukemias, myelomas or lymphoid malignancies, as well as spleen cancer and lymph node tumors.

In a preferred embodiment, the bispecific antibody of the present disclosure or nucleic acids or polynucleotides encoding the antibody of the present disclosure or immunoconjugates or pharmaceutical compositions or combination therapies are useful for the treatment, prevention or alleviation of cancer. Examples of cancers include, but are not limited to, carcinomas, lymphomas, glioblastomas, melanomas, sarcomas, leukemia, myelomas or lymphoid malignancies. More specific examples of such cancers are described below and include: squamous cell carcinoma (e.g., epithelial squamous cell carcinoma), Ewing's sarcoma, Wilms' tumor, astrocytoma, lung cancer (including small-cell lung cancer, non-small-cell lung cancer, lung adenocarcinoma, and lung squamous-cell carcinoma), peritoneal carcinoma, hepatocellular carcinoma, stomach or gastric cancer (including gastrointestinal cancer), pancreatic cancer, glioblastoma multiforme, cervical cancer, ovarian cancer, liver cancer, bladder cancer, hepatoma, hepatocellular carcinoma, neuroendocrine tumor, medullary thyroid cancer, differentiated thyroid cancer, breast cancer, ovarian cancer, colon cancer, rectal cancer, endometrial or uterine carcinoma, salivary gland tumors, kidney or renal carcinoma, prostate cancer, vaginal cancer, anal cancer, penile cancer, and head and neck cancer.

The bispecific antibody, the nucleic acid or polynucleotide encoding the antibody of the present application, the immunoconjugate, the pharmaceutical composition or the combination therapy of the present disclosure may be used for treating malignant or premalignant conditions and for preventing the development into neoplastic or malignant conditions, which include, but are not limited to, those conditions described above. Such uses are indicated for conditions that are known or suspected to have previously progressed to neoplasia or cancer. Specifically, non-neoplastic cell growth consists of hyperplasia and metaplasia. Most specifically, developmental abnormalities occur (for the summary of such abnormal growth conditions, see Robbins and Angell, Basic Pathology., 2nd Edition, W.B. Saunders Co., Philadelphia, pp 68-79, 1976).

### Pharmaceutical composition

The bispecific antibody of the present disclosure or the nucleic acids or polynucleotide (such as Her2xCD3) encoding the antibody of the present application may be applied to the preparation of the pharmaceutical composition or a sterile composition. For example, the bispecific antibody is mixed with a pharmaceutically acceptable carrier, excipient or stabilizer. The pharmaceutical composition may include one bispecific antibody or a combination of (for example, two or more different) bispecific antibodies of the present disclosure. For example, the pharmaceutical composition of the present disclosure may include a combination of antibodies or antibody fragments (or immunoconjugates) that have complementary activity and bind to different epitopes on a target antigen. Formulations of therapeutic and diagnostic agents may be prepared by mixing the antibody with the pharmaceutically acceptable carrier, excipient or stabilizer in the form of, for example, lyophilized powder, slurry, an aqueous solution or a suspension.

The term "pharmaceutically acceptable" means that a molecule, molecule fragment or composition does not produce unfavorable, allergic or other adverse effects when properly administered to animals or humans. Specific examples of some substances that may act as the pharmaceutically acceptable carriers or components thereof include sugars (such as lactose), starch, cellulose and its derivatives, vegetable oils, gelatin, polyols (such as propylene glycol), alginic acid and the like.

In some preferred embodiments, the pharmaceutical composition of the present disclosure is used for treating, preventing or alleviating diseases including, but not limited to, cancer, lymphatic system diseases, autoimmune diseases, inflammatory diseases, infectious diseases, immunodeficiency syndromes and other related diseases or symptoms.

TAAs that can be targeted include, but are not limited to, those described above.

The bispecific antibody of the present disclosure or the nucleic acid or polynucleotide encoding the antibody of the present application may be used alone or in combination with one or more other therapeutic agents such as toxins, cytotoxic agents, radioisotopes, immunotherapeutic agents or vaccines. The therapeutic agents include, but are not limited to, antineoplastic agents such as doxorubicin (adriamycin), cisplatin bleomycin sulfate, nitrosourea nitrogen mustard, chlorambucil and cyclophosphamide hydroxyurea. These therapeutic agents themselves are effective only at the level of toxicity or sub-toxicity to patients. The above therapeutic agents are well known in the art and also include therapeutic agents that may be developed in the future.

The bispecific antibody of the present disclosure may be administered along with, for example, effector cell co-stimulatory molecules, effector cells, DC cell surface molecules, DC cells, molecules activating T cells (Hutloff A et al., Nature, 397: 262-266, 1999). Targeted effector cells may be human leukocytes such as macrophages, neutrophils or monocytes. Other cells include eosinophils, NK cells and other cells with IgG or IgA receptors. If required, effector cells may be obtained from subjects to be treated.

The bispecific antibody of the present disclosure may be administered in combination with, for example, immunomodulators or immunogenic agents such as cancer cells, purified tumor antigens (including recombinant proteins, peptides and carbohydrate molecules) or cells transfected with genes encoding immunostimulatory cytokines (He YF et al., J. Immunol., 173: 4919-28, 2004).

The bispecific antibody of the present disclosure or the nucleic acid or polynucleotide encoding the antibody of the present application may also be administered in combination with, for example, standard cancer therapies (such as a surgery, radiation and chemotherapy). For example, anti-tumor therapy using the compositions of the present disclosure and/or effector cells equipped with these compositions is used in combination with chemotherapy. Non-limiting examples of antibody combination therapies of the present disclosure include surgery, chemotherapy, radiotherapy, immunotherapy, gene therapy, DNA therapy, RNA therapy, nano therapy, viral therapy, adjuvant therapy and combinations thereof.

The compositions of the present disclosure may be in various forms. The forms include, for example, liquid, semi-solid and solid dosage forms such as liquid solutions (e.g. injectable and non-molten solutions), dispersants, suspension tablets, pills, powders, liposomes and suppositories. A preferred manner depends on the mode of administration and the therapeutic use. Preferred typical compositions are injectable or non-molten solutions, such as those compositions that passively immunize humans like other antibodies. The compositions of the present disclosure may be administered through various routes including oral, rectal, transmucosal, trans-intestinal, parenteral; intramuscular, subcutaneous, intradermal, intramedullary, intrathecal, direct intraventricular, intravenous, intraperitoneal, intranasal, intraocular, inhalation, insufflation, topical, skin, transdermal or intraarterial. A preferred mode of administration is parenteral (e.g., intravenous, subcutaneous, intraperitoneal, intramuscular). In a preferred embodiment, the antibody is administered by intravenous infusion or injection. In another preferred embodiment, the antibody is injected intramuscularly or subcutaneously.

The above combination methods, treatment methods and administration methods are well known. Combination, treatment and administration methods that may be developed in the future are also included.

### Combination therapy

The present disclosure relates to uses of a combination of the bispecific antibody or nucleic acids or polynucleotides encoding the antibody of the present disclosure or immunoconjugates or pharmaceutical compositions and one or more active therapeutic agents (e.g., chemotherapeutic agents) or other prophylactic or therapeutic modes (e.g., radiation). In such combination therapies, the various active agents often have different complementary mechanisms of action, and the combination therapy may lead to synergistic effects. The combination therapy includes therapeutic agents that affect immune responses (e.g., an enhanced or activated response) and therapeutic agents that affect (e.g., inhibit or kill) tumor/cancer cells. The combination therapy may reduce the likelihood of drug-resistant cancer cells. The combination therapy may allow the dose reduction of one or more reagents to reduce or eliminate adverse effects associated with the one or more reagents. Such combination therapies may have synergistic therapeutic or prophylactic effects on underlying diseases, disorders or symptoms.

The "combination" includes therapies that can be administered separately, for example, separate formulations for individual administration (e.g., which may be provided in a kit), and therapies that can be administered together in a single formulation (i.e., "co-formulation"). In some embodiments, the bispecific antibody of the present disclosure or nucleic acids or polynucleotides encoding the antibody of the present disclosure or immunoconjugates or pharmaceutical compositions may be administered sequentially. In some embodiments, the bispecific antibody of the present disclosure or nucleic acids or polynucleotides encoding the antibody of the present disclosure or immunoconjugates or pharmaceutical compositions may be administered simultaneously. The bispecific antibody or nucleic acids or polynucleotides encoding the antibody of the present disclosure or immunoconjugates or pharmaceutical compositions may be used in any manner in combination with at least one other (active) agent.

Treatment with the bispecific antibody of the present disclosure may be combined with other treatments that are effective against the to-be-treated disease. Non-limiting examples of antibody combination therapies of the present disclosure include surgery, chemotherapy, radiation therapy, immunotherapy, gene therapy, DNA therapy, RNA therapy, nanotherapy, viral therapy, and adjuvant therapy.

Combination therapies also include all other combination therapies known in the art or developed in the future.

### Use

As described herein, the bispecific antibody, the nucleic acid or polynucleotide encoding the antibody of the present application, the immunoconjugate, the pharmaceutical composition or the combination therapy of the present application may be used in the treatment and diagnosis of cancer, autoimmune diseases, inflammatory diseases, lymphatic system diseases, infections or infectious diseases, immunodeficiency syndromes, and other related diseases or symptoms described herein. The treatment includes administering the bispecific antibody of the present application to a patient such as an animal, a mammal and a human, for the treatment or diagnosis of one or more diseases or symptoms described herein. Therapeutic compounds of the present application include, but are not limited to, the antibody (including variants, isotypes, derivatives and species homologues thereof as described herein) of the present application or the nucleic acids or polynucleotide (including variants, isotypes, derivatives and species homologues thereof as described herein) encoding the antibody of the present application.

The bispecific antibody, the nucleic acid or polynucleotide encoding the antibody of the present application, the immunoconjugate or the pharmaceutical composition of the present disclosure may be used for diagnostic purposes. In one embodiment, the antibody of the present disclosure is used for diagnostic tests *in vitro* such as laboratory tests for detecting antigens of interest or care tests for detecting antigens of interest. Established in vitro tests using an antibody include ELISAs, RIAs, Western blotting, etc. In another embodiment, the antibody of the present disclosure is used for diagnostic tests in vivo, such as imaging tests in vivo. For example, the antibody is labeled with a detectable substance that can be detected in vivo, the labelled antibody may be applied to a subject and the labelled antibody may be detected in vivo, whereby in vivo imaging may be performed. Related diagnostic techniques include other well-known techniques and techniques that may be developed.

The bispecific antibody of the present disclosure or the nucleic acid or polynucleotide encoding the antibody of the present application may be used for preparing an immunoconjugate. The immunoconjugate includes, but is not limited to, immunoconjugates described above. A linking manner includes, but is not limited to, those in the immunoconjugates described above.

The bispecific antibody, the nucleic acid or polynucleotide encoding the antibody of the present application, or the immunoconjugate of the present disclosure may be used for preparing a drug. The pharmaceutical composition includes, but is not limited to, pharmaceutical compositions described above. The bispecific antibody, the nucleic acid or polynucleotide encoding the antibody of the present application or the immunoconjugate of the present disclosure may be used alone or in combination with one or more other therapeutic agents. The therapeutic agents include, but are not limited to, those described above. In some preferred embodiments, the bispecific antibody, the nucleic acid or polynucleotide encoding the antibody of the present application or the immunoconjugate and other pharmaceutical active agents are provided as separate components or components of the same composition, or the bispecific antibody, the nucleic acid or polynucleotide encoding the antibody of the present application or the immunoconjugate may be administered with other known therapies. Other known therapies include, but are not limited to, those described above. Combination, treatment and administration methods of the pharmaceutical composition include, but are not limited to, those described above. Further provided are a container that includes the bispecific antibody, the nucleic acid or polynucleotide encoding the antibody of the present application, the immunoconjugate, or the pharmaceutical composition of the present disclosure, and instructions.

The present disclosure provides a combination therapy that includes the bispecific antibody, the nucleic acid or polynucleotide encoding the antibody of the present application, the immunoconjugate, or the pharmaceutical composition of the present disclosure. The combination therapy includes, but is not limited to, those described above.

The present disclosure further provides a detection method and a kit that include the bispecific antibody, the nucleic acid or polynucleotide encoding the antibody of the present application, the immunoconjugate, or the pharmaceutical composition of the present disclosure. Related content includes, but is not limited to, those described above.

The bispecific antibody, the nucleic acid or polynucleotide encoding the antibody of the present application, the immunoconjugate, or the pharmaceutical composition of the present application may be used for diagnosing, treating, inhibiting, or preventing diseases or symptoms, which include malignant diseases, disorders, or conditions related to such diseases or disorders, such as diseases related to increased cell survival or inhibited apoptosis, including, but are not limited to, cancer, autoimmune diseases, inflammatory diseases, lymphatic system diseases, infections or infectious diseases, immunodeficiency syndromes, and other related diseases or symptoms described above. For example, such diseases involve unregulated and/or inappropriate proliferation of cells, sometimes accompanied by destruction of adjacent tissues and new vascular growth, which may allow cancer cells to invade new regions, i.e. metastases.

The bispecific antibody, the nucleic acid or polynucleotide encoding the antibody of the present application, the immunoconjugate, or the pharmaceutical composition of the present application may be used for treating, preventing, diagnosing, and/or predicting other diseases or symptoms associated with increased cell survival, which include, but are not limited to, the development and/or metastasis of malignancies and the development and/or metastasis of the other related diseases or symptoms (such as inflammatory diseases, lymphatic system diseases, or infections or infectious diseases) described above.

The form of the bispecific antibody, the nucleic acid or polynucleotide encoding the antibody of the present application, the immunoconjugate, or the pharmaceutical composition includes, but is not limited to, those described above. The method for administering the bispecific antibody, the nucleic acid or polynucleotide encoding the antibody of the present application, the immunoconjugate, or the pharmaceutical composition of the present disclosure includes, but is not limited to, those described above. Treatment methods and combination therapies include, but are not limited to, those described above.

For example, the bispecific antibody, the immunoconjugate, or the pharmaceutical composition may be intravenously administered through, for example, bolus injection or continuous infusion or even administered to mammals by other parenteral means such as intravenous, intramuscular, intraperitoneal, or intravascular administration. Preferably, the bispecific antibody, the nucleic acid or polynucleotide encoding the antibody of the present application, the immunoconjugate, or the pharmaceutical composition is injected continuously for less than about 4 hours, and more preferably, for less than about 3 hours. An injectable formulation may be provided in a unit dosage form, for example, in ampoules or multi-dosage containers where preservatives are added. The composition may be in the form of for example, a suspension, solution, or emulsion in an oily or aqueous vehicle and may contain formulations such as suspensions, stabilizers, and/or dispersants. Alternatively, active components may be in the form of powder reconstituted with an appropriate vehicle (e.g. sterile pyrogen-free water) before used.

Various delivery systems are known and may be used for administering the antibody, the nucleic acid or polynucleotide encoding the antibody of the present application, the immunoconjugate, or the pharmaceutical composition of the present application, for example, encapsulation in liposomes, microparticles, microcapsules, recombinant cells capable of expressing the compound, or receptor-mediated endocytosis (see Wu GY etal., J. Biol. Chem., 262: 4429-4432, 1987), construction of the nucleic acid as a retrovirus or other vectors. The delivery systems also include all other well-known techniques and parts that may develop in the future.

Additional pharmaceutical methods may be used for controlling the duration of the action of the therapeutic composition, such as a controlled release system. Non-limiting examples of the controlled release system are pumps (see, for example, Sefton, CRC, Crit. Ref. Biomed. Eng., 14: 201, 1987; Buchwald H et al., Surgery, 88: 507, 1980), polymeric materials (see, for example, Medical Applications of Controlled Release, Langer and Wise, CRC Pres. Boca Raton, Fla., 1974), or the like. Such pharmaceutical methods also include all other well-known techniques and parts that may develop in the future.

A therapeutically effective dose of the bispecific antibody, the nucleic acid or polynucleotide encoding the antibody of the present application, the immunoconjugate, or the pharmaceutical composition may be administered. The therapeutically effective dose of the bispecific antibody, the polynucleotide encoding the antibody of the present application, the immunoconjugate, or the pharmaceutical composition may vary depending on an indication being treated, the weight of a patient, and the calculated surface area of the skin of the patient. The administration may be adjusted in order to achieve desired effects. In many cases, repeated administration may be required. For example, the administration may be conducted three times, twice, or once every week, once every two, three, four, five, six, seven, eight, nine, or ten weeks, or once every two, three, four, five, or six months. The bispecific antibody of the present application or the nucleic acid or polynucleotide encoding the antibody of the present application is generally administered to the patient at a dosage of about 0.001-100 mg/kg, for example, about 0.1-50 mg/kg, about 0.1-20 mg/kg, about 0.1-10 mg/kg, about 0.5 mg/kg, about 0.3 mg/kg, about 1 mg/kg, or about 3 mg/kg. Optionally, the dosage at which the antibody is administered may be adjusted based on the estimated surface area of the skin of the patient or based on the weight of the patient. The administration schedule may optionally be repeated at other intervals, and the dosage may be administered by various parenteral means with the appropriate adjustment of the dosage and a schedule.

In general, a human antibody has a longer half-life in humans than an antibody from other species, due to the immune response to exogenous polypeptides. Therefore, a low dose of the human antibody may be usually allowed to be administered at a lower frequency. In addition, the administration dosage and frequency of the antibody of the present application may be reduced by enhancing the absorption and tissue penetration (e.g., into the brain) of the antibody, which is achieved through modifications, e.g., lipidation.

The bispecific antibody of the present disclosure or the nucleic acid or polynucleotide encoding the antibody of the present application may be used for preparing a diagnostic or therapeutic kit which includes the antibody of the present disclosure or an antigen binding fragment thereof and/or instructions for use. The immunoconjugate of the present disclosure may be used for preparing a diagnostic or therapeutic kit which includes the immunoconjugate of the present disclosure and/or instructions for use. The pharmaceutical composition of the present disclosure may be used for preparing a diagnostic or therapeutic kit which includes the pharmaceutical composition of the present disclosure and/or instructions for use.

### BRIEF DESCRIPTION OF DRAWINGS

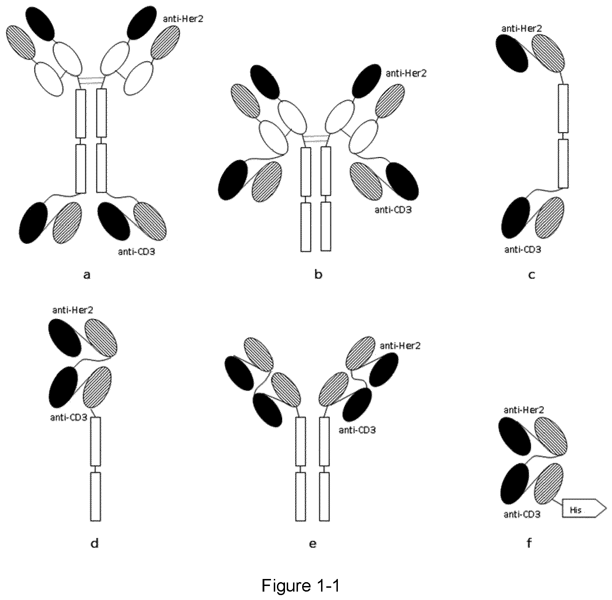

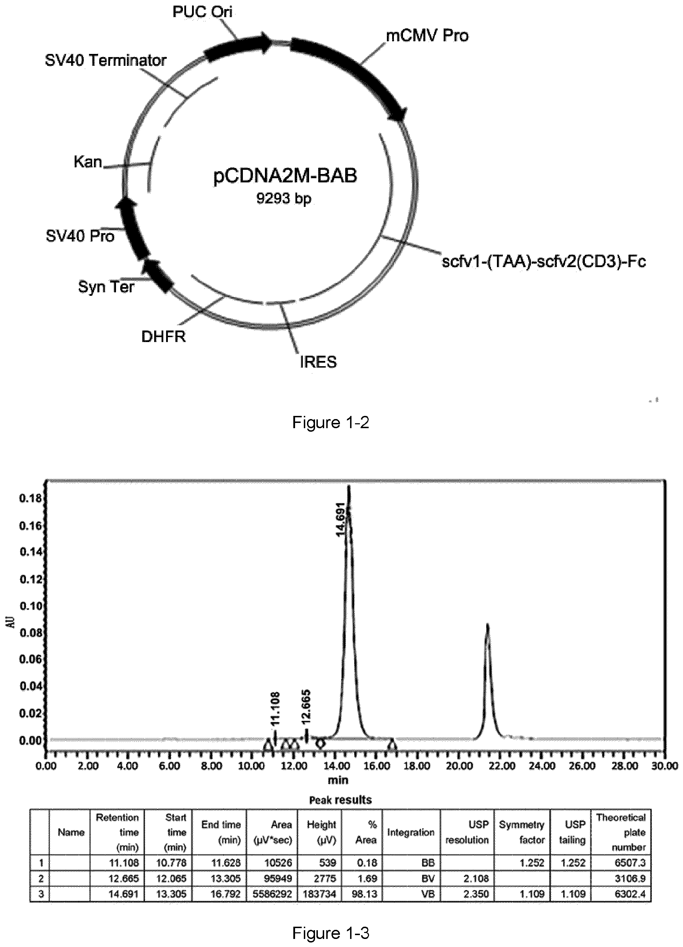

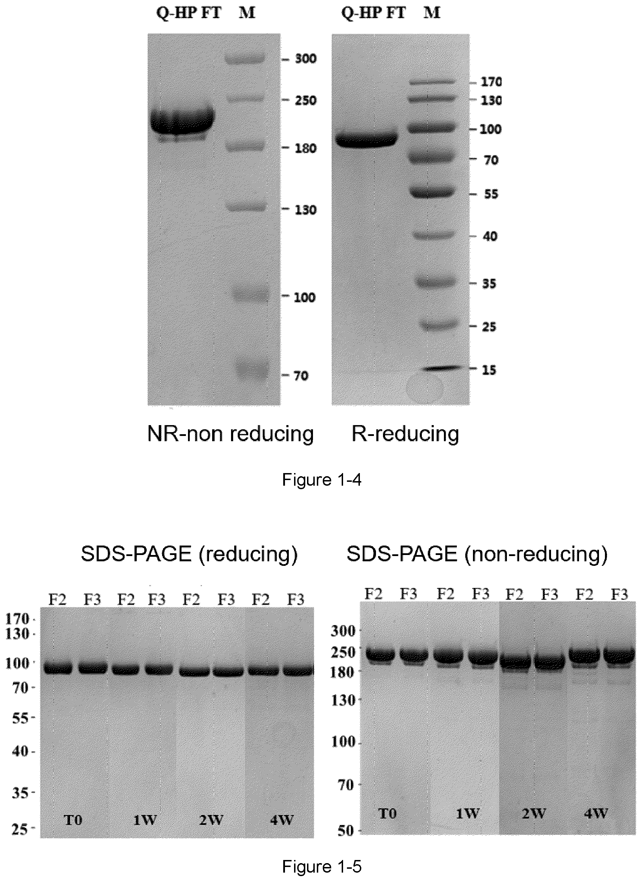

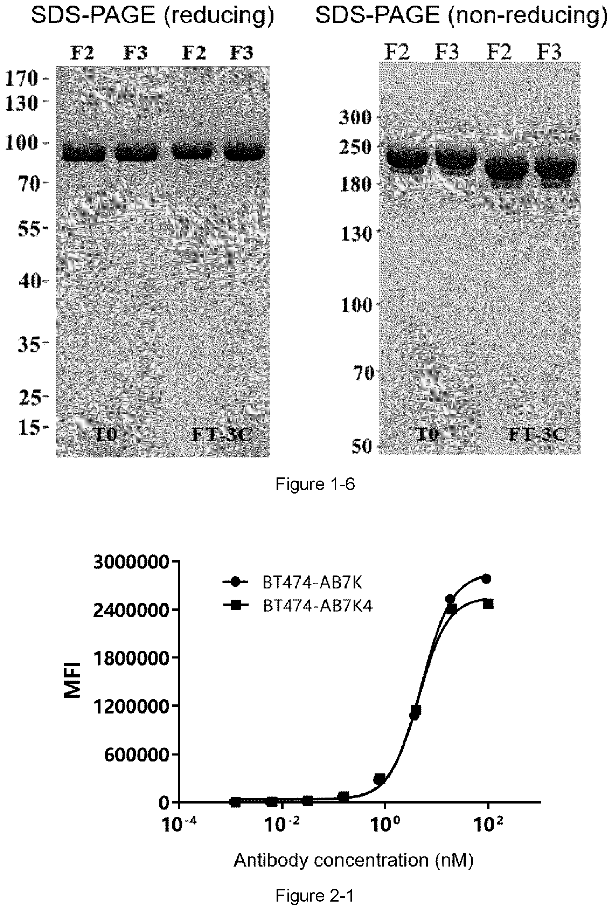

FIG. 1-1 illustrates configurations of bispecific antibodies AB7K, AB7K4, AB7K5, AB7K6, AB7K7 and AB7K8 as shown in a, b, c, d, e and f, respectively.
FIG. 1-2 illustrates an expression plasmid map of bispecific antibody AB7K7. The expression plasmid has a full length of 9293 bp and contains nine major gene fragments which are (1) an hCMV promoter, (2) target genes, (3) EMCV IRES, (4) mDHFR screening gene, (5) a Syn discontinuation sequence, (6) an SV40 promoter, (7) Kalamycin resistance gene; (8) an SV40 termination sequence, and (9) a PUC replicon.
FIG. 1-3 illustrates SEC-HPLC test results of purified samples of AB7K7.
FIG. 1-4 illustrates SDS-PAGE electrophoresis results of purified samples of AB7K7.
FIG. 1-5 illustrates SDS-PAGE results of AB7K7 in acceleration experiments at 25 °C.
FIG. 1-6 illustrates SDS-PAGE results of AB7K7 in freeze-thaw experiments.
FIG. 2-1 illustrates abilities, detected through an FACS, of bispecific antibodies AB7K and AB7K4 to bind to tumor cells BT474.
FIG. 2-2 illustrates abilities, detected through an FACS, of bispecific antibodies AB7K and AB7K5 to bind to tumor cells BT474.

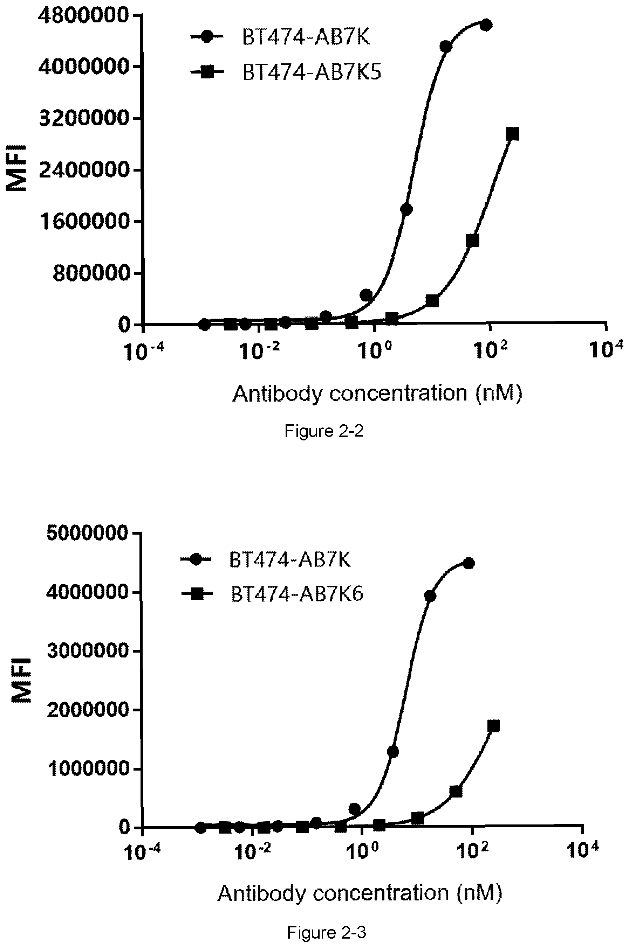

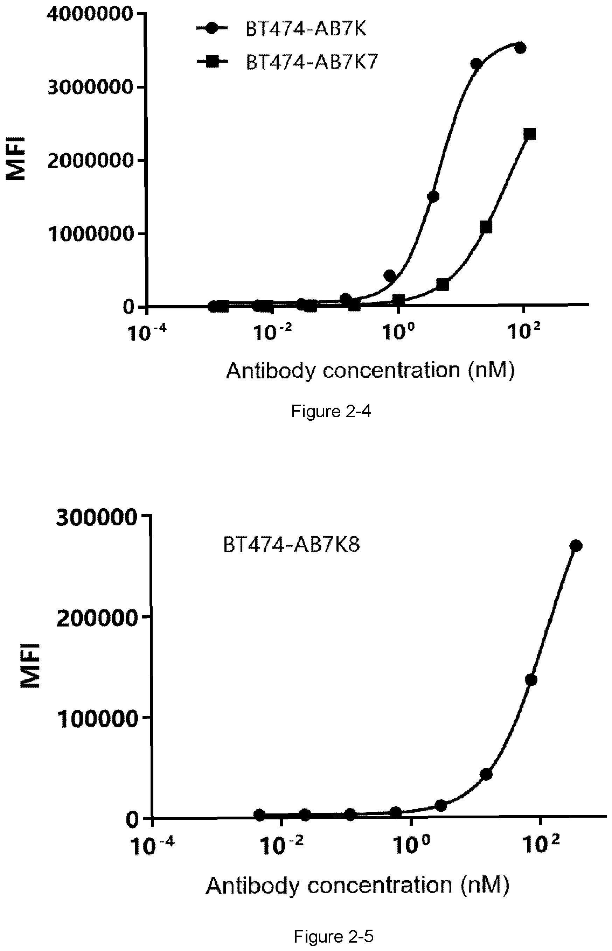

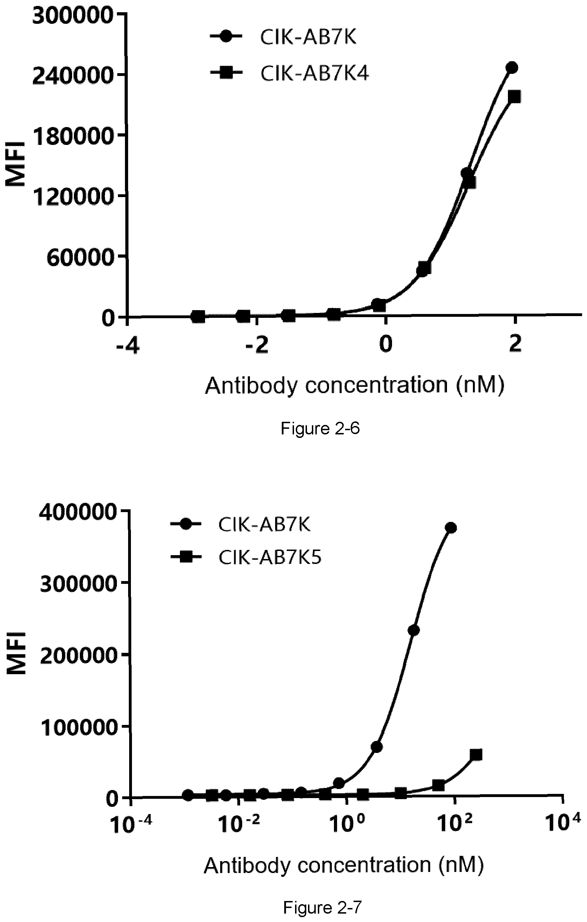

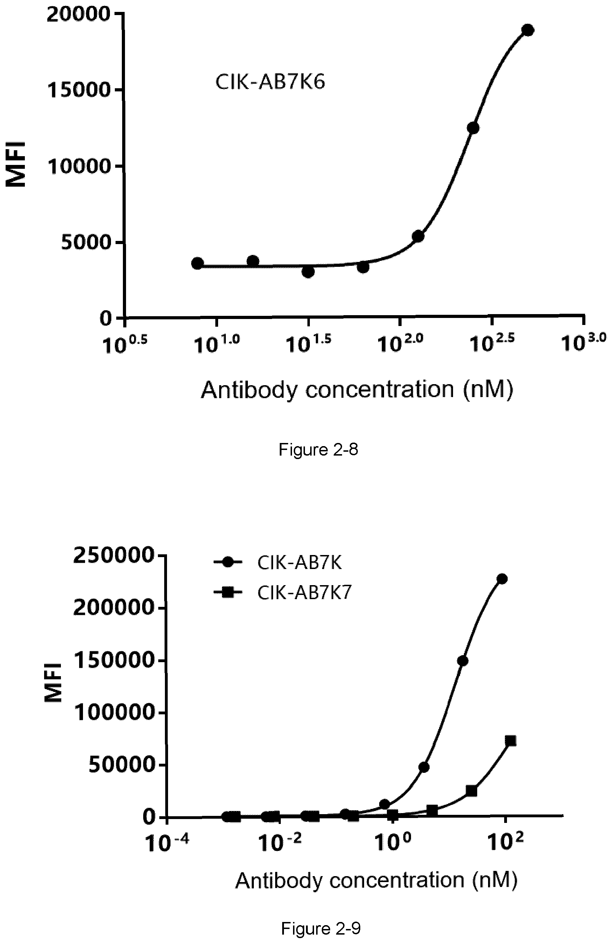

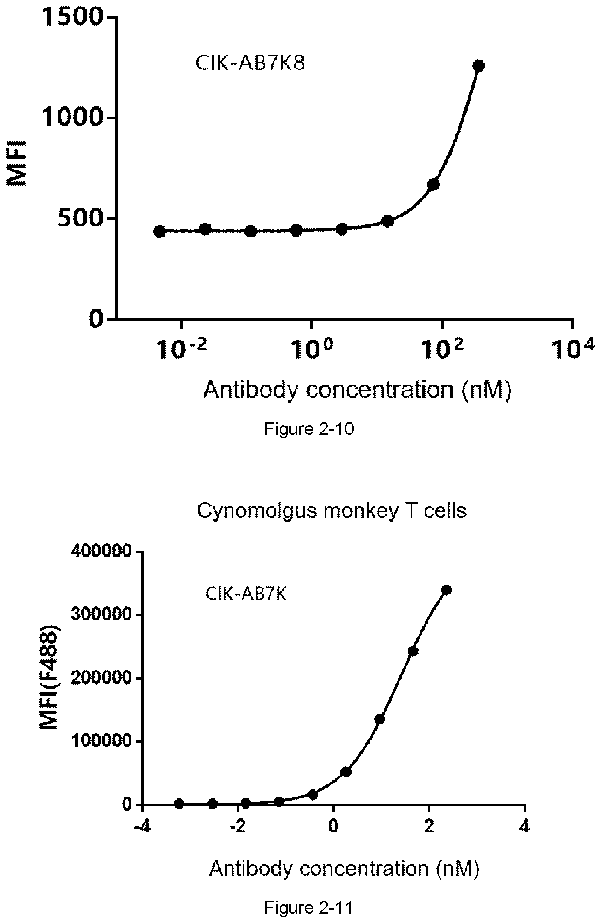

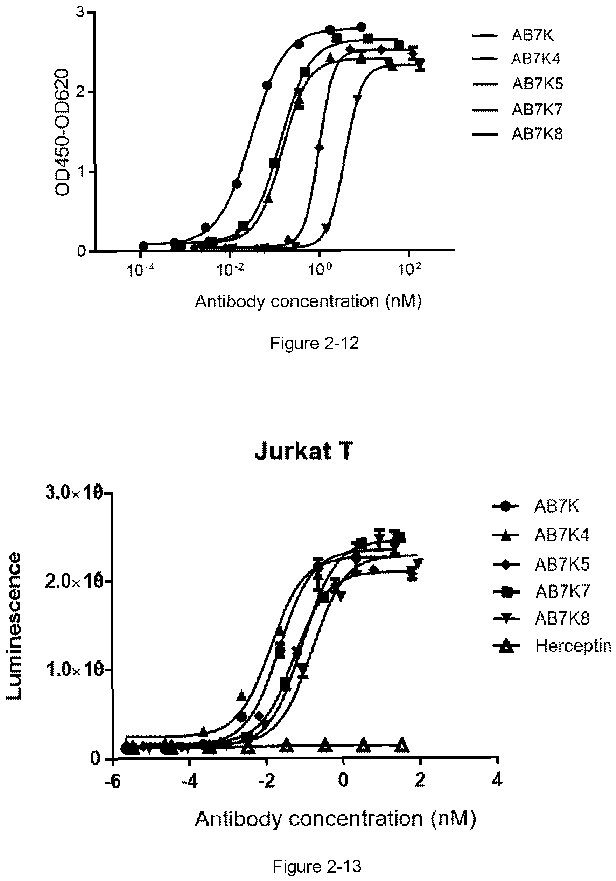

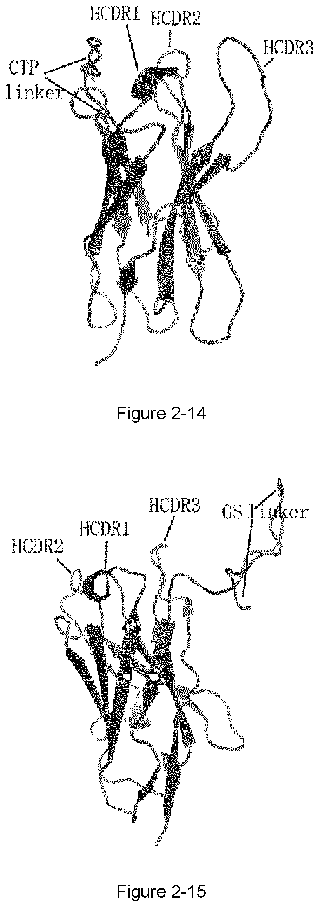

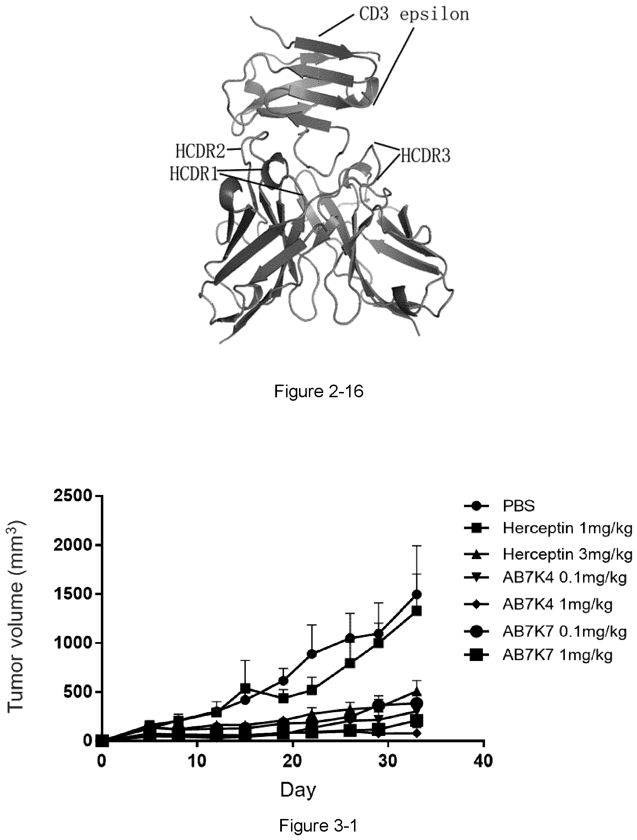

FIG. 2-3 illustrates abilities, detected through an FACS, of bispecific antibodies AB7K and AB7K6 to bind to tumor cells BT474.
FIG. 2-4 illustrates abilities, detected through an FACS, of bispecific antibodies AB7K and AB7K7 to bind to cancer cells BT474.
FIG. 2-5 illustrates an ability, detected through an FACS, of a bispecific antibody AB7K8 to bind to tumor cells BT474.
FIG. 2-6 illustrates abilities, detected through an FACS, of bispecific antibodies AB7K and AB7K4 to bind to effector cells CIK.
FIG. 2-7 illustrates abilities, detected through an FACS, of bispecific antibodies AB7K and AB7K5 to bind to effector cells CIK.
FIG. 2-8 illustrates an ability, detected through an FACS, of a bispecific antibody AB7K6 to bind to effector cells CIK.
FIG. 2-9 illustrates abilities, detected through an FACS, of bispecific antibodies AB7K and AB7K7 to bind to effector cells CIK.
FIG. 2-10 illustrates an ability, detected through an FACS, of a bispecific antibody AB7K8 to bind to effector cells CIK.
FIG. 2-11 illustrates an ability, detected through an FACS, of a bispecific antibody AB7K to bind to T cells of cynomolgus monkeys.
FIG. 2-12 illustrates abilities, detected through an ELISA, of five Anti-Her2xCD3 bispecific antibodies to bind to CD3 molecules and Her2 molecules.
FIG. 2-13 illustrates abilities, detected through a microplate reader, of five Anti-Her2×CD3 bispecific antibodies to activate Jurkat T cells of a reporter gene cell line.
FIG. 2-14 illustrates structural modeling of a CTP linker and anti-CD3 scFv VH.
FIG. 2-15 illustrates structural modeling of a GS linker and anti-CD3 scFv VH.
FIG. 2-16 illustrates a molecular docking model of anti-CD3 scFv and a CD3 epsilon chain.

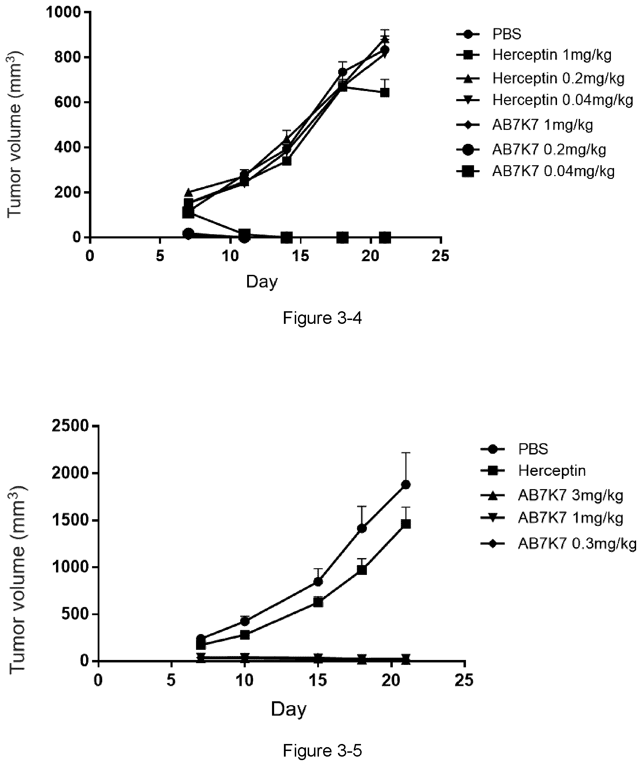

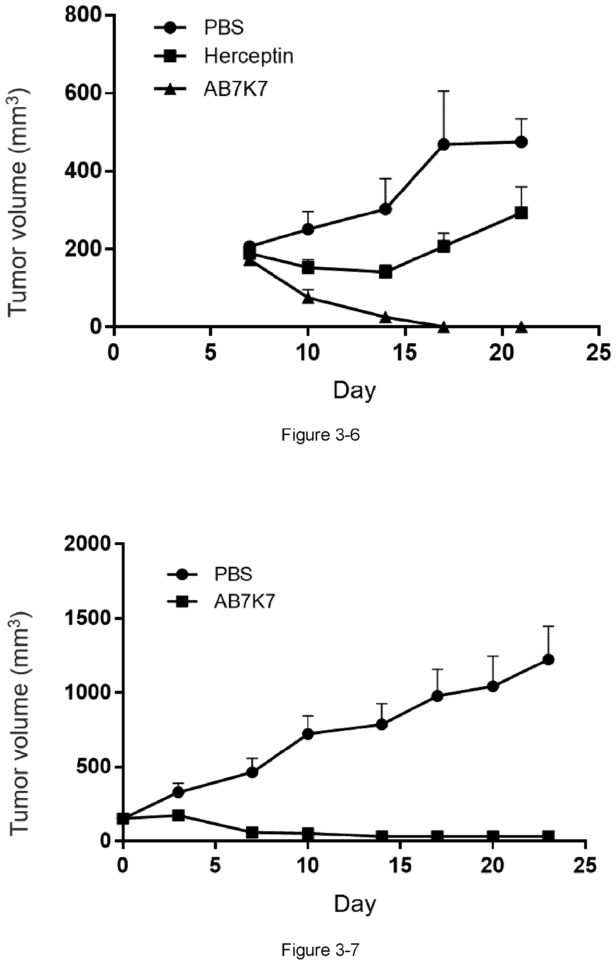

FIG. 3-1 illustrates in vivo anti-tumor effects of bispecific antibodies AB7K4 and AB7K7 in a transplanted tumor model in which human CIK cells and HCC1954 cells were co-inoculated subcutaneously in NCG mice.

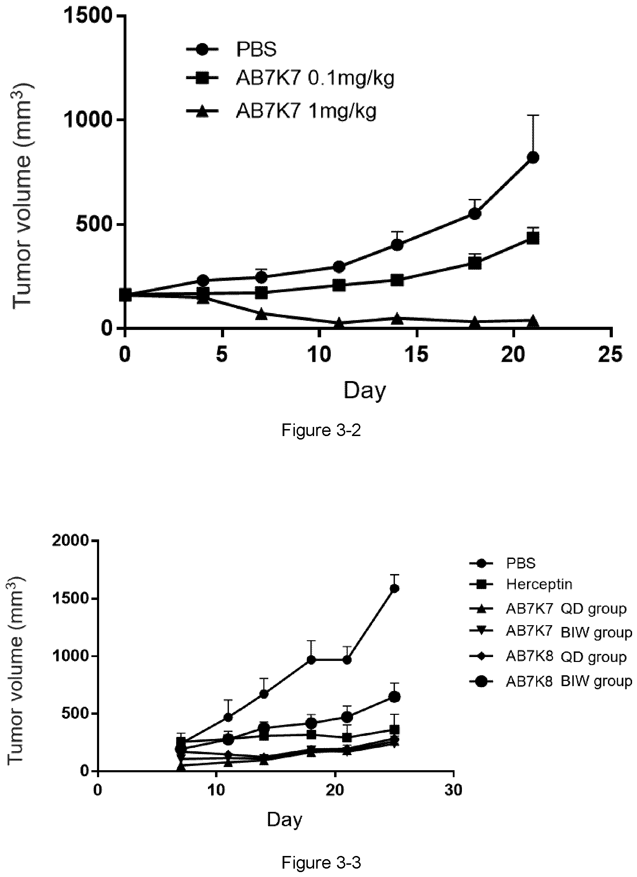

FIG. 3-2 illustrates an in vivo anti-tumor effect of a bispecific antibody AB7K7 in a transplanted tumor model in which human CIK cells and human breast cancer cells HCC1954 were co-inoculated subcutaneously in NPG mice.
FIG. 3-3 illustrates in vivo anti-tumor effects of bispecific antibodies AB7K7 and AB7K8 at different administration frequencies in a transplanted tumor model in which human CIK cells and human breast cancer cells HCC1954 were co-inoculated subcutaneously in NPG mice.
FIG. 3-4 illustrates an in vivo anti-tumor effect of a bispecific antibody AB7K7 in a transplanted tumor model in which human CIK cells and SK-OV-3 cells were co-inoculated subcutaneously in NPG mice.
FIG. 3-5 illustrates an in vivo anti-tumor effect of a bispecific antibody AB7K7 in a transplanted tumor model in which human CIK cells and HT-29 cells were co-inoculated subcutaneously in NPG mice.
FIG. 3-6 illustrates an in vivo anti-tumor effect of a bispecific antibody AB7K7 in a transplanted tumor model in which human breast cancer cells HCC1954 were inoculated subcutaneously in NPG mice with immunology reconstituted by CD34.
FIG. 3-7 illustrates an in vivo anti-tumor effect of a bispecific antibody AB7K7 in a transplanted tumor model in which human breast cancer cells HCC1954 were inoculated in NPG mice with immunology reconstituted by PBMC.

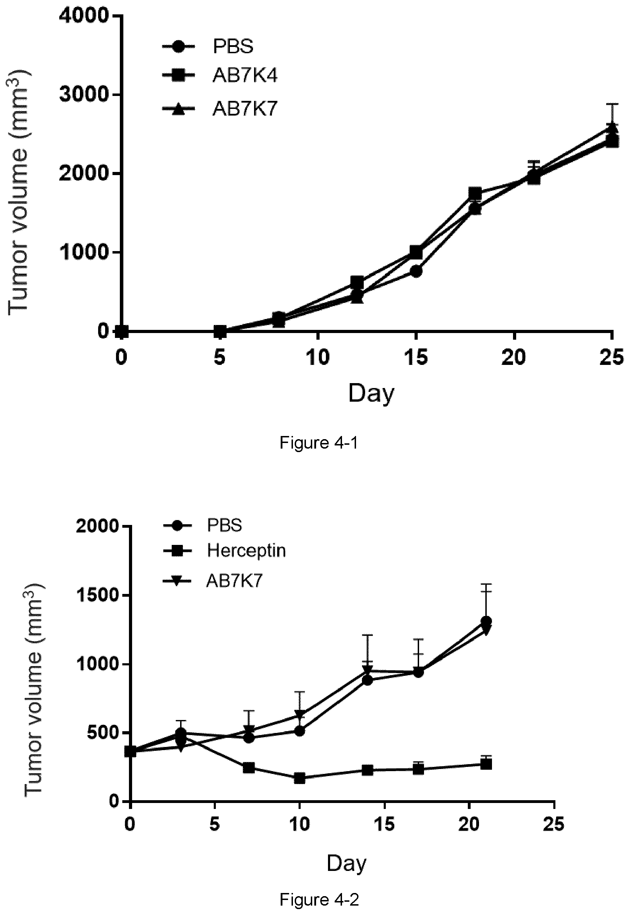

FIG. 4-1 illustrates anti-tumor effects of bispecific antibodies AB7K4 and AB7K7 in a transplanted tumor model in which human CIK cells and human Burkkit's lymphoma Raji cells were co-inoculated subcutaneously in NCG mice.
FIG. 4-2 illustrates an anti-tumor effect of a bispecific antibody AB7K7 in a transplanted tumor model in which human breast cancer cells HCC1954 were inoculated separately in NPG mice.

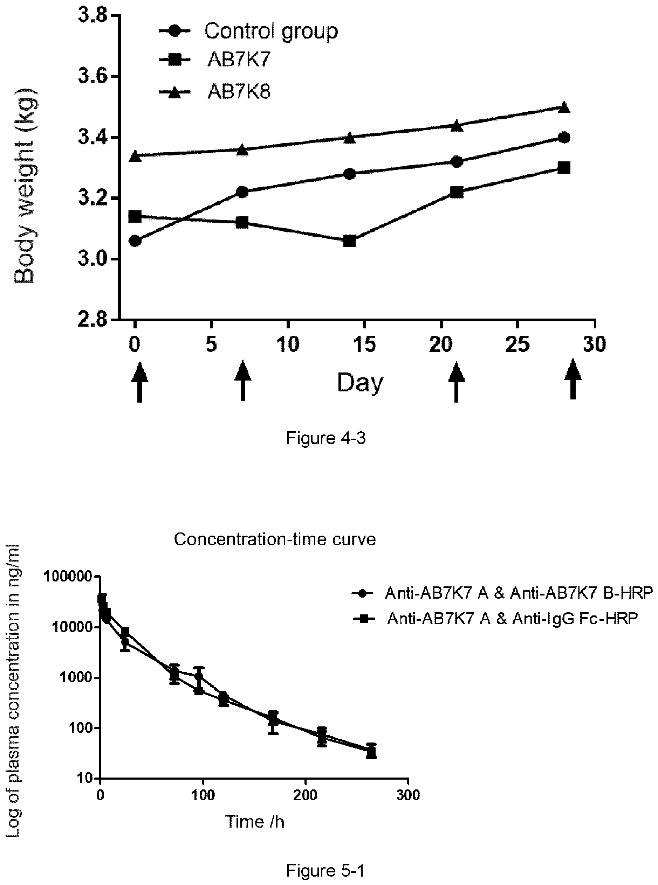

FIG. 4-3 illustrates changes in weight of normal cynomolgus monkeys administered with bispecific antibodies AB7K7 and AB7K8 multiple times.
FIG. 5-1 illustrates concentration-time curves of a bispecific antibody AB7K7 in SD rats by two ELISA methods.

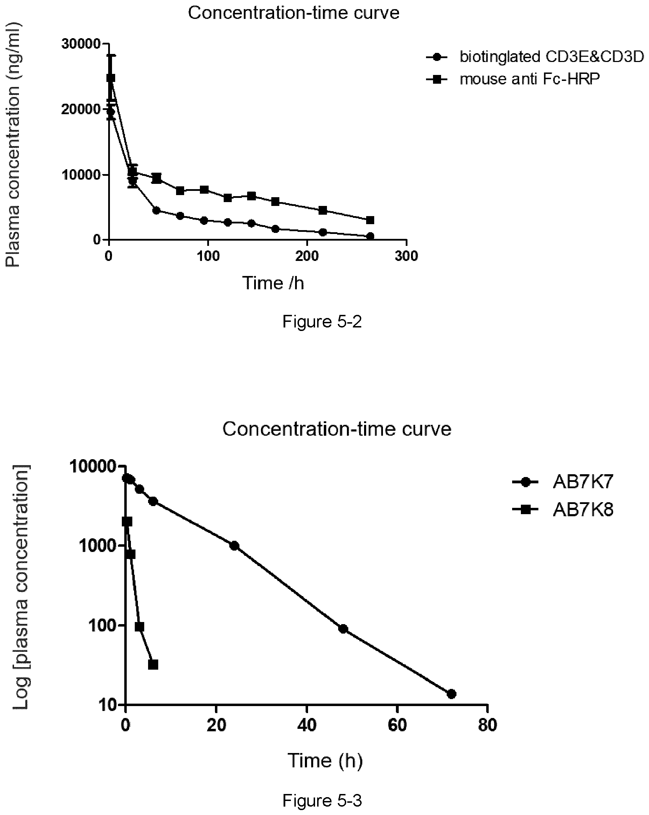

FIG. 5-2 illustrates concentration-time curves of a bispecific antibody AB7K8 in SD rats by two ELISA methods.
FIG. 5-3 illustrates concentration-time curves of bispecific antibodies AB7K7 and AB7K8 in cynomolgus monkeys.

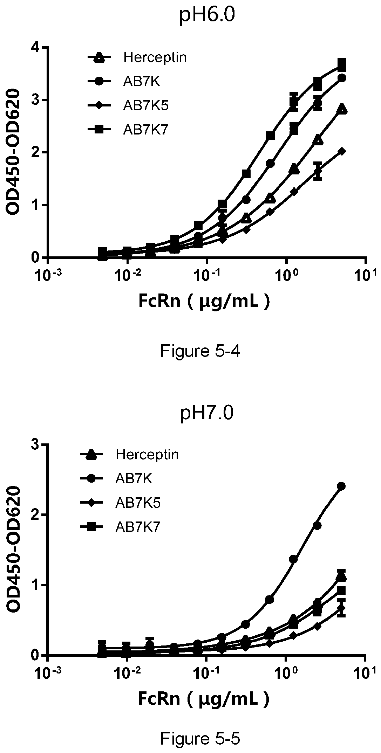

FIG. 5-4 illustrates abilities of bispecific antibodies AB7K, AB7K5 and AB7K7 to bind to FcRn, which are determined at a pH of 6.0.
FIG. 5-5 illustrates abilities of bispecific antibodies AB7K, AB7K5 and AB7K7 to bind to FcRn, which are determined at a pH of 7.0.

### DETAILED DESCRIPTION

The present disclosure is further described through examples which should not be construed as further limitations. All drawings, all reference documents, and the contents of patents and published patent applications cited in the entire application are expressly incorporated herein by reference.

### Example 1 Design and preparation of Anti-Her2xCD3 bispecific antibodies having different structures

### 1.1 Design of bispecific antibodies having different structures

In order to screen bispecific antibodies having suitable configuration, bispecific antibodies having six different configurations were designed for Her2 and CD3, among which AB7K5, AB7K6, and AB7K8 are single-chain bivalent bispecific antibodies while AB7K, AB7K4, and AB7K7 are double-chain tetravalent bispecific antibodies (see FIG. 1-1), where only AB7K8 is free of Fc fragments. Specifically, the configuration of the bispecific antibodies with the above four configurations and their composition from the N-terminus to the C-terminus as well as their amino acid sequence numbers are shown in Table 1-1. The specific structural composition properties of the six bispecific antibodies are described below:
Bispecific antibody AB7K consists of an anti-Her2 full-length antibody whose two heavy chains are each linked at the C-terminus to an anti-CD3 scFv domain by a linker peptide (L1). For the amino acid sequence of the intact antibody against Her2 contained in AB7K, reference is made to the sequence of monoclonal antibody Herceptin® (IMGT database INN 7637), wherein AB7K contains an Fc fragment from human IgG1 and has D356E/L358M mutations (EU numbering). The linker peptide L1 consists of a flexible peptide and a rigid peptide, wherein the composition of the flexible peptide is GS(GGGGS)₃ and the rigid peptide is SSSSKAPPPSLPSPSRLPGPSDTPILPQ, wherein the composition of the linker peptide L2 between VH and VL of the anti-CD3 scFv is (GGGGS)₃.
Bispecific antibody AB7K4 consists of an anti-Her2 full-length antibody whose two light chains are each linked at the C-terminus to an anti-CD3 scFv domain by a linker peptide (L1). For the amino acid sequence of the heavy chain variable region of the intact antibody against Her2 contained in AB7K4, reference is made to the available region sequence of the monoclonal antibody Herceptin®, and for the light chain amino acid sequence thereof, reference is made to the light chain amino acid sequence of the monoclonal antibody Herceptin® (IMGT database INN 7637). The AB7K4 heavy chain contains an Fc fragment from human IgG1, has multiple amino acid substitutions/replacements, which are L234A, L235A, T250Q, N297A, P331S, and M428L (EU numbering), respectively, and also has a deleted/missed K447 (EU numbering) at the C-terminus of the Fc fragment. The linker peptide L1 consists of a flexible peptide and a rigid peptide, wherein the composition of the flexible peptide is G₂(GGGGS)₃ and the rigid peptide is SSSSKAPPPS, wherein the composition of the linker peptide L2 between VH and VL of the anti-CD3 scFv is (GGGGS)₃.
Bispecific antibody AB7K5 consists of an anti-Her2 scFv, an Fc fragment, a linker peptide L2 and an anti-CD3 scFv, which are sequentially connected in series, wherein VH and VL in the anti-Her2 scFv are connected by a linker peptide L1, and VH and VL in the anti-CD3 scFv are connected by a linker peptide L3. For the amino acid sequence of the scFv against Her2 contained in AB7K5, reference is made to the available region sequence of the monoclonal antibody Herceptin®. The AB7K5 contains an Fc fragment from human IgG1 and has multiple amino acid substitutions/replacements, which are C226S, C229S, L234A, L235A, T250Q, N297A, P331S, T366R, L368H, K409T, and M428L (EU numbering), respectively. Mutations at the five sites C226S, C229S, T366R, L368H, and K409T can prevent polymerization between Fc fragments, thereby promoting the formation of a single-chain bivalent bispecific antibody. ADCC and CDC activities are removed from Fc fragments carrying the mutations L234A/L235A/P331S. The mutations T250Q/M428L can enhance the binding affinity of Fc fragments for the receptor FcRn, thereby extending the half-life. The mutation N297A avoids antibody glycosylation and loses the ability to bind FcyRs. In addition, K447 (EU numbering) at the C-terminus of the Fc fragment is deleted/missed, thereby eliminating the charge heterogeneity of the antibody. The linker peptide (L2) consists of a flexible peptide and a rigid peptide, wherein the flexible peptide is G₂(GGGGS)₃ and the rigid peptide is SSSSKAPPPS. The composition of the linker peptides L1 and L3 inside each scFv is (GGGGS)₃.
Bispecific antibody AB7K6 consists of an anti-Her2 scFv, a linker peptide L2, an anti-CD3 scFv, and an Fc fragment, which are sequentially connected in series, wherein VH and VL in the anti-Her2 scFv are connected by a linker peptide L1, and VH and VL in the anti-CD3 scFv are connected by a linker peptide L3. The AB7K6 contains an Fc fragment from human IgG1 and has multiple amino acid substitutions/replacements, which are C226S, C229S, L234A, L235A, T250Q, N297A, P331S, T366R, L368H, K409T, and M428L (EU numbering), respectively. Mutations at the five sites C226S, C229S, T366R, L368H, and K409T can prevent polymerization between Fc fragments, thereby promoting the formation of a single-chain bivalent bispecific antibody. ADCC and CDC activities are removed from Fc fragments carrying the mutations L234A/L235A/P331S. The mutations T250Q/M428L can enhance the binding affinity of Fc fragments for the receptor FcRn, thereby extending the half-life. The mutation N297A avoids antibody glycosylation and loses the ability to bind FcyRs. In addition, K447 (EU numbering) at the C-terminus of the Fc fragment is deleted/missed, thereby eliminating the charge heterogeneity of the antibody. The linker peptide (L2) consists of a flexible peptide and a rigid peptide, wherein the flexible peptide is G₂(GGGGS)₃ and the rigid peptide is SSSSKAPPPS. The composition of the linker peptides L1 and L3 inside each scFv is (GGGGS)₃.
Bispecific antibody AB7K7 consists of an anti-Her2 scFv, a linker peptide L2, an anti-CD3 scFv, and an Fc fragment, which are sequentially connected in series, wherein VH and VL in the anti-Her2 scFv are connected by a linker peptide L1, and VH and VL in the anti-CD3 scFv are connected by a linker peptide L3. For the amino acid sequence of the scFv against Her2 contained in AB7K7, reference is made to the available region sequence of the monoclonal antibody Herceptin®. The AB7K7 contains an Fc fragment from human IgG1, and has multiple amino acid substitutions/replacements, which were L234A, L235A, T250Q, N297A, P331S, and M428L (EU numbering), respectively, and also has a deleted/missed K447 (EU numbering) at the C-terminus of the Fc fragment. The linker peptide (L2) consists of a flexible peptide and a rigid peptide, wherein the flexible peptide is G₂(GGGGS)₃ and the rigid peptide is SSSSKAPPPS. The composition of the linker peptides L1 and L3 inside each scFv is (GGGGS)₃.
Bispecific antibody AB7K8 consists of an anti-Her2 scFv, a linker peptide L2, an anti-CD3 scFv, and a His-tag, which are sequentially connected in series, wherein VH and VL in the anti-Her2 scFv are connected by a linker peptide L1, and VH and VL in the anti-CD3 scFv are connected by a linker peptide L3. For the amino acid sequence of the scFv against Her2 contained in AB7K8, reference is made to the available region sequence of the monoclonal antibody Herceptin®. AB7K8 is added with a His-tag at the C-terminus of the anti-CD3 scFv to facilitate antibody purification, wherein the composition of the His tag is HHHHHHHH. The linker peptide (L2) consists of a flexible peptide and a rigid peptide, wherein the flexible peptide is G₂(GGGGS)₃ and the rigid peptide is SSSSKAPPPS. The composition of the linker peptides L1 and L3 inside each scFv is (GGGGS)₃.

VH and VL amino acid sequences of the anti-CD3 scFv contained in the above six bispecific antibodies are as shown in SEQ ID NO: 247 and SEQ ID NO: 248, respectively, wherein VH and VL are connected to each other by (GGGGS)₃. The monoclonal antibody (designated as CD3-3) specifically binds to human and cynomolgus monkey CD3 antigens and has a weak binding affinity for CD3.

**Table 1-1 Bispecific antibodies with four different structures against Her2 and CD3**

| | Code | Configuration | Composition from N-terminus to C-terminus | Amino acid sequence No. |
|---|---|---|---|---|
| Single-chain bivalent bispecific antibody | AB7K5 | scFv-mFc-scFv | [VH-L1-VL]_{Her2}-mFc-L2-[VH-L3-VL]_{CD3} | SEQ ID NO: 1 |
| | AB7K6 | scFv-scFv-mFc | [VH-LL-VL]_{Her2}-L2-[VH-L3-VL]_{CD3}-mFc | SEQ ID NO: 2 |
| | AB7K8 | scFv-scFv-His tag | [VH-L1-VL]_{Her2}-L2-[VH-L3-VL]_{CD3}-H₈ | SEQ ID NO: 3 |
| Double-chain tetravalent bispecific antibody | AB7K | IgG(H)-scFv | [VH-CH]_{Her2}-L1-[VH-L2-VL]_{CD3} | SEQ ID NO: 4 |
| | | | [VL-CL]_{Her2} | SEQ ID NO: 5 |
| | AB7K4 | IgG(L)-scFv | [VH-CH]_{Her2} | SEQ ID NO: 6 |
| | | | [VL-CL]_{Her2}-Ll -[VH-L2-VL]_{CD3} | SEQ ID NO: 7 |
| | AB7K7 | scFv-scFv-mFc | [VH-L1-VL]_{Her2}-L2-[VH-L3-VL]_{CD3}-mFc | SEQ ID NO: 8 |

| | | | | |
|---|---|---|---|---|
| Note: Ln in the table represents the linker peptides between different structural units, wherein n is numbered sequentially in the order of the linker peptides contained between different structural units from the N-terminus to the C-terminus of the bispecific antibody. | | | | |

### 1.2 Construction of an expression vector of a bispecific antibody molecule

Genes encoding the preceding five bispecific antibodies were synthesized by conventional molecular biology method, and cDNA encoding the obtained fusion genes were inserted into corresponding restriction endonuclease sites of eukaryotic expression plasmids pCMAB2M modified with PCDNA3.1. The heavy chains and light chains of AB7K and AB7K4 may be constructed into one vector or two different vectors, respectively. For example, a cDNA sequence (as shown in SEQ ID NO: 56) encoding AB7K7 was inserted into the expression plasmid shown in FIG. 1-2, which contains cytomegalovirus early promoter. The promoter is an enhancer required for the high-level expression of foreign genes in mammalian cells. The plasmid pCMAB2M also contains a selective marker so that kanamycin resistance may be present in bacteria, and G418 resistance may be present in mammalian cells. In addition, when host cells are deficient in the expression of DHFR genes, the pCMAB2M expression vector contains mouse dihydrofolate reductase (DHFR) genes so that target genes and the DHFR genes can be co-amplified in the presence of methotrexate (MTX) (see U.S. patent No. 4399216).

### 1.3 Expression of bispecific antibody molecules

The preceding constructed expression plasmids were transfected into a mammalian host cell line to express bispecific antibodies. To maintain stable and high-level expression, the preferred host cell line is a DHFR deficient CHO-cell (see U.S. Patent No. 4,818,679), and in this Example, the host cell was selected as the CHO-derived cell strain DXB11. A preferred transfection method is electroporation. Other methods, including calcium phosphate co-precipitation and lipofection may also be used. During electroporation, 50 µg of expression vector plasmids DNA were added to 5 × 10⁷ cells in a cuvette with a Gene Pulser Electroporator (Bio-Rad Laboratories, Hercules, CA) with an electric field of 300 V and capacitance of 1500 µFd. After two-day transfection, the medium was changed to a growth medium containing 0.6 mg/mL G418. Transfectants were subcloned by the limiting dilution method, and the secretion rate of each cell line was determined by ELISA. Cell strains expressing bispecific antibodies at high levels were screened.

To achieve the high-level expression of fusion proteins, DHFR genes inhibited by MTX should be used for co-amplification. The transfected fusion protein genes were co-amplified with the DHFR genes in growth media containing MTX with increasing concentrations. Subclones that were positive for DHFR expression were subjected to limiting dilution with gradually increased pressure to screen transfectants capable of growing in media with MTX of up to 6 µM. The secretion rates of the transfectants were determined and cell lines with high foreign protein expression were screened. Cell lines with a secretion rate of greater than about 5 µg/10⁶ (millions) cells/24 hours (preferably about 15 µg/10⁶ cells/24 hours) were adaptively suspended using a serum-free medium. Cell supernatants were collected and bispecific antibodies were separated and purified.

Hereinafter, the purification process, stability, *in vitro* and *in vivo* biological functions, safety, and pharmacokinetics of the bispecific antibodies of several configurations were evaluated to screen the bispecific antibody of an appropriate configuration.

### 1.4 Purification process and stability detection of bispecific antibodies

Antibodies are generally purified by a three-step purification strategy: crude purification (sample capture), intermediate purification, and fine purification. In the crude purification stage, the target antibodies are generally captured by affinity chromatography which can effectively remove a large number of impurities such as heterologous proteins, nucleic acids, endotoxins, and viruses from the sample. The intermediate purification is often carried out using hydrophobic chromatography or CHT hydroxyapatite chromatography to remove most of the remaining impurity proteins and polymers. Fine purification is mostly carried out using anion exchange chromatography or gel filtration chromatography (molecular sieve) to remove the small or trace amount of remaining impurity proteins whose nature is similar to the nature of the target antibodies and further to remove contaminants such as HCP and DNA.

In the present disclosure, the culture supernatant of bispecific antibody AB7K8 fused with His-tag can be crudely purified using a metal chelation affinity chromatography column (e.g., HisTrap FF from GE). The bispecific antibodies AB7K4, AB7K5, AB7K6, AB7K, and AB7K7 containing Fc can be crudely purified using a Protein A/G affinity chromatography column (e.g., Mabselect SURE from GE). The products obtained after the above crude purification are then subjected to the intermediate purification and the fine purification to finally obtain purified target antibodies of high purity and high quality. The preservation buffers for the above bispecific antibodies are then replaced with PBS or other suitable buffers using desalination columns (e.g., HiTrap desalting from GE).

### a) Purification of double-chain tetravalent bispecific antibody AB7K7

Specific purification steps and solutions for such bispecific antibodies of a tetravalent homodimer configuration are illustrated below by using an example of AB7K7.

The bispecific antibody AB7K7 was purified by three-step chromatography. The three-step chromatography included affinity chromatography, hydrophobic chromatography, and anion exchange chromatography. (The protein purifier used in this example was AKTA pure 25 M from GE, U.S. Reagents used in this example were purchased from Sinopharm Chemical Reagent Co., Ltd and had purity at an analytical grade).

In a first step, affinity chromatography was performed. Sample capture and concentration and the removal of partial pollutants were performed using an affinity chromatography medium MabSelect Sure from GE or other commercially available affinity media (e.g., Diamond Protein A from Bestchrom). First, chromatography columns were equilibrated with 3-5 column volumes (CVs) of an equilibration buffer (20 mM PB, 140 mM NaCl, pH 7.4) at a linear flow rate of 100-200 cm/h. The clarified fermentation broth was loaded at a linear flow rate of 100-200 cm/h with a load not higher than 20 mg/mL. After loading, the chromatography columns were equilibrated with 3-5 column volumes (CVs) of an equilibration buffer (20 mM PB, 140 mM NaCl, pH 7.4) at a linear flow rate of 100-200 cm/h to remove unbound components. The chromatography columns were rinsed with 3-5 column volumes of decontamination buffer 1 (50 mM NaAc-HAc, 1 M NaCl, pH 5.0) at a linear flow rate of 100-200 cm/h to remove partial pollutants. The chromatography columns were equilibrated with 3-5 column volumes (CVs) of decontamination buffer 2 (50 mM NaAc-HAc, pH 5.0) at a linear flow rate of 100-200 cm/h. The target product was eluted using an elution buffer (40 mM NaAc-HAc, pH 3.5) at a linear flow rate not higher than 100 cm/h and target peaks were collected.

In a second step, hydrophobic chromatography was performed. Intermediate purification was performed using Butyl HP from Bestchrom or other commercially available hydrophobic chromatography media to reduce the content of polymers. After the target proteins were polymerized, since the polymers and monomers differed in property such as charge characteristics and hydrophobicity, the polymers and the monomers could be separated on the basis of the above differences between them. First, chromatography columns were equilibrated with 3-5 column volumes (CVs) of an equilibration buffer (20 mM PB, 0.3 M (NH₄)₂SO₄, pH 7.0) at a linear flow rate of 100-200 cm/h. The target proteins separated through the affinity chromatography in the first step were subjected to conductivity adjustment to 40-50 ms/cm with the solution of 2 M (NH₄)₂SO₄ and then loaded with a load controlled to be less than 20 mg/mL. After loading, the chromatography columns were rinsed with 3-5 column volumes (CVs) of an equilibration buffer (20 mM PB, 0.3 M (NH₄)₂SO₄, pH 7.0) at a linear flow rate of 100-200 cm/h. Finally, the target proteins were eluted using 3-5 column volumes (CVs) of an elution buffer (20 mM PB, pH 7.0) with gradients of 40%, 80% and 100% at a linear flow rate not higher than 100 cm/h. Eluted fractions were collected and sent for SEC-HPLC, respectively. Target components with the percentage of monomers being greater than 90% were combined for chromatography in the next step.

In a third step, anion exchange chromatography was performed. Fine purification was performed by using Q-HP from Bestchrom or other commercially available anion exchange chromatography media (e.g., Q HP from GE, Toyopearl GigaCap Q-650 from TOSOH, DEAE Beads 6FF from Smart-Lifesciences, Generik MC-Q from Sepax Technologies, Inc, Fractogel EMD TMAE from Merck, and Q Ceramic HyperD F from Pall) to separate structural variants and further remove pollutants such as HCP and DNA. First, chromatography columns were rinsed with 3-5 column volumes (CVs) of an equilibration buffer (20 mM PB, pH 7.0) at a linear flow rate of 100-200 cm/h. The target proteins separated through the hydroxyapatite chromatography in the second step were loaded and through-flow peaks were collected. After loading, the chromatography columns were rinsed with 3-5 column volumes (CVs) of an equilibration buffer (20 mM PB, pH 7.0) at a linear flow rate of 100-200 cm/h. The through-flow components were collected and sent for the detection of protein content, SEC-HPLC and electrophoresis.

The SEC-HPLC purity results and SDS-PAGE electrophoresis results of the samples are shown in FIG. 1-3 and FIG. 1-4. The SEC-HPLC results show that the purity of the main peak of the bispecific antibody was more than 95% after three-step chromatography. The band pattern in the SDS-PAGE electrophoresis was as expected, wherein a band was shown at 180 KDa in the non-reducing electrophoresis and a clear single-chain band (90 KDa) was obtained after reduction.

### b) Purification of single-chain bivalent bispecific antibodies AB7K5 and AB7K6

The bispecific antibody AB7K5 was purified by Protein A affinity chromatography and hydroxyapatite (CHT) chromatography. After the SEC-HPLC test, it was found that the purity of bispecific antibody AB7K5 was low, its yield was not high, and there was a problem of extremely low expression yield.

For another single-chain bivalent bispecific antibody AB7K6, there also was a problem of high process development difficulty. The bispecific antibody AB7K6 was subjected to two-step purification, that is, Protein A affinity chromatography and molecular sieve chromatography Superdex 200. After the SEC-HPLC test, it was found that it was difficult to quantify the purity of bispecific antibody AB7K6, and there was a significant "shoulder peak" in the main peak; in addition, the expression yield of AB7K6 was very low and very unstable. After 24 hours of standing in a refrigerator at 4 °C, it was found that the peak shape in the SEC-HPLC result was changed from two peaks to one main peak, which attributed, presumably, to the conversion from the single-chain structure to the double-chain structure in AB7K6. From the above, it can be seen that the current process development difficulty of AB7K6 is too high to achieve process scale-up and industrialization.

In summary, AB7K7 had significant advantages over AB7K5 and AB7K6 in terms of process development and had advantages such as high yield, simple and efficient purification methods, and stable downstream processes. The physicochemical stability of AB7K7 in different buffer systems and at different storage conditions was further studied.

### c) Assay on stability of bispecific antibody AB7K7

The stability of AB7K7 proteins in a citrate buffer system (20 mM citrate, pH 5.5) and a histidine buffer system (20 mM histidine, pH 5.5) was studied, respectively. AB7K7 proteins were stored for four weeks under accelerated conditions at 25°C for the evaluation of protein stability.

AB7K7 proteins were transferred to the preceding citrate buffer system (F2) and the histidine buffer system (F3), respectively, with the concentration adjusted to 0.5 mg/mL, wherein 8% sucrose (w/v) and 0.02% PS80 (w/v) were added to both buffer systems as excipients. The above buffer systems were filtered using a 0.22 µm PES membrane needle filter, and then vialed into 2 mL penicillin bottles, respectively, 0.8 mL in each bottle. After the vialing, a stopper was immediately pressed and capped. Samples were placed in different stability chambers according to the schemes in Table 1-2. Samples were taken at each sampling point for detection and analysis, wherein the detection terms included appearance, concentration, purity (detected by SEC-HPLC), HMW%, LMW%, and turbidity (A340) of the sample.

**Table 1-2 Stability detection scheme**

| **Condition** | **T₀** | Sampling point and detection term | | |
|---|---|---|---|---|
| 40 °C | X, Y | 1 Week (W) | 2W | 4W |
| | | X, Y | X | X, Y |
| 25 °C | | 1W | 2W | 4W |
| | | X, Y | X | X, Y |
| Freeze-thaw (-70 °C/room temperature) | | 3 cycles | | |
| | | X, Y | | |

| | | | | |
|---|---|---|---|---|
| Note: X = appearance, concentration, SEC-HPLC, SDS-PAGE (reducing & non-reducing); Y = turbidity (A340) | | | | |

The appearance, concentration, turbidity and SEC-HPLC detection results of two preparations stored for 0-4 weeks at 25°C are shown in Table 1-3 and Table 1-4, and SDS-PAGE (reducing/non-reducing) results thereof are shown in FIG. 1-5. There was no significant change in the appearance and concentration of the two preparations. In the SEC-HPLC results, the SEC results of the F2 and F3 preparations did not show any significant change. The purity after 4 weeks was 97.9% and 98.2%, respectively. SDS-PAGE (reducing/non-reducing) results were generally consistent with the trend of LMW% results, and F2 and F3 slightly changed.

**Table 1-3 Appearance, concentration, and turbidity results in the acceleration test at 25 °C**

| | Appearance | | | | Concentration | | | | Turbidity A340 | | |
|---|---|---|---|---|---|---|---|---|---|---|---|
| | T0 | 1W | 2W | 4W | T0 | 1W | 2W | 4W | T0* | 1W | 4W |
| F2 | Colorless clear liquid without visible foreign matter | | | | 0.46 | 0.46 | 0.45 | 0.46 | 0.003 | 0.004 | 0.002 |
| F3 | | | | | 0.47 | 0.46 | 0.45 | 0.47 | 0.004 | 0.003 | 0.005 |

| | | | | | | | | | | | |
|---|---|---|---|---|---|---|---|---|---|---|---|
| *T0 turbidity: the sample to be detected was a sample subjected to 1 cycle of freeze-thaw. | | | | | | | | | | | |

**Table 1-4 SEC-HPLC results in the acceleration test at 25 °C**

| | SEC-Purity% | | | | SEC-HMW% | | | | SEC-LMW% | | | |
|---|---|---|---|---|---|---|---|---|---|---|---|---|
| | T0 | 1W | 2W | 4W | T0 | 1W | 2W | 4W | T0 | 1W | 2W | 4W |
| F2 | 97.5 | 98.3 | 98.5 | 97.9 | 2.5 | 1.5 | 1.5 | 1.5 | 0 | 0.3 | 0.0 | 0.6 |
| F3 | 97.7 | 98.8 | 98.7 | 98.2 | 2.3 | 1.2 | 1.3 | 1.2 | 0 | 0.0 | 0.0 | 0.6 |

To know the unfolding temperature of AB7K7 proteins in the two buffer systems, the Tm (unfolding temperature) and Tmonset (the temperature at which the protein begins to unfold) in the two preparations were measured by DSF and the results are shown in Table 1-5. Both preparations had low Tmonset values, and F2 and F3 had Tmonset values less than 45 °C.

**Table 1-5 DSF results**

| | Tmonset (°C) | Tm1 (°C) | Tm2 (°C) |
|---|---|---|---|
| F2 | 42.0 | 46.0 | 60.5 |
| F3 | 41.0 | 45.0 | 58.0 |

The stability of AB7K7 proteins in the above two buffer systems during freeze-thaw (-70 °C/room temperature, 3 cycles of freeze-thaw) was studied by performing 3 cycles of freeze-thaw. The preparation and detection solution of the sample were the same as those described above.

The appearance, concentration, turbidity and SEC-HPLC detection results of samples are shown in Table 1-6, and SDS-PAGE (reducing/non-reducing) results thereof are shown in FIG. 1-6. In the SDS-PAGE (non-reducing) results, there were no significant changes in the results of each detection item of both F2 and F3 preparations subjected to three cycles of freeze-thaw.

**Table 1-6 Appearance, concentration, turbidity, and SEC-HPLC results in the freeze-thaw test**

| | Appearance | | Concentration | | Turbidity A340 | | SEC-Purity% | | SEC-HMW% | | SEC-LMW% | |
|---|---|---|---|---|---|---|---|---|---|---|---|---|
| | T0 | FT-3C | T0 | FT-3C | T0 | FT-3C | T0 | FT-3C | T0 | FT-3C | T0 | FT-3C |
| F2 | Colorle ss clear liquid without visible foreign matter | Colorless clear liquid without visible foreign matter | 0.46 | 0.46 | 0.003 | 0.005 | 97.5 | 98.4 | 2.5 | 1.6 | 0 | 0.0 |
| F3 | | | 0.47 | 0.48 | 0.004 | 0.005 | 97.7 | 98.5 | 2.3 | 1.4 | 0 | 0.2 |

### Example 2 Evaluation of in vitro biological functions of Anti-Her2xCD3 bispecific antibodies

### 2.1 Detection of binding activities of bispecific antibodies to effector cells and target cells (FACS)

### a) Detection of binding activities of bispecific antibodies to Her2-positive tumor cells BT-474 by flow cytometry

Tumor cells BT-474 that were positive for Her2 expression (from the cell bank of Chinese Academy of Sciences, Shanghai) were cultured, then digested with 0.25% trypsin, and centrifuged to collect cells. The collected cells were resuspended with 1% PBSB, placed in 96-well plates after the cell density was adjusted to (2 × 10⁶) cells/ml, 100 µl (2 × 10⁵ cells) per well, and blocked for 0.5 hours at 4 °C. The blocked cells were centrifuged to discard the supernatant, and a series of diluted bispecific antibodies were added to the cells. The cells were incubated for 1 hour at 4 °C, then centrifuged to discard the supernatant, and washed three times using a PBS solution with 1% BSA (PBSB). Diluted AF488-labeled goat anti-human IgG antibodies or murine anti-6×His IgG antibodies were added to the cells, and the cells were incubated for 1 hour at 4 °C in the dark. The obtained cells were centrifuged to discard the supernatant, and washed twice with 1% PBSB, and cells in each well were resuspended with 100 µl of 1% paraformaldehyde (PF). The signal intensity was detected by flow cytometry. The analysis was performed with the average fluorescence intensity as the Y-axis and the antibody concentration as the X-axis through software GraphPad to calculate the EC₅₀ value for the binding of bispecific antibodies to tumor cells BT-474.

The results show that bispecific antibodies with different structures had good binding activity to tumor cells overexpressing Her2. FIG. 2-1 to FIG. 2-5 show binding curves of bispecific antibodies with different structures to tumor cells BT-474. As shown in Table 2-1, the ECso for the binding of AB7K to tumor cells and the EC₅₀ for the binding of AB7K4 to tumor cells both were around 5 nM, the EC₅₀ for the binding of AB7K7 to tumor cells was close to 50 nM, the EC₅₀ for the binding of AB7K5 to tumor cells and the EC₅₀ for the binding of AB7K8 to tumor cells both were greater than 100 nM, and the EC₅₀ for the binding of AB7K6 to tumor cells was greater than 200 nM.

**Table 2-1 Detection of abilities of Anti-Her2xCD3 bispecific antibodies to bind to tumor cells BT474**

| | AB7K | AB7K4 | AB7K5 | AB7K6 | AB7K7 | AB7K8 |
|---|---|---|---|---|---|---|
| EC₅₀ (nM) | 5.009 | 4.388 | 125.0 | 239.9 | 51.98 | 125.3 |

### b) Detection of binding activities of bispecific antibodies to human T cells by FACS

PBMCs were prepared from fresh human blood by density gradient centrifugation. The prepared PBMCs were resuspended in a 1640 medium containing 10% heat-inactivated FBS, added with 2 µg/ml OKT3 for activation for 24 h, then added with 250 IU/ml IL-2 for amplification for 7 days, to prepare cytokine-induced killer (CIK) cells which were detected by flow cytometry to be positive for CD3 expression on the surface. The to-be-detected samples were prepared and detected in the same manner as in a) of Example 2.1. Cells resuspended with 1% PF were detected on a machine and, with the average fluorescence intensity, analyzed by software OriginPro 8 to calculate the EC₅₀ value for the binding of each bispecific antibody to human CIK cells.

The results show that there were great differences among the binding of each bispecific antibody to CIK cells (FIG. 2-6 to FIG. 2-10). As shown in Table 2-2, the EC₅₀ of AB7K was about 20 nM, which was roughly equal to the EC₅₀ of AB7K4, the EC₅₀ of AB7K7 was more than 6 times higher than the EC₅₀ of AB7K, and the EC₅₀ of AB7K5, AB7K6 and AB7K8 was more than 10 times higher than the EC₅₀ of AB7K.

**Table 2-2 Detection of abilities of Anti-Her2xCD3 bispecific antibodies to bind to effector cells CIK**

| | AB7K | AB7K4 | AB7K5 | AB7K6 | AB7K7 | AB7K8 |
|---|---|---|---|---|---|---|
| EC₅₀(nM) | 20.51 | 19.44 | 375.2 | 241.7 | 132.3 | 504.1 |

### c) Detection of cross-reactivity of bispecific antibodies with cynomolgus monkey CIK cell membrane CD3 by FACS

PBMCs were prepared from fresh cynomolgus monkey blood by density gradient centrifugation. The prepared PBMCs were resuspended in a 1640 medium containing 10% heat-inactivated FBS, added with 2 µg/ml OKT3 for activation for 24 h, then added with 250 IU/ml IL-2 for amplification for 7 days, to prepare cynomolgus monkey CIK cells for use. Human CIK cells and cynomolgus monkey CIK cells were collected by centrifugation, followed by the same test procedure as in the above examples. Cells resuspended with 1% paraformaldehyde solution were detected on a machine and, with the average fluorescence intensity, analyzed by software OriginPro 8 to calculate the EC₅₀ values for the binding of bispecific antibodies to human CIK cells and the EC₅₀ values for the binding of bispecific antibodies to cynomolgus monkey CIK cells.

As shown in FIG. 2-11, the bispecific antibody AB7K bound well to cynomolgus monkey T cells, the ability of AB7K to bind to cynomolgus monkey T cells was roughly equal to the ability of AB7K to bind to human T cells, and the EC₅₀ for the binding of AB7K to cynomolgus monkey T cells was approximately 26 nM as detected by flow cytometry. Bispecific antibodies AB7K4, AB7K5, AB7K6, AB7K7, and AB7K8 bound specifically to cynomolgus monkey T cells, as did AB7K.

### 2.2 Detection of abilities of bispecific antibodies to bind to antigens

The binding of bispecific antibodies to soluble CD3 and Her2 was detected by double antigen sandwich ELISA.

Her2 proteins (SinoBiological, Beijing, Cat. No. 10004-H08H4) were diluted with PBS to a concentration of 0.1 µg/ml and added to 96-well plates, 100 µl per well. The plates were coated at 4 °C overnight. The plates were then blocked with 1% skimmed milk powder for 1 hour at room temperature. Each bispecific antibody was diluted simultaneously with a 4-fold gradient for a total of 11 concentration gradients. The 96-well plates were then washed with PBST, and then the diluted bispecific antibodies were added. Control wells without antibodies were set. Incubated for 1 hour at room temperature. Unbound bispecific antibodies were washed away with PBST. Biotinylated CD3E and CD3D (ACRO Biosystem, Cat. No. CDD-H82W1) were mixed at 50 ng/ml with streptavdin HRP (BD, Cat. No. 554066), added in the 96-well plates, 100 µl per well, and incubated for 1 hour at room temperature. 96-well plates were washed with PBST, and TMB was added to the plates, 100 µl per wellss. Color development was performed at room temperature for 15 minutes, and then 0.2 M H₂SO₄ was added to stop the color development reaction. The light absorbance values at A450-620 nm were measured by a microplate reader. Analysis was performed by software OriginPro 8, and the EC₅₀ values for the binding of bispecific antibodies to two antigens were calculated.

The results show that each bispecific antibody bound specifically to both CD3 and Her2 molecules and exhibited good dose-dependence as the concentration of the antibodies changed (FIG. 2-12). The abilities of several bispecific antibodies to bind to soluble CD3 and Her2 are shown in Table 2-3, with EC₅₀ values ranging from 0.03 nM to 3.8 nM which differ by two orders of magnitude. AB7K had the best binding activity, binding activities of AB7K4 and AB7K7 differed by one order of magnitude, and AB7K5 and AB7K8 had the weakest binding activity.

**Table 2-3 Detection of abilities of Anti-Her2xCD3 bispecific antibodies to bind to CD3 and Her2 molecules**

| | AB7K | AB7K4 | AB7K5 | AB7K7 | AB7K8 |
|---|---|---|---|---|---|
| EC₅₀ (nM) | 0.03128 | 0.1518 | 1.004 | 0.1398 | 3.815 |

### 2.3 Evalution of abilities of bispecific antibodies to activate T cells through reporter gene cell strains

Jurkat T cells containing NFAT RE reporter genes (BPS Bioscience, Cat. No. 60621) can overexpress luciferase in the presence of bispecific antibodies and target cells, and the degree of activation of the Jurkat T cells can be quantified by detecting the activity of the luciferase. A four-parameter curve was fitted using the concentration of bispecific antibodies as the X-axis and the fluorescein signal as the Y-axis.

The test results from FIG. 2-13 show that the monoclonal antibody Herceptin targeting Her2 cannot activate Jurkat T cells. T cells can be activated only in the presence of both antibodies. The ability of each antibody to activate Jurkat T cells is shown in Table 2-4. AB7K4 had the strongest ability to activate T cells, AB7K8 had the weakest ability to activate T cells, and their EC₅₀ values differed by one order of magnitude.

**Table 2-4 Detection of abilities of Anti-Her2xCD3 bispecific antibodies to a reporter gene cell strain that are Jurkat T cells**

| | AB7K | AB7K4 | AB7K5 | AB7K7 | AB7K8 | Herceptin |
|---|---|---|---|---|---|---|
| EC50 (nM) | 0.02263 | 0.01338 | 0.05357 | 0.08952 | 0.1575 | 0.009907 |

### 2.4 Abilities of bispecific antibodies to mediate T cells to kill tumor cells

Normally cultured tumor cell lines, including SK-BR-3, MCF-7, HCC1937, NCI-N87, HCC1954 cells (all purchased from the cell bank of Chinese Academy of Sciences, Shanghai), as target cells, were digested with 0.25% trypsin to prepare single-cell suspensions, added to 96-well cell culture plates after the cell density was adjusted to 2 × 10⁵ cells/ml, 100 µl per well, and cultured overnight. The antibodies were diluted according to the test design, and added to the cells, 50 µl per well, while wells without the addition of antibodies were supplemented with the same volume of the medium. Effector cells (human PBMCs or expanded CIK cells) whose number was five times larger than the number of target cells, were then added, 100 µl per well. Control wells were set, and wells without the addition of effector cells were supplemented with the same volume of the medium. After incubation for 48 hours, the supernatant was discarded from the 96-well plates. The cells were then washed three times with PBS, and a complete medium containing 10% CCK-8 was added, 100 µl per well, and the cells were incubated for 4 hours at 37 °C. The light absorbance values at A450-620 nm were measured by a microplate reader. Analysis was performed by software OriginPro 8, and the ability of each bispecific antibody to mediate the killing of tumor cells and the ability of the same target monoclonal antibody Herceptin to mediate the killing of tumor cells were calculated and compared.

The ECso values of each bispecific antibody to mediate effector cells to kill tumor cells are shown in Table 2-5. The results show that each bispecific antibody exhibited a very significant killing effect on tumor cells (e.g., SK-BR-3, NCI-N87, and HCC1954) with high expression of Her2 in a dose-dependent manner. Each bispecific antibody, in particular AB7K7, also exhibited a good killing effect on breast cancer cells MCF-7 with low expression of Her2. Each bispecific antibody also had a good killing effect on the Herceptin-resistant cell strain HCC1954 while each bispecific antibody exhibited the killing effect on the cell strain HCC1937 that was negative for Her2 expression (with little expression) only at two highest concentrations.

**Table 2-5 EC₅₀ values of bispecific antibodies to mediate PBMCs to kill different tumor cells**

| EC50 (nM) | AB7K7 | AB7K8 | AB7K5 | Herceptin |
|---|---|---|---|---|
| SK-BR-3 | ∼ 0.001 | ∼ 0.002 | ∼ 0.001 | - |
| | ∼ 0.001 | 0.011 | - | 0.067 |
| MCF-7 | ∼ 0.005 | 0.079 | 0.055 | - |
| HCC1937 | 0.659 | ∼ 2.269 | 1.223 | - |
| | 0.579 | 4.011 | - | >6.667 |
| NCI-N87 | 0.015 | 0.034 | - | 0.129 |
| HCC1954 | 0.002 | 0.018 | - | 0.050 |

| | | | | |
|---|---|---|---|---|
| Note: ∼ means approximately equal to, and - means that no detection is performed. | | | | |

### 2.5 Evaluation of the effect of GS-CTP linker peptide on the ability of anti-CD3 scFv to bind to CD3 molecules by computer techniques

The anti-CD3 scFv VH containing the GS-CTP linker peptide was structurally modeled using computer software and the spatial conformation of molecular docking of anti-CD3 scFv and its antigen CD3 epsilon chain was simulated and predicted.

The sequence of the GS-CTP linker peptide between anti-Her2 scFv and anti-CD3 scFv in the bispecific antibody AB7K7 is (GGGGGGSGGGGSGGGGSSSSSKAPPPS), wherein the first half of the sequence is a GS-flexible peptide (GGGGGGSGGGGSGGGGS), and the second half is CTP-rigid peptide (SSSSKAPPPS). The rigid CTP portion (SSSSKAPPPS) is connected to the N-terminus of the anti-CD3 scFv VH. Through three-dimensional structural modeling using software phyre2, it is found that the CTP peptide fragment structurally overlays on the CDR1 region of VH of the anti-CD3 scFv (FIG. 2-14), which may hinder or disrupt the binding of the CD3 antibody to its antigen. The VH of the anti-CD3 scFv connected to the GS linker peptide (containing only the GS flexible peptide with the removal of CTP) was subjected to three-dimensional structural modeling using software phyre2, and then it is found that the GS linker peptide is far from the CDR region (FIG. 2-15) and does not affect antigen-antibody binding. Even if the GS linker peptide is close to the CDR region, the GS linker peptide can freely move away from the antigen-antibody binding region due to its own flexibility and thus does not affect antigen-antibody binding.

Further, the molecular docking between the anti-CD3 scFv and its antigen CD3 epsilon chain was simulated by software Discovery Studio. Since the structure of the double-chain anti-CD3 FV is highly similar to the structure of the anti-CD3 scFv, the structure simulation was performed using the double-chain anti-CD3 FV instead of the anti-CD3 scFv. The simulation results show that the antigen CD3 epsilon chain binds to CDR2 and CDR3 of VH of the anti-CD3 Fv while does not bind to the CDR1 region (FIG. 2-16), which indicates that the CTP overlaying the VH CDR1 region of the anti-CD3 Fv does not interfere with the binding of the anti-CD3 scFv to the antigen. However, given that the CD3 molecule is a complex including one CD3 gamma chain, one CD3 delta chain, and two CD3 epsilon chains, the CD3 molecule, together with the TCR and Zeta chains, constitutes a T-cell receptor complex. Although the CTP peptide fragment covering the VH CDR1 of the anti-CD3 scFv does not directly interfere with the binding of the anti-CD3 scFv to its antigen CD3 epsilon chain, the CTP peptide fragment may indirectly affect the binding of the anti-CD3 scFv to its antigen CD3 epsilon chain by making spatial structural contact with a certain constituent protein of the T-cell receptor complex.

Due to the presence of CTP covering the VH CDR1 region of the anti-CD3 scFv, the binding affinity of the anti-CD3 scFv for its antigen is greatly diminished so that there is no substantial release of cytokines caused by the overactivation of T cells, thereby avoiding some unnecessary T cell-mediated non-specific killing.

### Example 3 Pharmacodynamics study of Anti-Her2xCD3 bispecific antibodies in a mouse transplanted tumor model

### 3.1 NCG mouse model of transplanted tumor constructed by subcutaneously co-inoculating human CIK cells and human breast cancer cells HCC1954

Her2-positive human breast cancer cells HCC1954 were selected to study the effect of bispecific antibodies in inhibiting tumor growth *in vivo* in an NCG mouse model of transplanted tumor constructed by subcutaneously co-inoculating human CIK cells and HCC1954 cells.

The peripheral blood of a normal human was subjected to density gradient centrifugation (Lymphoprep™, Lymphocytes Separation Medium, STEMCELL) to separate human PBMCs. Then the human PBMCs were resuspended in RPMI-1640 culture medium added with 10% inactivated FBS, and added with OKT3 at a final concentration of 1 µg/mL and human IL-2 at 250 IU/mL. After three days of culture, the human PBMCs were centrifuged at 300 g for 5 minutes, and the medium was changed. The cells were cultured in RPMI-1640 added with 10% inactivated FBS and added with human IL-2 at 250 IU/mL. After that, a fresh medium was then added every 2 days and CIK cells were collected on the tenth day of culture. Female NCG mice at the age of seven to eight weeks (purchased from Jiangsu GemPharmatech Co. Ltd Company) were selected and HCC1954 cells in the logarithmic growth stage were collected. 5 × 10⁶ HCC1954 cells and 5 × 10⁵ CIK cells were mixed and inoculated subcutaneously on the right back of each NCG mouse. One hour later, the mice were randomly divided into seven groups with five mice in each group according to their weights and intraperitoneally administered with corresponding drugs. All positive control groups and PBS control group were administered twice a week for a total of 3 doses, wherein the positive control groups were administered with Herceptin (from Roche) at doses of 1 mg/kg and 3 mg/kg, respectively, and the PBS control group was administered with a PBS solution of the same volume as Herceptin. The treated groups were administered with bispecific antibodies AB7K4 and AB7K7 every day at doses of 0.1 mg/kg and 1 mg/kg, respectively, for a total of 10 doses. The day of administration was recorded as Day 0. The maximum diameter (D) and the minimum diameter (d) of the tumor were measured weekly with an electronic vernier caliper. The volume of the tumor was calculated using the following formula: volume (mm³) = [D × d²]/2. The tumor growth inhibition rate (TGI) was calculated for each treated group using the following formula: TGI (%) = (1 - volume of the treated group/volume of the control group) × 100%.

As shown in FIG. 3-1, on Day 33 of administration, the average tumor volume of the PBS control group was 1494.61 ± 500.28 mm³; the average tumor volume of the treated group administrated with Herceptin at a dose of 1 mg/kg was 1327.29 ± 376.65 mm³; the average tumor volume of the treated group administrated with Herceptin at a dose of 3 mg/kg was 510.49 ± 106.07 mm³, and the TGI was 65.84%, which was not significantly different from that of the control group. The average tumor volumes of treated groups administrated with AB7K4 at doses of 0.1 mg/kg and 1 mg/kg were 304.10 ± 108.50 mm³ and 79.70 ± 58.14 mm³, respectively, and TGIs thereof were 79.65% and 94.67%, respectively, which were significantly different from that of the PBS control group (*P* < *0.05*). The average tumor volumes of treated groups administrated with AB7K7 at doses of 0.1 mg/kg and 1 mg/kg were 385.82 ± 95.41 mm³ and 209.98 ± 51.74 mm³, respectively, and TGIs thereof were 74.19% and 85.95%, respectively, which were significantly different from that of the PBS control group (*P* < *0.05*). In summary, the results show that the bispecific antibodies AB7K4 and AB7K7 at different doses could inhibit the growth of tumor cells by activating human immune cells in animals and exhibited great anti-tumor effects; and at the same dose of 1 mg/kg, the anti-tumor effect of the bispecific antibody was better than that of the monoclonal antibody Herceptin.

### 3.2 NPG mouse model of transplanted tumor constructed by subcutaneously co-inoculating human CIK cells and human breast cancer cells HCC1954

Her2-positive human breast cancer cells HCC1954 were selected to study the inhibiting effect of bispecific antibodies on tumor growth *in vivo* in an NPG mouse model of transplanted tumor constructed by subcutaneously co-inoculating human CIK cells and human breast cancer cells HCC1954.

CIK cells were prepared in the method as described in Example 3.1. Female NPG mice at the age of seven to eight weeks (purchased from Beijing Vitalstar Biotechnology Co., Ltd.) were selected and HCC1954 cells in the logarithmic growth stage were collected. 5 × 10⁶ HCC1954 cells and 5 × 10⁵ CIK cells were mixed and inoculated subcutaneously on the right back of each NPG mouse. After 6 days of tumor growth, the mice were randomly divided into three groups with six mice in each group according to the tumor volumes and weights and intraperitoneally administered with corresponding drugs. Specifically, AB7K7 treated groups were administered twice a week at doses of 0.1 mg/kg and 1 mg/kg, respectively, and the control group was administered with a PBS solution of the same volume as AB7K7. The day of administration was recorded as Day 0. The maximum diameter (D) and the minimum diameter (d) of the tumor were measured weekly. The volume (mm³) of the tumor of each group and the tumor growth inhibition rate (TGI) (%) of each treated group were calculated using the formulas as shown in Example 3.1.

As shown in FIG. 3-2, on Day 21 of administration, the average tumor volume of the PBS control group was 821.73 ± 201.82 mm³; the average tumor volume of the treated group administrated with AB7K7 at a dose of 0.1 mg/kg was 435.60 ± 51.04 mm³, and the TGI was 50.83%, which was not significantly different from that of the control group; the average tumor volume of the treated group administrated with AB7K7 at a dose of 1 mg/kg was 40.98 ± 12.64 mm³, and the TGI was 95.37%, which was significantly different from that of the control group (*P* < *0.01*). The above results show that the administration of the bispecific antibody AB7K7 had a good therapeutic effect even when tumors had grown to a certain volume, wherein 50% tumor inhibition effect was achieved at the low dose of 0.1 mg/kg, and there was complete tumor regression in 4 of 6 mice in the treated group at the dose of 1 mg/kg and the tumor volumes in the other 2 mice were both less than 100 mm³, which was smaller than the tumor volume at the time of grouping (the average tumor volume of this group at the time of grouping was 161.37 ± 18.98 mm³). Therefore, the bispecific antibody AB7K7 had a great therapeutic effect on tumors.

In addition, the inhibiting effect of bispecific antibodies AB7K7 and AB7K8 on tumor growth in the above-described transplanted tumor model at two administration frequencies were also studied. CIK cells were prepared in the method as described above. Female NPG mice at the age of seven to eight weeks were selected, and 5 × 10⁶ HCC1954 cells and 5 × 10⁵ CIK cells were mixed and inoculated subcutaneously on the right back of each NPG mouse. One hour later, the mice were randomly divided into six groups with six mice in each group according to their weights and intraperitoneally administered with corresponding drugs. Specifically, the control group and the Herceptin treated group were administered twice a week, wherein Herceptin was administrated at a dose of 3 mg/kg and the control group was administered with a PBS solution of the same volume as Herceptin. The bispecific antibody AB7K7 was administered at a dose of 1 mg/kg and AB7K8 was administered at a dose of 0.7 mg/kg. Two administration frequencies were set for each of the two bispecific antibodies, wherein the QD group was administered once a day for 10 consecutive days and the BIW group was administered twice a week. The day of administration was recorded as Day 0. The maximum diameter (D) and the minimum diameter (d) of the tumor were measured weekly. The tumor volume (mm³) of each group and the tumor growth inhibition rate (TGI) (%) of each treated group were calculated using the formulas as shown above.

As shown in FIG. 3-3, on Day 25 of administration, the average tumor volume of the PBS control group was 1588.12 ± 120.46 mm³; the average tumor volume of the treated group administrated with Herceptin at a dose of 3 mg/kg was 361.72 ± 134.70 mm³; the average tumor volumes of the QD group and the BIW group administrated with AB7K7 were 260.18 ± 45.96 mm³ and 239.39 ± 40.62 mm³, respectively, and TGIs were 83.62% and 84.93%, respectively, which were significantly different from that of the PBS control group (P < *0.01*); the average tumor volumes of the QD group and the BIW group administrated with AB7K8 were 284.98± 26.62 mm³ and 647.14 ± 118.49 mm³, respectively, and TGIs were 82.06% and 59.25 %, respectively, which were significantly different from that of the PBS control group (*P* < *0.01*). As can be seen from the above results, the anti-tumor effect of the bispecific antibody AB7K7 was superior to that of the Herceptin in both the QD group and the BIW group; at equimolar doses, AB7K8 and AB7K7 in the QD group exhibited basically equal tumor inhibiting effects, while the anti-tumor effect of AB7K7 in the BIW group was significantly superior than that of AB7K8, presumably due to the fact that AB7K8 is a bispecific antibody of BiTE configuration with no Fc domain, and therefore AB7K7 has a longer half-life than AB7K8, from which it is anticipated that the clinical administration frequency of AB7K7 is reduced and AB7K7 has a better therapeutic effect.

### 3.3 NPG mouse model of transplanted tumor constructed by subcutaneously co-inoculating human CIK cells and human ovarian cancer cells SK-OV-3

Her2-positive human ovarian cancer cells SK-OV-3 were selected to study the inhibiting effect of bispecific antibodies on tumor growth *in vivo* in an NPG mouse model of transplanted tumor constructed by subcutaneously co-inoculating human CIK cells and SK-OV-3 cells.

The peripheral blood of a normal human was subjected to density gradient centrifugation to separate human PBMCs. Then the human PBMCs were resuspended in McCoy's 5A culture medium added with 10% inactivated FBS, and added with OKT3 at a final concentration of 1 µg/mL and human IL-2 at 250 IU/mL. After three days of culture, the human PBMCs were centrifuged at 300 g for 5 minutes, and the supernatant was discarded. The cells were resuspended in RPMI-1640 added with 10% inactivated FBS and added with 250 IU/mL of human IL-2. After that, a fresh medium was then added every 2 days and CIK cells were collected on the tenth day of culture. Female NPG mice at the age of seven to eight weeks were selected and SK-OV-3 cells (purchased from the cell bank of Chinese Academy of Sciences, Shanghai) in the logarithmic growth stage were collected. 3 × 10⁶ SK-OV-3 cells and 3 × 10⁵ CIK cells were mixed and inoculated subcutaneously on the right back of each NPG mouse. One hour after inoculation, the mice were randomly divided into seven groups with six mice in each group according to their weights and intraperitoneally administered with corresponding drugs. Herceptin and AB7K7 treated groups were administered twice a week at doses of 1 mg/kg, 0.2 mg/kg, and 0.04 mg/kg, respectively, and the control group was administered with a PBS solution of the same volume. The day of administration was recorded as Day 0. The maximum diameter (D) and the minimum diameter (d) of the tumor were measured weekly. The volume (mm³) of the tumor of each group and the tumor growth inhibition rate (TGI) (%) of each treated group were calculated using the formulas as shown in Example 3.1.

As shown in FIG. 3-4, on Day 21 of administration, the average tumor volume of the PBS control group was 834.09 ± 45.64 mm³; the average tumor volumes of the treated groups administrated with Herceptin at doses of 1 mg/kg, 0.2 mg/kg, and 0.04 mg/kg were 644.84 ± 58.22 mm³, 884.95 ± 38.63 mm³, and 815.79 ± 78.39 mm³, respectively; and the tumors in all AB7K7 treated groups were completely regressed. The above results show that in the ovarian cancer SK-OV-3 model, AB7K7 enabled the tumor to completely regress even at a very low dose of 0.04 mg/kg, exhibiting an excellent anti-tumor effect.

### 3.4 NPG mouse model of transplanted tumor constructed by subcutaneously co-inoculating human CIK cells and human colon cancer cells HT-29

Her2-positive human colon cancer cells HT-29 were selected to study the inhibiting effect of bispecific antibodies on tumor growth *in vivo* in an NPG mouse model of transplanted tumor constructed by subcutaneously co-inoculating human CIK cells and HT-29 cells.

CIK cells were prepared in the method as described in Example 3.1. Female NPG mice at the age of seven to eight weeks were selected and HT-29 cells (purchased from the cell bank of Chinese Academy of Sciences, Shanghai) in the logarithmic growth stage were collected. 3 × 10⁶ HT-29 cells and 3 × 10⁶ CIK cells were mixed and inoculated subcutaneously on the right back of each NPG mouse. One hour after inoculation, the mice were randomly divided into five groups with six mice in each group according to their weights and intraperitoneally administered with corresponding drugs. Specifically, Herceptin was administered at a dose of 3 mg/kg, and AB7K7 was administered at doses of 3 mg/kg, 1 mg/kg, and 0.3 mg/kg, respectively. All treated groups were administered twice a week. The control group was administered with a PBS solution of the same volume. The day of administration was recorded as Day 0. The maximum diameter (D) and the minimum diameter (d) of the tumor were measured weekly. The volume (mm³) of the tumor of each group and the tumor growth inhibition rate (TGI) (%) of each treated group were calculated using the formulas as shown in Example 3.1.

As shown in FIG. 3-5, on Day 21 of administration, the average tumor volume of the PBS control group was 1880.52 ± 338.26 mm³; the average tumor volume of the treated group administrated with Herceptin at a dose of 3 mg/kg was 1461.36 ± 177.94 mm³; the average tumor volumes of the treated groups administrated with AB7K7 at doses of 3 mg/kg, 1 mg/kg, and 0.3 mg/kg were 13.94 ± 7.06 mm³, 26.31 ± 10.75 mm³, and 10.47 ± 6.71 mm³, wherein tumors in four mice in the treated group at a dose of 0.3 mg/kg were completely regressed, tumors in three mice in the treated group at a dose of 1 mg/kg were completely regressed, and tumors in four mice in the treated group at a dose of 3 mg/kg were completely regressed. The above results show that in the colon cancer HT-29 model, Herceptin had few pharmacological effect on this tumor model, whereas AB7K7 exhibited complete tumor regression in mice at all three doses and exhibited excellent anti-tumor effect even at very low doses.

### 3.5 CD34 immune-reconstituted NPG mouse model of transplanted tumor constructed by inoculating human breast cancer cells HCC1954

Her2-positive human breast cancer cells HCC1954 were selected to study the inhibiting effect of bispecific antibodies on tumor growth *in vivo* in a CD34 immune-reconstituted NPG mouse model of transplanted tumor constructed by subcutaneously inoculating human breast cancer cells HCC1954.

CD34-positive hematopoietic stem cells were enriched from fresh umbilical cord blood using CD34-positive selective magnetic beads (purchased from Miltenyi Biotec, Germany). Female NPG mice at the age of seven to eight weeks (purchased from Beijing Vitalstar Biotechnology Co., Ltd.) were selected and injected with CD34-positive hematopoietic stem cells via the tail vein to reconstitute a human immune system in each mouse. Sixteen weeks later, blood was collected from the orbital venous plexus of mice for flow cytometry, and when the proportion of human CD45 in mice was greater than 15%, it was considered that the immune reconstitution succeeded. HCC1954 cells in the logarithmic growth stage were collected and 5 × 10⁶ HCC1954 cells were inoculated subcutaneously on the right back of the mice with successful immune reconstitution. One hour after inoculation, the mice were randomly divided into three groups with six mice in each group according to their weights. The treated groups were intraperitoneally administered with AB7K7 and Herceptin at a dose of 1 mg/kg, and the control group was administered with a PBS solution of the same volume, twice a week for a total of 6 doses. The day of administration was recorded as Day 0. The maximum diameter (D) and the minimum diameter (d) of the tumor were measured weekly. The volume (mm³) of the tumor of each group and the tumor growth inhibition rate (TGI) (%) of each treated group were calculated using the formulas as shown in Example 3.1.

As shown in FIG. 3-6, on Day 21 of administration, the average tumor volume of the PBS control group was 475.23 ± 58.82 mm³; the average tumor volume of the treated group administrated with Herceptin was 293.27 ± 66.35 mm³, and the TGI was 38.29%, which was not significantly different from that of the control group; the average tumor volume of the treated group administrated with AB7K7 was 0.67 ± 0.67 mm³, and the TGI was 99.86%, meaning that basically all tumors were regressed, which was significantly different from that of the control group (P < *0.01*). In summary, the above results show that the bispecific antibody AB7K7 had an excellent anti-tumor effect in the CD34 immune-reconstituted model.

### 3.6 PBMC immune-reconstituted NPG mouse model of transplanted tumor constructed by inoculating human breast cancer cells HCC1954

Her2-positive HCC1954 cells were selected to study the inhibiting effect of bispecific antibodies on tumor growth *in vivo* in a PBMC immune-reconstituted NPG mouse model of transplanted tumor constructed by inoculating human breast cancer cells HCC1954.

The peripheral blood of a normal human was subjected to density gradient centrifugation to separate human PBMCs. Female NPG mice at the age of five to six weeks were selected and intraperitoneally injected with human PBMC cells to reconstitute a human immune system in each mouse. After seven days of PBMC injection, HCC1954 cells in the logarithmic growth stage were collected and 5 × 10⁶ HCC1954 cells were inoculated subcutaneously on the right back of each mouse. After 13 days of PBMC injection, blood was collected from the orbital venous plexus for flow cytometry, and when the proportion of human CD45 in mice was greater than 15%, it was considered that the immune reconstitution succeeded. After 14 days of PBMC injection, the mice with successful immune reconstitution were randomly divided into two groups with six mice in each group according to the tumor volumes and weights. The treated group was intraperitoneally administered with AB7K7 at a dose of 1 mg/kg, and the control group was administered with PBS, three times a week. The day of administration was recorded as Day 0. The maximum diameter (D) and the minimum diameter (d) of the tumor were measured weekly. The volume (mm³) of the tumor of each group and the tumor growth inhibition rate (TGI) (%) of each treated group were calculated using the formulas as shown in Example 3.1.

As shown in FIG. 3-7, on Day 23 of administration, the average tumor volume of the PBS control group was 1224.05 ± 224.39 mm³; the average tumor volume of the treated group administrated with AB7K7 was 32.00 ± 0.00 mm³, and the TGI was 97.41%, meaning that basically all tumors were regressed, which was significantly different from that of the control group (*P* < *0.001*). In summary, the above results show that the bispecific antibody AB7K7 had an excellent anti-tumor effect in the PBMC immune-reconstituted model.

### Example 4 Evaluation of the safety of Anti-Her2xCD3 bispecific antibodies

### 4.1 Bispecific antibodies being incapable of mediating non-specific killing on Her2-negative tumor cells

Her2-negative human Burkkit's lymphoma Raji cells were selected to study whether bispecific antibodies can inhibit tumor growth in an NCG mouse model of transplanted tumor constructed by subcutaneously co-inoculating human CIK cells and human Burkkit's lymphoma Raji cells.

CIK cells were prepared in the method as described in Example 3.1. Female NCG mice at the age of seven to eight weeks were selected and Raji cells (purchased from the cell bank of Chinese Academy of Sciences, Shanghai) in the logarithmic growth stage were collected. 5 × 10⁶ Raji cells and 2 × 10⁶ CIK cells were mixed and inoculated subcutaneously on the right back of each NCG mouse. One hour after inoculation, the mice were randomly divided into three groups with five mice in each group according to their weights. The treated groups were intraperitoneally administered with AB7K4 and AB7K7 at a dose of 1 mg/kg, and the control group was administered with a PBS solution of the same volume, once a day continuously for 10 days. The day of administration was recorded as Day 0. The maximum diameter (D) and the minimum diameter (d) of the tumor were measured weekly. The volume (mm³) of the tumor of each group and the tumor growth inhibition rate (TGI) (%) of each treated group were calculated using the formulas as shown in Example 3.1.

As shown in FIG. 4-1, on Day 25 of administration, the average tumor volume of the PBS control group was 2439.88 ± 193.66 mm³; the average tumor volume of the treated group administrated with AB7K4 was 2408.81 ± 212.44 mm³; the average tumor volume of the treated group administrated with AB7K7 was 2598.11 ± 289.35 mm³; and there was no difference between the average tumor volume of each of the two treated groups and the average tumor volume of the control group. In summary, the results show that bispecific antibodies AB7K4 and AB7K7 exhibited no non-specific killing on Her2-negative cell strains, which indicates that AB7K4 and AB7K7 do not mediate T cells to kill non-target tissues *in vivo* (i.e., specifically dependent on binding of bispecific antibodies to corresponding target antigens), there is no off-target toxicity, and the safety is high.

### 4.2 Bispecific antibodies killing tumor cells depending on the activation of T cells

Her2-positive human breast cancer cells HCC1954 were selected to study whether bispecific antibodies inhibit tumor growth in an NPG mouse model of transplanted tumor constructed by subcutaneously inoculating human breast cancer cells HCC1954.

Female NPG mice at the age of seven to eight weeks were selected and HCC1954 cells in the logarithmic growth stage were collected. 5 × 10⁶ HCC1954 cells and Matrigel (Corning, Cat. No. 354234) were mixed in a volume ratio of 1 : 1 and then inoculated subcutaneously on the right back of each NPG mouse. After 6 days of tumor growth, the mice were randomly divided into three groups with six mice in each group according to the tumor volumes and weights. The treated groups were intraperitoneally administered with Herceptin at a dose of 3 mg/kg and AB7K7 at a dose of 1 mg/kg, respectively, and the control group was administered with a PBS solution of the same volume, twice a week. The day of administration was recorded as Day 0. The maximum diameter (D) and the minimum diameter (d) of the tumor were measured weekly. The volume (mm³) of the tumor of each group and the tumor growth inhibition rate (TGI) (%) of each treated group were calculated using the formulas as shown in Example 3.1.

As shown in FIG. 4-2, on Day 21 of administration, the average tumor volume of the PBS control group was 1311.35 ± 215.70 mm³; the average tumor volume of the treated group administrated with Herceptin was 273.98 ± 60.10 mm³; the average tumor volume of the treated group administrated with AB7K7 was 1243.20 ± 340.31 mm³, which was not different from the average tumor volume of the control group. In summary, the results show that AB7K7 did not inhibit the growth of HCC1954 subcutaneous tumors in the absence of human immune cells, indicating that bispecific antibody AB7K7 needs to be mediated by immune effector cells so as to kill tumor cells, unlike Herceptin, which primarily depends on FcyR-mediated ADCC or CDC effects to kill tumor cells. Thus, it is proved that Fc variants contained in AB7K7 cannot bind to FcyR, which avoids mediating systemic activation of T cells caused by extensive expression of its receptor FcyR, resulting in higher drug safety.

### 4.3 Evaluation of toxicity of bispecific antibodies to normal cynomolgus monkeys

Adult cynomolgus monkeys (purchased from Guangzhou Xiangguan Biotechnology Co., Ltd.) at the age of 3-4 years and with the weight of 3-4 kg were divided into three groups with one monkey in each group, wherein the three groups were a vehicle control group, an AB7K7 treated group and an AB7K8 treated group. The groups were administrated via intravenous drip by a peristaltic pump for 1 hour on Day 0 (D0), Day 7 (D7), Day 21 (D21), and Day 28 (D28), respectively, for a total of four doses, and the drug dose was gradually increased each time. The monkeys were weighed weekly. The dose amount and volume administered are shown in Table 4-1.

**Table 4-1 Dosing schedule for cynomolgus monkey acute toxicity evaluation**

| Group | To-be-tested drugs name | Dose volume | Dose amount |
|---|---|---|---|
| G1 | Vehicle control group | D0: 5 mL/kg | N/A |
| | | D7: 5 mL/kg | |
| | | D21: 10 mL/kg | |
| | | D28: 10 mL/kg | |
| G2 | AB7K7 | D0: 5 mL/kg | D0: 0.06 mg/kg |
| | | D7: 5 mL/kg | D7: 0.3 mg/kg |
| | | D21: 10 mL/kg | D21: 1.5 mg/kg |
| | | D28: 10 mL/kg | D28: 3 mg/kg |
| G3 | AB7K8 | D0: 5 mL/kg | D0: 0.04 mg/kg |
| | | D7: 5 mL/kg | D7: 0.2 mg/kg |
| | | D21: 10 mL/kg | D21: 1 mg/kg |
| | | D28: 10 mL/kg | D28: 2 mg/kg |

On D0, after administration, cynomolgus monkeys in the AB7K8 treated group exhibited somnolence and pupil contraction and recovered to normal the next day while there was no abnormality in the other groups. On D7, after administration, cynomolgus monkeys in the AB7K7 treated group exhibited vomiting symptoms 2-3 hours after administration and recovered to normal the next day of administration while there was no abnormality in the other groups. On D21, after administration, cynomolgus monkeys in the AB7K7 treated group exhibited symptoms of vomiting food 3 hours after administration and excreted jelly-like feces, cynomolgus monkeys in the AB7K8 treated group exhibited symptoms of vomiting food 1 hour after administration, and cynomolgus monkeys in both groups recovered to normal on the second day after administration; On D28, after administration, cynomolgus monkeys in both AB7K7 treated group and AB7K8 treated group exhibited vomiting symptoms 40 to 50 minutes later and excreted feces 3 hours later, in which jelly-like mucus was found; cynomolgus monkeys in the AB7K7 treated group excreted watery feces with fishy smelling; and 24 hours later, all the animals recovered to normal and ingested normally. The body weight change of cynomolgus monkeys is shown in FIG. 4-3, wherein the arrow represents the administration time. It can be seen that the body weight of each group does not change too much and fluctuates within the normal physiological range.

The different degree of diarrhea observed in this example may be related to the expression of related receptors in the gut, which is supposed to be caused by the imbalance of chloride ion in the gut caused by the inhibition of heterodimer of Her1/Her2 or Her2/Her3 by bispecific antibodies, which belongs to the extension of pharmacological action and can recover to normal after 24 hours of administration. Cynomolgus monkeys were still well tolerated when administrated with AB7K7 at a high dose of 3 mg/kg. The results of pharmacodynamics test in mice show that AB7K7 at a low dose shows a good anti-tumor effect, which indicates that AB7K7 has a wide treatment window and high safety.

### Example 5 Pharmacokinetics study of Anti-Her2xCD3 antibodies

### 5.1 In vivo pharmacokinetics test of bispecific antibody AB7K7 in SD mice

AB7K7 was administered to four healthy Sprague-Dawley (SD) rats (purchased from Shanghai Salccas Laboratory Animals Co., Ltd.,) via the tail vein at a dose of 1 mg/kg. The blood sampling time points were Hour 1, Hour 3, Hour 6, Hour 24, Hour 72, Hour 96, Hour 120, Hour 168, Hour 216 and Hour 264, respectively. A certain amount of whole blood was taken at each time point, the serum was separated, and then the drug concentration in the serum was detected by two ELISA methods.

Method I. Plates were coated with the anti-AB7K7 antibody A (Ampsource Biopharma Shanghai Inc., mouse-anti-herceptin) at a concentration of 0.5 µg/mL. AB7K7 was formulated at concentrations of 100 ng/mL, 50 ng/mL, 25 ng/mL, 12.5 ng/mL, 6.25 ng/mL, 3.125 ng/mL and 1.56 ng/mL, separately. Standard curves were established. HRP-labeled anti-AB7K7 antibody B (Ampsource Biopharma Shanghai Inc., anti-herceptin-HRP) was used at a concentration of 1 : 5000, and developed with TMB. The pharmacokinetics parameters were calculated using software PKSolver. Specific parameters are shown in Table 5-1.

Method II. The drug concentration in the serum of the SD rats was detected. Plates were coated with the anti-AB7K7 antibody A (Ampsource Biopharma Shanghai Inc., mouse-anti-herceptin) at a concentration of 0.5 µg/mL. AB7K7 was formulated at concentrations of 5 ng/mL, 2.5 ng/mL, 1.25 ng/mL, 0.625 ng/mL, 0.3125 ng/mL, 0.156 ng/mL and 0.078 ng/mL, separately. Standard curves were established. Mouse anti-human IgG Fc-HRP (Ampsource Biopharma Shanghai Inc.) was added at a concentration of 1 : 5000, and developed with TMB. The pharmacokinetics parameters were calculated using software PKSolver. Specific parameters are shown in Table 5-2.

FIG. 5-1 shows the blood drug concentration of AB7K7 in the body of rats detected using two different detection methods. It can be seen that the blood drug concentrations obtained by detecting the concentration of AB7K7in blood using two different detection methods were basically the same, and the calculated pharmacokinetics parameters were roughly equivalent, which indicates that AB7K7 can be metabolized in the form of intact molecules *in vivo,* thereby ensuring the biological function of AB7K7.

**Table 5-1 Pharmacokinetics parameters of bispecific antibody AB7K7 in SD rats (Method 1)**

| AB7K7 | t_{1/2} (h) | AUC 0-inf_ob (ng/mL*h) | Vz_obs (µg)/(ng/mL) | Cl_obs (µg)/(ng/mL)/h |
|---|---|---|---|---|
| Pharmacoki netics parameter | 42.10 | 550236.77 | 0.02351 | 3.811E-4 |

**Table 5-2 Pharmacokinetics parameters of bispecific antibody AB7K7 in SD rats (Method 2)**

| AB7K7 | t1/2 (h) | AUC 0-inf_ob (ng/mL*h) | Vz_obs (µg)/(ng/mL) | Cl_obs (µg)/(ng/mL)/h |
|---|---|---|---|---|
| Pharmaco kinetics parameter | 41.02 | 706126.89 | 0.01720 | 2.899E-4 |

### 5.2 In vivo pharmacokinetics test on bispecific antibody AB7K7 in NPG model mice

NPG mice (purchased from Beijing Vitalstar Biotechnology Co., Ltd.) were inoculated with HCC1954 cells (purchased from the Institute of Cells, Chinese Academy of Sciences) one week before administration, with an inoculum density of 3.5 × 10⁶/mouse. CIK cells were resuscitated two days before administration, cultured for 24 hours and then collected and injected intravenously into mice. The mice were randomly divided into three groups with four mice in each group. The three treated groups were administrated at doses of 0.3 mg/kg, 1 mg/kg and 3 mg/kg, respectively. The blood sampling time points were Hour 1, Hour 3, Hour 6, Hour 24, Hour 48, Hour 72, Hour 96, Hour 120, Hour 168, Hour 216 and Hour 264, respectively. A certain amount of whole blood was taken at each time point, the serum was separated, and then the drug concentration in the serum was detected by ELISA.

Plates were coated with the anti-AB7K7 antibody A (Ampsource Biopharma Shanghai Inc., mouse-anti-herceptin) at a concentration of 0.5 µg/mL. AB7K7 was formulated at concentrations of 100 ng/mL, 50 ng/mL, 25 ng/mL, 12.5 ng/mL, 6.25 ng/mL, 3.125 ng/mL and 1.56 ng/mL, separately. Standard curves were established. HRP-labeled anti-AB7K7 antibody B (Ampsource Biopharma Shanghai Inc., mouse-anti-herceptin) was used at a concentration of 1 : 5000, and developed with TMB. The pharmacokinetics parameters were calculated using software PKSolver. Specific parameters are shown in Table 5-3.

It can be seen from Table 5-3 that the pharmacokinetics parameters of AB7K7 in NPG model mice were not significantly different from those in SD rats.

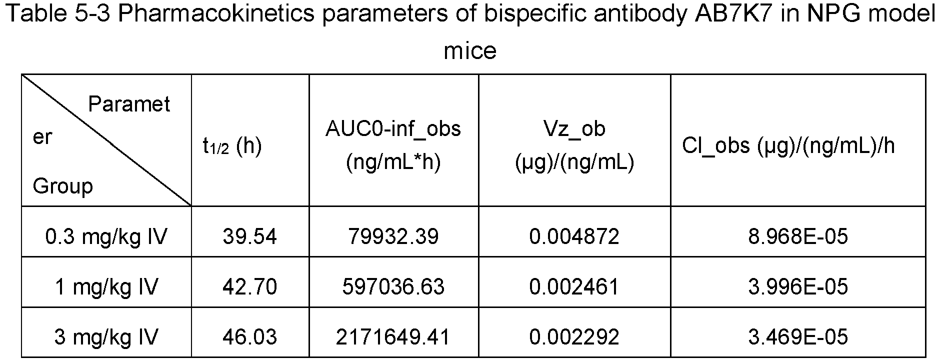

### 5.3 In vivo pharmacokinetics test on bispecific antibody AB7K8 in SD rats

AB7K8 was administered to three healthy SD rats via the tail vein at doses of 1 mg/kg and 3 mg/kg, respectively. The blood sampling time points were Hour 0.25, Hour 0.5, Hour 1, Hour 2, Hour 3, Hour 4, Hour 5, and Hour 7, respectively. A certain amount of whole blood was taken at each time point, the serum was separated, and then the drug concentration in the serum was detected by ELISA.

Plates were coated with the anti-AB7K8 antibody C (Ampsource Biopharma Shanghai Inc., mouse-anti-herceptin) at a concentration of 2.5 µg/mL. AB7K8 was formulated at concentrations of 25 ng/mL, 12.5 ng/mL, 6.25 ng/mL, 3.125 ng/mL, 1.56 ng/mL, and 0.78 ng/mL, separately. Standard curves were established. HRP-labeled anti-his antibody (Ampsource Biopharma Shanghai Inc.) was used at a concentration of 1 : 5000, and developed with TMB. The pharmacokinetics parameters were calculated using software PKSolver. Specific parameters are shown in Table 5-4.

The pharmacokinetics parameter T_{1/2} of AB7K8 were almost the same at two doses, which indicates that AB7K8 showed linear metabolic kinetics in SD rats. Since AB7K8 does not contain Fc, T_{1/2} of AB7K8 is very short, about twenty times shorter than T_{1/2} of AB7K7.

**Table 5-4 Pharmacokinetics parameters of bispecific antibody AB7K8 in SD rats**

| AB7K8 | t_{1/2} (h) | AUC 0-inf_ob (ng/mL*h) | Vz_obs (µg)/(ng/mL) | Cl_obs (µg)/(ng/mL)/h |
|---|---|---|---|---|
| 1 mg/kg IV | 2.27 | 4623.14 | 0.17082 | 0.05191 |
| 3 mg/kg IV | 1.98 | 20608.77 | 0.10220 | 0.03579 |

### 5.4 In vivo pharmacokinetics test on bispecific antibody AB7K in SD rats

AB7K was administered to four healthy SD rats via the tail vein at a dose of 0.8 mg/kg. The blood sampling time points were Hour 2, Hour 24, Hour 48, Hour 72, Hour 96, Hour 120, Hour 144, Hour 168, Hour 216 and Hour 264, respectively. A certain amount of whole blood was taken at each time point, the serum was separated, and then the drug concentration in the serum was detected by two ELISA methods.

Method I. Plates were coated with the anti-AB7K antibody A (Ampsource Biopharma Shanghai Inc., mouse-anti-herceptin) at a concentration of 1 µg/mL. AB7K was formulated at concentrations of 20 ng/mL, 10 ng/mL, 5 ng/mL, 2.5 ng/mL, 1.25 ng/mL, 0.625 ng/mL and 0.3125 ng/mL, separately. Standard curves were established. 25 ng/mL of biotin-labeled human CD3E&CD3D (Acro, Cat. No. CDD-H82W0) was added, incubated for 1 hour, and after that, HRP-labeled streptavidin (BD Pharmingen, Cat. No. 554066) diluted at a factor of 1 : 500 was added, and developed with TMB. The pharmacokinetics parameters were calculated using software PKSolver. Specific parameters are shown in Table 5-5.

Method II. Plates were coated with the anti-AB7K antibody A (Ampsource Biopharma Shanghai Inc., mouse-anti-herceptin) at a concentration of 1 µg/mL. AB7K was formulated at concentrations of 20 ng/mL, 10 ng/mL, 5 ng/mL, 2.5 ng/mL, 1.25 ng/mL, 0.625 ng/mL and 0.3125 ng/mL, separately. Standard curves were established. Mouse anti-human IgG Fc-HRP (diluted at 1 : 10000) (Ampsource Biopharma Shanghai Inc.) was added, incubated in an incubator for 1 hour, and developed with TMB.

FIG. 5-2 shows the blood drug concentration of AB7K in rats by using two different detection methods. From the results, it is showed that the difference between the two detection methods is large. The concentrations of the first two points (2h, 1D) on the curve were close, but after the next day, the concentrations detected by the two methods differed greatly, which is supposed to be caused by the fact that the linkage peptide between the heavy chains of anti-CD3 scFv and anti-Her2 antibodies was broken. AB7K is structurally unstable in vivo and thus can't play its biological function, while the improved AB7K7 can metabolize in complete form in vivo and thus can play its biological function normally.

**Table 5-5 Pharmacokinetics parameters of bispecific antibody AB7K**

| AB7K | t_{1/2} (h) | AUC 0-inf_ob (ng/mL *h) | Vz_obs (µg)/(ng/mL) | Cl_obs (µg)/(ng/mL)/h |
|---|---|---|---|---|
| Pharmacokinetics parameter | 60.47 | 1022788.69 | 0.01726 | 1.985E-4 |

### 5.5 In vivo pharmacokinetics test on bispecific antibodies AB7K7 and AB7K8 in cynomolgus monkeys

Female cynomolgus monkeys (purchased from Guangzhou Xiangguan Biotechnology Co., Ltd.) with the weight of 3-4 kg were divided into three groups with one monkey in each group. The first group (G1-1) was a blank control group; the second group (G2-1) was an AB7K7 treated group administrated at a dose of 0.3 mg/kg; and the third group (G3-1) was an AB7K8 treated group administrated at a dose of 0.2 mg/kg. The blood sampling time points were Minute 15, Hour 1, Hour 3, Hour 6, Hour 24, Hour 48, Hour 72, Hour 96, Hour 144, Hour 192, Hour 240 and Hour 288, respectively, a total of 13 time points. Serum was collected from blood and frozen at -80°C. The concentration of the drug in serum was determined by ELISA.

Plates were coated with the anti-AB7K7 antibody A (Ampsource Biopharma Shanghai Inc., mouse-anti-herceptin) at a concentration of 0.5 µg/mL. AB7K7 was formulated at concentrations of 100 ng/mL, 50 ng/mL, 25 ng/mL, 12.5 ng/mL, 6.25 ng/mL, 3.125 ng/mL and 1.56 ng/mL, separately. Standard curves were established. HRP-labeled anti-AB7K7 antibody B (Ampsource Biopharma Shanghai Inc., mouse-anti-herceptin) was used at a concentration of 1 : 5000, and developed with TMB. The pharmacokinetics parameters were calculated using software PKSolver. Specific parameters are shown in Table 5-6.

FIG. 5-3 shows the blood drug concentration of AB7K7 in rats. T_{1/2} of AB7K7 in the normal cynomolgus monkey was only about eight hours. Pharmacokinetics parameters of AB7K8 could not be calculated due to too few points on the concentration-time curve of AB7K8. However, it can be seen from the concentration-time curve that the half-life of AB7K7 in the normal cynomolgus monkey was much longer than the half-life of AB7K8.

**Table 5-6 Pharmacokinetics parameters of bispecific antibody AB7K7 in cynomolgus monkeys**

| AB7K7 | t_{1/2} (h) | AUC 0-inf_obs (ng/mL*h) | Vz_obs (µg)/(ng/mL) | Cl_obs (µg)/(ng/mL)/h |
|---|---|---|---|---|
| Pharmacokin etics parameter | 7.95 | 87995.48 | 0.1563 | 0.01364 |

### 5.6 Evaluation of abilities of bispecific antibodies to bind to FcRn by ELISA

Each bispecific antibody was diluted with the PBS solution to a concentration of 10 µg/ml and added to 96-well plates, 100 µl per well. The plates were coated at 4 °C overnight. The plates were then blocked with 1% skimmed milk powder for 1 hour at room temperature. Biotin-labeled FcRn proteins (ACRO Biosystem, Cat. No. FCM-H8286) were diluted using diluents at pH of 6.0 and 7.0, respectively, with a 4-fold gradient for a total of 11 concentration gradients. The 96-well plates were then washed with PBST of the same pH, and then each of the bispecific antibodies diluted with the diluent at the same pH was added. Control wells without antibodies were set. Incubated for 1 hour at room temperature. Plates were washed with the PBST solution of the same pH, streptavidin-HRP (BD, Cat. No. 554066) was added to 96-well plates, 100 µl per well, and the plates were incubated for 0.5 hours at room temperature. 96-well plates were washed with PBST, and TMB was added to the plates, 100 µl per wells. Color development was performed at room temperature for 15 minutes, and then 0.2 M H₂SO₄ was added to stop the color development reaction. The light absorbance values at A450-620 nm were measured by a microplate reader. Analysis was performed by software OriginPro 8, and the EC₅₀ values for the binding of bispecific antibodies to FcRn were calculated.

The results show that the ability of each antibody to bind to FcRn was different under different pH conditions, and it is analyzed in conjunction with data of *in vivo* PK that the half-life of bispecific antibody AB7K7 was longer than the half-life of AB7K but shorter than the half-life of Herceptin, which may be more favorable for clinical application (FIGS. 5-4 and 5-5). Table 5-7 and Table 5-8 show the detection results of the ability of each antibody to bind to FcRn at pHs 6.0 and 7.0, respectively.

**Table 5-7 Detection of abilities of bispecific antibodies AB7K, AB7K5 and AB7K7 to bind to FcRn at pH 6.0**

| | Herceptin | AB7K | AB7K5 | AB7K7 |
|---|---|---|---|---|
| EC50 (µg/ml) | 2.591 | 0.8027 | 1.706 | 0.4630 |

**Table 5-8 Detection of abilities of bispecific antibodies AB7K, AB7K5 and AB7K7 to bind to FcRn at pH 7.0**

| | Herceptin | AB7K | AB7K5 | AB7K7 |
|---|---|---|---|---|
| EC50 (µg/ml) | ∼ 287.1 | 1.651 | 13.43 | 4.838 |

Based on the results of the preceding researches on six anti-Her2×CD3 bispecific antibodies from multiple aspects, it can be determined that such bispecific antibodies with the configuration of scFv1-scFv2-Fc as AB7K7 are easy to be prepared and simple and efficient to be purified and has good stability during preparation and storage. More favorably, such bispecific antibodies have the significant advantages of weak non-specific killing effects on normal cells, controlled toxic side effects that may be caused by over-activation of effector cells, and good druggability.

Some preferred amino acid sequences of the VH domain and its complementarity determining regions (HCDR1, HCDR2 and HCDR3) and amino acid sequences of the VL domain and its complementarity determining regions (LCDR1, LCDR2 and LCDR3) of a first single-chain Fv against Her2 are listed in Table 5-9, wherein amino acid residues contained in the CDRs are defined according to the Kabat rule. The amino acid composition of the linker peptide between VH and VL of anti-Her2 scFv is (GGGGS)n, wherein n = 1, 2, 3, 4 or 5.

**Table 5-9: Amino acid sequences of the anti-Her2 ScFv contained in the bispecific antibody and its CDRs**

| Her2 | | |
|---|---|---|
| SEQ ID NO: 9 | HCDR1 | DTYIH |
| SEQ ID NO: 10 | HCDR2 | RIYPTNGYTRYADSVKG |
| SEQ ID NO: 11 | HCDR3 | WGGDGFYAMDY |
| SEQ ID NO: 12 | LCDR1 | RASQDVNTAVA |
| SEQ ID NO: 13 | LCDR2 | SASFLYS |
| SEQ ID NO: 14 | LCDR3 | QQHYTTPPT |
| SEQ ID NO: 15 | VH | |
| SEQ ID NO: 16 | VL | |
| | | |

| Her2 | | |
|---|---|---|
| SEQ ID NO: 17 | HCDR1 | DYTMD |
| SEQ ID NO: 18 | HCDR2 | DVNPNSGGSIYNQRFKG |
| SEQ ID NO: 19 | HCDR3 | NLGPSFYFDY |
| SEQ ID NO: 20 | LCDR1 | KASQDVSIGVA |
| SEQ ID NO: 21 | LCDR2 | SASYRYT |
| SEQ ID NO: 22 | LCDR3 | QQYYIYPYT |
| SEQ ID NO: 23 | VH | |
| SEQ ID NO: 24 | VL | |

| Her2 | | |
|---|---|---|
| SEQ ID NO: 25 | HCDR1 | DTYIH |
| SEQ ID NO: 26 | HCDR2 | RIYPTNGYTRYDPKFQD |
| SEQ ID NO: 27 | HCDR3 | WGGDGFYAMDY |
| SEQ ID NO: 28 | LCDR1 | KASQDVNTAVA |
| SEQ ID NO: 29 | LCDR2 | SASFRYT |
| SEQ ID NO: 30 | LCDR3 | QQHYTTPPT |
| SEQ ID NO: 31 | VH | QVQLQQSGPELVKPGASLKLSCTASGFNIKDTYIHW VKQRPEQGLEWIGRIYPTNGYTRYDPKFQDKATITA DTSSNTAYLQVSRLTSEDTAVYYCSRWGGDGFYAM DYWGQGASVTVSS |
| SEQ ID NO: 32 | VL | DIVMTQSHKFMSTSVGDRVSITCKASQDVNTAVAW YQQKPGHSPKLLIYSASFRYTGVPDRFTGSRSGTDF TFTISSVQAEDLAVYYCQQHYTTPPTFGGGTKVEIK |

The anti-CD3 scFv binds to an effector cell at an EC₅₀ value greater than about 50 nM, or greater than 100 nM, or greater than 300 nM, or greater than 500 nM in an in vitro FACS binding assay; more preferably, the second single-chain Fv of the bispecific antibody is capable of binding to human CD3 and specifically binding to CD3 of a cynomolgus monkey or a rhesus monkey.

Some preferred amino acid sequences of the VH domain and its complementarity determining regions (HCDR1, HCDR2 and HCDR3) and amino acid sequences of the VL domain and its complementarity determining regions (LCDR1, LCDR2 and LCDR3) of the anti-CD3 scFv are listed in Table 5-10, wherein amino acid residues contained in the CDRs are defined according to the Kabat rule. The amino acid composition of the linker peptide between VH and VL of the anti-CD3 scFv is (GGGGS)n, where n = 1, 2, 3, 4 or 5.

**Table 5-10 Amino acid sequences of the anti-CD3 ScFv contained in the bispecific antibody and its CDRs**

| CD3-3 | | |
|---|---|---|
| SEQ ID NO: 33 | HCDR1 | TYAMN |
| SEQ ID NO: 34 | HCDR2 | RIRSKYNNYATYYADSVKD |
| SEQ ID NO: 35 | HCDR3 | HGNFGNSYVSWFAY |
| SEQ ID NO: 36 | LCDR1 | RSSTGAVTTSNYAN |
| SEQ ID NO: 37 | LCDR2 | GTNKRAP |
| SEQ ID NO: 38 | LCDR3 | ALWYSNLVW |
| SEQ ID NO: 39 | VH | EVQLLESGGGLVQPGGSLKLSCAASGFTFNTYAMN WVRQAPGKGLEWVARIRSKYNNYATYYADSVKDRF TISRDDSKNTAYLQMNNLKTEDTAVYYCVRHGNFG NSYVSWFAYWGQGTLVTVSS |
| SEQ ID NO: 40 | VL | ELVVTQEPSLTVSPGGTVTLTCRSSTGAVTTSNYAN WVQQKPGQAPRGLIGGTNKRAPGTPARFSGSLLG GKAALTLSGVQPEDEAEYYCALWYSNLWVFGGGTK LTVL |

| CD3-4 | | |
|---|---|---|
| SEQ ID NO: 41 | HCDR1 | KYAMN |
| SEQ ID NO: 42 | HCDR2 | RIRSKYNNYATYYADSVKD |
| SEQ ID NO: 43 | HCDR3 | HGNFGNSYISYWAY |
| SEQ ID NO: 44 | LCDR1 | GSSTGAVTSGYYPN |
| SEQ ID NO: 45 | LCDR2 | GTKFLAP |
| SEQ ID NO: 46 | LCDR3 | ALWYSNRWV |
| SEQ ID NO: 47 | VH | EVQLLESGGGLVQPGGSLKLSCAASGFTFNKYAMN WVRQAPGKGLEWVARIRSKYNNYATYYADSVKDRF TISRDDSKNTAYLQMNNLKTEDTAVYYCVRHGNFG |
| | | NSYISYWAYWGQGTLVTVSS |
| SEQ ID NO: 48 | VL | ELVVTQEPSLTVSPGGTVTLTCGSSTGAVTSGYYPN WVQQKPGQAPRGLIGGTKFLAPGTPARFSGSLLGG KAALTLSGVQPEDEAEYYCALWYSNRWVFGGGTKL TVL |

The linker peptide linking the anti-Her2 scFv and the anti-CD3 scFv consists of a flexible peptide and a rigid peptide; preferably, the amino acid composition structure of the flexible peptide has a general formula of GₓS_{y}(GGGGS)_{z}, wherein x, y and z are integers greater than or equal to 0 and x + y + z ≥ 1. The rigid peptide is derived from a full-length sequence (as shown in SEQ ID NO: 49) consisting of amino acids 118 to 145 at the carboxyl terminus of natural human chorionic gonadotropin β-subunit or a truncated fragment thereof; preferably, the composition of the CTP rigid peptide is SSSSKAPPPS (CTP¹). Some preferred amino acid sequences of the linker peptide linking the anti-Her2 scFv and the anti-CD3 scFv are listed in Table 5-11.

**Table 5-11 Amino acid sequences of the linker peptide linking the anti-Her2 scFv and the anti-CD3 scFv**

| | | |
|---|---|---|
| SEQ ID NO: 50 | G₂(GGGGS)₃CTP¹ | GGGGGGSGGGGSGGGGSSSSSKAPPPS |
| SEQ ID NO: 51 | (GGGGS)₃CTP¹ | GGGGSGGGGSGGGGSSSSSKAPPPS |
| SEQ ID NO: 52 | GS(GGGGS)₂CTP¹ | GSGGGGSGGGGSSSSSKAPPPS |
| SEQ ID NO: 53 | (GGGGS)₁CTP⁴ | GGGGSSSSSKAPPPSLPSPSRLPGPSDTPILPQ |

The Fc fragment is linked to the anti-CD3 scFv directly or by a linker peptide, and the linker peptide includes 1-20 amino acids, and preferably selected from the following amino acids: Gly(G), Ser(S), Ala(A) and Thr(T), more preferably selected from Gly (G) and Ser (S), most preferably, the linker peptide consists of (GGGGS)n, wherein n = 1, 2, 3 or 4.

The Fc fragment is preferably selected from heavy chain constant regions of human IgG1, IgG2, IgG3 and IgG4, and more particularly selected from heavy chain constant regions of human IgG1 or IgG4; and Fc is mutated to modify the properties of the bispecific antibody molecule, e.g., to show reduced affinity to at least one of human FcγRs (FcγRI, FcγRIIa or FcγRIIIa) and C1q, a reduced effector cell function, or a reduced complement function. In addition, the Fc fragment may also contain amino acid substitutions that change one or more other characteristics (such as an ability of binding to an FcRn receptor, the glycosylation of the antibody or the charge heterogeneity of the antibody).

Some amino acid sequences of the Fc fragment with one or more amino acid mutations are listed in Table 5-12.

**Table 5-12: Amino acid sequences of Fc from human IgG**

| **Amino acid sequence of constant region of IgG1 Fc (L234A, L235A) mutant (EU numbering)** | |
|---|---|
| SEQ ID NO: 54 | DKTHTCPPCP APEAAGGPSV FLFPPKPKDT LMISRTPEVT CVVVDVSHED PEVKFNWYVD GVEVHNAKTK PREEQYNSTY RVVSVLTVLH QDWLNGKEYK CKVSNKALPA PIEKTISKAK GQPREPQVYT LPPSREEMTK NQVSLTCLVK GFYPSDIAVE WESNGQPENN YKTTPPVLDS DGSFFLYSKL TVDKSRWQQG NVFSCSVMHE ALHNHYTQKS LSLSPGK |

| **Amino acid sequence of constant region of IgG1 (L234A, L235A, T250Q, N297A, P331S, M428L, K447⁻) mutant (EU numbering)** | |
|---|---|
| SEQ ID NO: 55 | DKTHTCPPCP APEAAGGPSV FLFPPKPKDQ LMISRTPEVT CVVVDVSHED PEVKFNWYVD GVEVHNAKTK PREEQYASTY RVVSVLTVLH QDWLNGKEYK CKVSNKALPA SIEKTISKAK GQPREPQVYT LPPSRDELTK NQVSLTCLVK GFYPSDIAVE WESNGQPENN YKTTPPVLDS DGSFFLYSKL TVDKSRWQQG NVFSCSVLHE ALHNHYTQKS LSLSPGK |

All the publications mentioned in the present disclosure are incorporated herein by reference as if each publication is separately incorporated herein by reference. In addition, it should be understood that those skilled in the art, who have read the disclosure, can make various changes or modifications to the present disclosure, and these equivalent forms fall within the scope of the appended claims.

## Claims

1. A bispecific antibody, which is a tetravalent homodimer formed by two identical polypeptide chains that bind to each other by a covalent bond, wherein each of the polypeptide chains comprises a first single-chain Fv that specifically binds to human epidermal growth factor receptor 2, a second single-chain Fv that specifically binds to effector cell antigen CD3, and an Fc fragment in sequence from N-terminus to C-terminus; wherein the first single-chain Fv is linked to the second single-chain Fv by a linker peptide, the second single-chain Fv is linked to the Fc fragment directly or by a linker peptide, and the Fc fragment has no effector functions comprising CDC, ADCC, and ADCP.

2. The bispecific antibody according to claim 1, wherein the first single-chain Fv comprises a VH domain and a VL domain that are linked by a linker peptide which has an amino acid sequence of (GGGGX)ₙ, wherein X comprises Ser or Ala, preferably Ser, and n is a natural number from 1 to 5, preferably 3.

3. The bispecific antibody according to claim 1 or 2, wherein the first single-chain Fv comprises a VH domain and a VL domain selected from the group consisting of:
(i) a VH domain that contains HCDR1, HCDR2 and HCDR3 as shown in SEQ ID NOs: 9, 10 and 11, respectively or having sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 9, 10 and 11; and a VL domain that contains LCDR1, LCDR2 and LCDR3 as shown in SEQ ID NOs: 12, 13 and 14, respectively or having sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 12, 13 and 14;
(ii) a VH domain that contains HCDR1, HCDR2 and HCDR3 as shown in SEQ ID NOs: 17,18 and 19, respectively or having sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 17, 18 and 19; and a VL domain that contains LCDR1, LCDR2 and LCDR3 as shown in SEQ ID NOs: 20, 21 and 22, respectively or having sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 20, 21 and 22; and
(iii) a VH domain that contains HCDR1, HCDR2 and HCDR3 as shown in SEQ ID NOs: 25, 26 and 27, respectively or having sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 25, 26 and 27; and a VL domain that contains LCDR1, LCDR2 and LCDR3 as shown in SEQ ID NOs: 28, 29 and 30, respectively or having sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 28, 29 and 30.

4. The bispecific antibody according to claim 1, 2 or 3, wherein the first single-chain Fv comprises a VH domain and a VL domain selected from the group consisting of:
(i) a VH domain comprising an amino acid sequence as shown in SEQ ID NO: 15 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 15; and a VL domain comprising an amino acid sequence as shown in SEQ ID NO: 16 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 16;
(ii) a VH domain comprising an amino acid sequence as shown in SEQ ID NO: 23 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 23; and a VL domain comprising an amino acid sequence as shown in SEQ ID NO: 24 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 24; and
(iii) a VH domain comprising an amino acid sequence as shown in SEQ ID NO: 31 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 31; and a VL domain comprising an amino acid sequence as shown in SEQ ID NO: 32 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 32.

5. The bispecific antibody according to claim 1, wherein the second single-chain Fv comprises a VH domain and a VL domain that are linked by a linker peptide which has an amino acid sequence of (GGGGX)ₙ, wherein X comprises Ser or Ala, preferably Ser, and n is a natural number from 1 to 5, preferably 3.

6. The bispecific antibody according to claim 1 or 5, wherein the second single-chain Fv binds to an effector cell at an EC₅₀ value greater than about 50 nM, or greater than 100 nM, or greater than 300 nM, or greater than 500 nM in an *in vitro* binding affinity assay; more preferably, the second single-chain Fv of the bispecific antibody is capable of binding to human CD3 and specifically binding to CD3 of a cynomolgus monkey or a rhesus monkey.

7. The bispecific antibody according to claim 6, wherein the VH domain of the second single-chain Fv contains HCDR1, HCDR2 and HCDR3 as shown in SEQ ID NOs: 33, 34 and 35, respectively or having sequences that are at least 80%, 85%, 90%, 92%, 95%, 97%, 98%, 99% or more similar to or have one or more amino acid substitutions than SEQ ID NOs: 33, 34 and 35; and the VL domain of the second single-chain Fv contains LCDR1, LCDR2 and LCDR3 as shown in SEQ ID NOs: 36, 37 and 38, respectively or having sequences that are at least 80%, 85%, 90%, 92%, 95%, 97%, 98%, 99% or more similar to or have one or more amino acid substitutions than SEQ ID NOs: 36, 37 and 38.

8. The bispecific antibody according to claim 6, wherein the VH domain of the second single-chain Fv contains HCDR1, HCDR2 and HCDR3 as shown in SEQ ID NOs: 41, 42 and 43, respectively or having sequences that are at least 80%, 85%, 90%, 92%, 95%, 97%, 98%, 99% or more similar to or have one or more amino acid substitutions than SEQ ID NOs: 41, 42 and 43; and the VL domain of the second single-chain Fv contains LCDR1, LCDR2 and LCDR3 as shown in SEQ ID NOs: 44, 45 and 46, respectively or having sequences that are at least 80%, 85%, 90%, 92%, 95%, 97%, 98%, 99% or more similar to or have one or more amino acid substitutions than SEQ ID NOs: 44, 45 and 46.

9. The bispecific antibody according to claim 7, wherein the second single-chain Fv specifically binds to CD3; the VH domain of the second single-chain Fv contains an amino acid sequence as shown in SEQ ID NO: 39 or has a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 39; and the VL domain of the second single-chain Fv contains an amino acid sequence as shown in SEQ ID NO: 40 or has a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 40.

10. The bispecific antibody according to claim 8, wherein the second single-chain Fv specifically binds to CD3; the VH domain of the second single-chain Fv contains an amino acid sequence as shown in SEQ ID NO: 47 or has a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 47; and the VL domain of the second single-chain Fv contains an amino acid sequence as shown in SEQ ID NO: 48 or has a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 48.

11. The bispecific antibody according to claim 1, wherein the linker peptide that links the first single-chain Fv to the second single-chain Fv consists of a flexible peptide and a rigid peptide; wherein the flexible peptide comprises two or more amino acids, and preferably selected from the following amino acids: Gly(G), Ser(S), Ala(A) and Thr(T); more preferably, the flexible peptide comprises G and S residues; most preferably, an amino acid composition structure of the flexible peptide has a general formula of GₓS_{y}(GGGGS)_{z}, wherein x, y and z are integers greater than or equal to 0 and x + y + z ≥ 1; the rigid peptide is derived from a full-length sequence consisting of amino acids 118 to 145 at carboxyl terminus of natural human chorionic gonadotropin β-subunit as shown in SEQ ID NO: 49 or a truncated fragment thereof; preferably, the rigid peptide comprises SSSSKAPPPS.

12. The bispecific antibody according to claim 11, wherein the linker peptide comprises an amino acid sequence as shown in SEQ ID NO: 50, 51, 52 or 53.

13. The bispecific antibody according to claim 1, wherein the linker peptide that links the Fc fragment to the second single-chain Fv comprises 1-20 amino acids, and preferably selected from the following amino acids: Gly(G), Ser(S), Ala(A) and Thr(T); preferably Gly (G) and Ser (S); more preferably, the linker peptide consists of (GGGGS)n, wherein n = 1, 2, 3 or 4.

14. The bispecific antibody according to claim 1, wherein the Fc fragment comprises a hinge region, a CH2 domain and a CH3 domain from a human immunoglobulin heavy chain constant region; preferably, the Fc fragment is selected from heavy chain constant regions of human IgG1, IgG2, IgG3, IgG4, IgM, IgA1, IgA2, IgD and IgE; more preferably, the Fc fragment is selected from heavy chain constant regions of human IgG1, IgG2, IgG3 and IgG4; further preferably, the Fc fragment is selected from a heavy chain constant region of human IgG1 or IgG4; and the Fc fragment has one or more amino acid substitutions, deletions or additions than a natural sequence from which the Fc fragment is derived.

15. The bispecific antibody according to claim 14, wherein the Fc fragment comprises amino acid substitutions, deletions or additions with reduced or eliminated effector functions (ADCP, ADCC and CDC effects).

16. The bispecific antibody according to claim 15, wherein the Fc fragment comprises amino acid substitutions L234A/L235A/P331S that are determined according to an EU numbering system.

17. The bispecific antibody according to claim 15 or 16, wherein the Fc fragment further comprises amino acid substitutions, deletions or additions with one or more of the following properties:
(i) enhanced binding affinity to a neonatal receptor (FcRn);
(ii) reduced or eliminated glycosylation; and
(iii) reduced or eliminated charge heterogeneity.

18. The bispecific antibody according to claim 17, wherein the Fc fragment further comprises one or more of amino acid substitutions, deletions or additions as follow:
(i) amino acid substitutions M428L, T250Q/M428L, M428L/N434S or M252Y/S254T/T256E determined according to the EU numbering system;
(ii) an amino acid substitution N297A determined according to the EU numbering system; and
(iii) an amino acid deletion K447 determined according to the EU numbering system.

19. The bispecific antibody according to claim 18, wherein the Fc fragment has an amino acid sequence as shown in SEQ ID NO: 55 that has six amino acid substitutions or replacements L234A/L235A/N297A/P331 S/T250Q/M428L determined according to the EU numbering system and a deleted or removed K447 determined according to the EU numbering system compared to the natural sequence from which the Fc fragment is derived.

20. The bispecific antibody according to claim 1, wherein the bispecific antibody comprises an amino acid sequence as follows:
(i) a sequence as shown in SEQ ID NO: 8;
(ii) a sequence with one or more substitutions, deletions or additions (such as 1, 2, 3, 4 or 5 substitutions, deletions or additions) than the sequence as shown in SEQ ID NO: 8; or
(iii) a sequence with at least 80%, at least 85%, at least 90%, at least 91%, at least 92%, at least 93%, at least 94%, at least 95%, at least 96%, at least 97%, at least 98%, at least 99% or 100% sequence identity relative to the sequence as shown in SEQ ID NO: 8.

21. A DNA molecule encoding the bispecific antibody according to any one of claims 1 to 20.

22. The DNA molecule according to claim 21, which has a nucleotide sequence as shown in SEQ ID NO: 56.

23. A vector, comprising the DNA molecule according to claim 21 or 22.

24. A host cell, comprising the vector according to claim 23, wherein the host cell comprises a prokaryotic cell, a yeast or a mammalian cell, preferably a CHO cell.

25. A pharmaceutical composition, comprising the bispecific antibody according to any one of claims 1 to 20 and a pharmaceutically acceptable excipient, carrier or diluent.

26. The pharmaceutical composition according to claim 25, wherein the pharmaceutical composition is a solution formulation comprising the bispecific antibody according to any one of claims 1 to 20 and further comprising a pH regulator, a stabilizer, and a surfactant; preferably, the pH regulator is a citrate buffer or a histidine buffer, the stabilizer is sucrose, and the surfactant is tween-80; more preferably, the formulation comprises 0.5 mg/mL of the bispecific antibody, 20 mM of citrate or histidine, 8% (w/v) of sucrose and 0.02% (w/v) of PS80; the formulation has a pH of 5.5.

27. A method for preparing the bispecific antibody according to any one of claims 1 to 20, comprising: (a) obtaining a fusion gene of the bispecific antibody, and constructing an expression vector of the bispecific antibody; (b) transfecting the expression vector into a host cell by a genetic engineering method; (c) culturing the host cell under conditions that allow the bispecific antibody to be generated; (d) separating and purifying the bispecific antibody;
wherein the expression vector in step (a) is one or more selected from a plasmid, a bacterium and a virus; preferably, the expression vector is an pCDNA3.4 vector;
wherein the host cell into which the constructed vector is transfected by the genetic engineering method in step (b) comprises a prokaryotic cell, a yeast cell or a mammalian cell, such as a CHO cell, an NSO cell or another mammalian cell, preferably a CHO cell; and
wherein the bispecific antibody is separated and purified in step (d) by a conventional immunoglobulin purification method comprising protein A affinity chromatography and ion exchange, hydrophobic chromatography or molecular sieve.

28. Use of the bispecific antibody according to any one of claims 1 to 20 or the pharmaceutical composition according to claim 25 or 26 for preparing a drug for treating, preventing or alleviating a tumor, wherein an example of the tumor comprises, but is not limited to, breast cancer, prostate cancer, non-small cell lung cancer, bladder cancer, ovarian cancer, gastric cancer, colorectal cancer, esophageal cancer, head and neck squamous cell carcinoma, cervical cancer, pancreatic cancer, testicular cancer, malignant melanoma, and soft tissue cancer; preferably, the tumor is selected from breast cancer, gastric cancer, or rectal cancer; more preferably, the tumor is selected from breast cancer.

29. The bispecific antibody according to any one of claims 1 to 20 or the pharmaceutical composition according to claim 25 or 26 for use in a method for treating, delaying development of, or reducing/inhibiting recurrence of a tumor, which comprises administering an effective amount of the bispecific antibody or the pharmaceutical composition to a subject suffering from the above disease or disorder, wherein an example of the tumor comprises, but is not limited to, breast cancer, prostate cancer, non-small cell lung cancer, bladder cancer, ovarian cancer, gastric cancer, colorectal cancer, esophageal cancer, head and neck squamous cell carcinoma, cervical cancer, pancreatic cancer, testicular cancer, malignant melanoma, and soft tissue cancer; preferably, the tumor is selected from breast cancer, gastric cancer, or rectal cancer; more preferably, the tumor is selected from breast cancer.

30. The bispecific antibody according to any one of claims 1 to 20 or the pharmaceutical composition according to claim 25 or 26 for use in a method for enhancing or stimulating an immune response or function, which comprises administering a therapeutically effective amount of the bispecific antibody or the pharmaceutical composition to a patient/subject.

31. The bispecific antibody according to any one of claims 1 to 20 or the pharmaceutical composition according to claim 25 or 26 for use in a method for treating, preventing or ameliorating an immune disorder or disease in a patient/subject, which comprises administering a therapeutically effective amount of the bispecific antibody or the pharmaceutical composition to the patient/subject.
